# EUROPÄISCHE PATENTANMELDUNG

(11) **EP 2 042 491 A1**
(43) Veröffentlichungstag der Anmeldung: **01.04.2009**
(21) Anmeldenummer: 07116470.1
(22) Anmeldetag: 14.09.2007
(51) Int. Cl.: C07D 237/12, C07D 237/14, C07D 237/16, C07D 237/18, C07D 237/20, C07D 237/22, A01N 43/58, C07F 7/08

(54) **Pyridazine als Fungizide**

(71) Anmelder: Bayer CropScience AG, 40789 Monheim (DE)
(72) Erfinder: Gaertzen, Oliver, 50735 Köln (DE); Hemmann, Stefan, 40764 Langenfeld (DE); Wachendorff-Neumann, Ulrike, 565666 Nuewied (DE); Dahmen, Peter, 41470 Neuss (DE); Voerste, Amd, 50674 Köln (DE); Cristau, Pierre, 50672 Köln (DE)

(57) **Zusammenfassung**

Pyridazine der Formel (I) 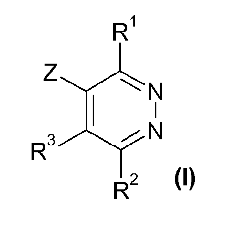 in welcher Z, R¹, R², R³, R⁴, R⁵, R⁶, R⁷, R⁸, R⁹ und R¹⁰ die in der Beschreibung angegebenen Bedeutungen haben, sowie agrochemisch wirksame Salze davon und deren Verwendung zur Bekämpfung von unerwünschten Mikroorganismen sowie Verfahren und Zwischenprodukte zu deren Herstellung.

## Beschreibung

Die Erfindung betrifft Pyridazine der allgemeinen Formel (I), ein Verfahren zu deren Herstellung, deren Verwendung zur Bekämpfung von unerwünschten Mikroorganismen, sowie ein Mittel zu diesem Zweck, umfassend die erfindungsgemäßen Pyridazine. Weiterhin betrifft die Erfindung ein Verfahren zum Bekämpfen unerwünschter Mikroorganismen durch Ausbringen der erfindungsgemäßen Verbindungen auf die Mikroorganismen und/oder in deren Lebensraum.

Es ist bereits bekannt, dass bestimmte Pyridazine als fungizide Mittel im Pflanzenschutz benutzt werden können (siehe WO 2005/121104, WO 2006/001175, WO 2007/066601 und WO 20 07/080720). Die fungizide Wirksamkeit dieser Verbindungen ist jedoch gerade bei niedrigeren Aufwandmengen nicht immer ausreichend.

Da sich die ökologischen und ökonomischen Anforderungen an moderne Fungizide laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Fungizide zu entwickeln, die zumindest in Teilbereichen Vorteile gegenüber den bekannten aufweisen.

Überraschenderweise wurde nun gefunden, dass die vorliegenden Pyridazine die genannten Aufgaben zumindest in Teilaspekten lösen und sich als Fungizide eignen.

Gegenstand der Erfindung sind Verbindungen der Formel (I), 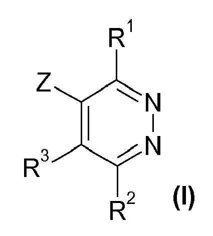 in welcher die Symbole folgende Bedeutungen haben:
- R¹: steht für Wasserstoff, jeweils geradkettiges oder verzweigtes C₁-C₆-Alkyl, C₂-C₆-Alkenyl, C₂-C₆-Alkinyl, C₃-C₇-Cycloalkyl oder C₃-C₇-Cycloalkenyl, wobei R¹ für den Fall, dass es ungleich Wasserstoff ist, durch einen bis drei gleiche oder verschiedene Reste substituiert sein kann, ausgewählt aus Halogen, Cyano, Oxo, Nitro, C₁-C₄-Alkylamino, Di-(C₁-C₄-) Alkylamino, C₁-C₄-Alkyl, C₁-C₄-Halogenalkyl, C₁-C₄-Alkoxy oder unsubstituiertes oder durch Halogen, C₁-C₄-Alkyl oder C₁-C₄-Halogenalkyl substituiertes C₃-C₆-Cycloalkyl,

- R²: steht für Fluor, Chlor, Brom, Cyano, jeweils geradkettiges oder verzweigtes C₁-C₄-Alkoxy, C₃-C₆-Cycloalkyloxy, C₃-C₆-Alkenyloxy, C₃-C₆-Alkinyloxy, C₁-C₄-Alkylthio, C₁-C₄-Alkylsulfinyl, C₁-C₄-Alkylsulfonyl, C₁-C₄-Halogenalkoxy, C₁-C₄-Halogenalkylthio, C₁-C₄-Halogenalkylsulfinyl oder C₁-C₄-Halogenalkylsulfonyl wobei die genannten Reste ausser für die Fälle, in denen R² Fluor, Chlor, Brom oder Cyano ist, durch einen bis drei gleiche oder verschiedene Reste ausgewählt aus Halogen, Cyano, Oxo, Nitro, C₁-C₄-Alkylamino, C₁-C₄-Dialkylamino, C₁-C₄-Alkylthio, C₁-C₄-Alkyl, C₁-C₄-Halogenalkyl, tri(C₁-C₄-Alkyl)silyl, C₁-C₄-Alkoxy oder unsubstituiertem oder durch Halogen, C₁-C₄-Alkyl oder C₁-C₄-Halogenalkyl substituiertem C₃-C₈-Cycloalkyl substituiert sein können,
oder
- R²: steht für jeweils unsubstituiertes oder im Phenylteil einfach bis dreifach substituiertes Phenoxy oder Benzyloxy, wobei die Substituenten im Phenylteil unabhängig voneinander gleichartig oder verschieden sein können ausgewählt aus Fluor, Chlor, Brom, Cyano, Nitro, Methyl, Methoxy, Dimethylamino, Trifluormethyl oder Trifluormethoxy,
- R³: steht für einen Phenylring oder einen ungesättigten Heterocyclus mit fünf bis sieben Ringgliedern und ein bis vier Heteroatomen aus der Gruppe Stickstoff, Sauerstoff und Schwefel, mit der Maßgabe dass zwei Sauerstoffatome nicht benachbart sein dürfen, gegebenenfalls annelliert mit einem weiteren fünf- bis sechsgliedrigen gesättigten oder ganz oder teilweise ungesättigten carbocyclischen oder heterocyclischen Ring, wobei der zusätzliche heterocyclische Ring gegebenenfalls ein bis zwei zusätzliche Heteroatome aus der Gruppe Stickstoff, Sauerstoff und Schwefel enthalten kann, mit der Maßgabe dass zwei Sauerstoffatome nicht benachbart sein dürfen,
welche jeweils einfach oder mehrfach unabhängig voneinander gleich oder verschieden durch folgende Reste substituiert sein können:
Wasserstoff, Fluor, Chlor, Brom, Jod, Cyano, Nitro, Amino, Hydroxy, Mercapto, Formyl, Carboxy, Carbamoyl, Thiocarbamoyl,
jeweils geradkettiges oder verzweigtes C₁-C₆-Alkyl, C₁-C₆-Alkoxy, C₁-C₆-Alkylthio, C₁-C₆-Alkylsulfinyl oder C₁-C₆-Alkylsulfonyl;
jeweils geradkettiges oder verzweigtes C₂-C₆-Alkenyl, C₂-C₆-Alkenylamino oder C₂-C₆-Alkenyloxy;
jeweils geradkettiges oder verzweigtes C₂-C₆-Alkinyl, C₂-C₆-Alkinylamino oder C₂-C₆-Alkinyloxy;
jeweils geradkettiges oder verzweigtes C₁-C₆-Halogenalkyl, C₁-C₆-Halogenalkoxy, C₁-C₇-Halogenalkylcarbonyl, C₁-C₆-Halogenalkylthio, C₁-C₆-Halogenalkylsulfinyl oder C₁-C₆-Halogenalkylsulfonyl mit jeweils 1 bis 13 gleichen oder verschiedenen Halogenatomen;
jeweils geradkettiges oder verzweigtes C₂-C₆-Halogenalkenyl oder C₂-C₆-Halogenalkenyloxy mit jeweils 1 bis 11 gleichen oder verschiedenen Halogenatomen oder geradkettiges oder verzweigtes C₂-C₆-Halogenalkinyloxy;
jeweils geradkettiges oder verzweigtes C₁-C₆-Alkylamino, Di-(C₁-C₆-alkyl)amino, C₁-C₆-Alkylcarbonyl, C₁-C₆-Alkylcarbonyloxy, C₁-C₆-Alkylcarbonylamino, C₁-C₆-Alkoxycarbonyl, C₁-C₆-Alkoxycarbonyloxy, C₁-C₆-Alkoxycarbonylamino, C₁-C₆-Alkylaminocarbonyl, C₁-C₆-Alkylaminocarbonyloxy, C₁-C₆-Alkylaminosulfonyl, Di-(C₁-C₆-alkyl)aminocarbonyl, Di-(C1-C₆-alkyl)aminocarbonyloxy, Di-(C₁-C₆-alkyl)aminosulfonyl, C₁-C₆-Alkylsulfonylamino, C₁-C₆-Alkylsulfonyloxy, Hydroximino-C₁-C₆-alkyl oder C₁-C₆-Alkoximino-C₁-C₆-alkyl;
unsubstituiertes oder in den Alkylteilen einfach oder mehrfach substituiertes C₃-C₈-Cycloalkyl, C₃-C₈-Cycloalkylamino, C₃-C₈-Cycloalkylaminocarbonyl, C₃-C₈-Cycloalkylaminocarbonyloxy, wobei die Substituenten unabhängig voneinander gleich oder verschieden sein können ausgewählt aus Fluor, Chlor, Methyl, Cyclopropyl oder Trifluormethyl;
jeweils geradkettiges oder verzweigtes C₁-C₆-Alkoxy-C₁-C₆-alkyl, C₁-C₆-Halogenalkoxy-C₁-C₆-alkyl, C₁-C₆-Alkylamino-C₁-C₆-alkyl, Di-(C₁-C₆-alkyl)amino-C₁-C₆-alkyl;
jeweils geradkettiges oder verzweigtes C₁-C₆-Alkoxy-C₁-C₆-alkoxy, C₁-C₆-Alkoxy-C₁-C₆-alkoxy-C₁-C₆-alkoxy, C₁-C₆-Halogenalkoxy-C₁-C₆-alkoxy, C₁-C₆-Alkylamino-C₁C₆-alkoxy, Di-(C₁-C₆-alkyl)amino-C₁C₆-alkoxy;
unsubstituiertes oder einfach bis fünffach substituiertes Phenyl, wobei die Substituenten unabhängig voneinander gleich oder verschieden sein können ausgewählt aus Fluor, Chlor, Methyl, Trifluormethyl, N-Methoxyethanimidoyl, (Methoxyimino)methyl;

- oder: zwei benachbarte Reste am Phenylring oder am Heterocyclus bilden zusammen einen fünf- bis siebengliedrigen gesättigten, teilweise gesättigten oder ungesättigten substituierten oder unsubstituierten Ring ggf. enthaltend bis zu zwei Heteroatome ausgewählt aus Stickstoff, Sauerstoff oder Schwefel, mit der Maßgabe dass zwei Sauerstoffatome nicht benachbart sein dürfen und wobei die Substituenten, unabhängig voneinander gleich oder verschieden sein können ausgewählt aus Fluor, Chlor, Methyl, Cyclopropyl oder Trifluormethyl;
- oder: zwei Reste am Heterocyclus bilden mit dem Kohlenstoffatom, an das sie gebundenen sind, eine Carbonyl- oder Thiocarbonylgruppe;
- Z: steht für NR⁴R⁵, NR⁴R⁹, OR⁸, OR¹⁰ oder S(O)ₙR⁴, wobei n gleich 0, 1 oder 2 sein kann;
- R⁴: ist gleich R³, Wasserstoff oder jeweils geradkettiges oder verzweigtes C₁-C₈-Alkyl, C₂-C₈-Alkenyl, C₂-C₆-Alkinyl, C₃-C₇-Cycloalkyl oder C₃-C₇-Cycloalkenyl, wobei R⁴, für den Fall, dass es ungleich R³ oder Wasserstoff ist, jeweils unsubstituiert oder einfach oder mehrfach gleich oder verschieden unabhängig voneinander substituiert sein kann durch Cyano, Oxo, Nitro, C₁-C₄-Alkoxycarbonyl, C₁-C₄-Alkylcarbonyl, Di-(C₁-C₄-Alkyl)aminocarbonyl oder einen gesättigten oder teilweise ungesättigten drei- bis siebengliedrigen Ring, gegebenenfalls enthaltend bis zu drei Heteroatome ausgewählt aus Stickstoff, Sauerstoff oder Schwefel mit der Maßgabe dass zwei Sauerstoffatome nicht benachbart sein dürfen, wobei der Ring selbst gegebenenfalls einfach oder mehrfach gleich oder verschieden unabhängig voneinander durch Fluor, C₁-C₄-Alkyl oder C₁-C₄-Halogenalkyl substituiert sein kann,
oder
- R⁴: steht für jeweils geradkettiges oder verzweigtes C₁-C₆-Halogenalkyl oder C₂-C₆-Halogenalkenyl mit jeweils 1 bis 11 gleichen oder verschiedenen Halogenatomen,jeweils geradkettiges oder verzweigtes C₁-C₆-Alkylamino-C₁-C₆-alkyl, Di-(C₁-C₆-alkyl)amino-C₁-C₆-alkyl, C₁-C₆-Alkoxy-C₁-C₆-alkyl oder C₁-C₆-Halogenalkoxy-C₁-C₆-alkyl, geradkettiges oder verzweigtes Phenyl-(C₁-C₆)alkyl, welches im Phenylteil unsubstituiert oder einfach oder mehrfach unabhängig voneinander gleich oder verschieden substituiert sein kann mit Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy oder für einen fünf- bis siebengliedrigen gesättigten oder teilweise ungesättigten substituierten oder unsubstituierten Ring, gegebenenfalls enthaltend bis zu drei Heteroatome ausgewählt aus Stickstoff, Sauerstoff oder Schwefel, mit der Maßgabe dass zwei Sauerstoffatome nicht benachbart sein dürfen;
- R⁵: steht für NR⁶R⁷, Wasserstoff, geradkettiges oder verzweigtes C₁-C₆-Halogenalkyl mit jeweils 1 bis 11 gleichen oder verschiedenen Halogenatomen, jeweils geradkettiges oder verzweigtes C₁-C₆-Alkyl, C₂-C₆-Alkenyl oder C₃-C₇-Cycloalkyl, jeweils geradkettiges oder verzweigtes C₁-C₆-Alkoxy, C₂-C₆-Alkenyloxy, C₂-C₆-Alkinyloxy oder C₃-C₇-Cycloalkoxy, wobei R⁵, falls es ungleich NR⁶R⁷ oder Wasserstoff ist, jeweils unsubstituiert oder einfach oder mehrfach unabhängig voneinander gleich oder verschieden substituiert sein kann durch Cyano, Oxo, Nitro, C₁-C₄-Alkoxycarbonyl, C₁-C₄-Alkylcarbonyl, Di-(C₁-C₄- Alkyl)aminocarbonyl oder durch einen gesättigten oder teilweise ungesättigten drei- bis siebengliedrigen Ring, gegebenenfalls enthaltend bis zu drei Heteroatome ausgewählt aus Stickstoff, Sauerstoff oder Schwefel mit der Maßgabe, dass zwei Sauerstoffatome nicht benachbart sein dürfen, wobei der Ring selbst gegebenenfalls einfach oder mehrfach gleich oder verschieden unabhängig voneinander durch Fluor, C₁-C₆-Alkyl oder C₁-C₆-Halogenalkyl substituiert sein kann;
oder
- R⁵: steht für jeweils geradkettiges oder verzweigtes C₁-C₆-Halogenalkoxy oder C₂-C₆-Halogenalkenyloxy mit jeweils 1 bis 11 gleichen oder verschiedenen Halogenatomen,jeweils geradkettiges oder verzweigtes C₁-C₆-Alkylamino-C₁-C₆-alkoxy, Di-(C₁-C₆-alkyl)amino-C₁-C₆-alkoxy, C₁-C₆-Alkoxy-C₁-C₆-alkoxy oder C₁-C₆-Halogenalkoxy-C₁-C₆-alkoxy, geradkettiges oder verzweigtes Phenyl-(C₁-C₆)alkoxy, welches im Phenylteil unsubstituiert oder einfach oder mehrfach unabhängig voneinander gleich oder verschieden mit Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy substituiert sein kann,
oder
- R⁵: ist gleich Phenoxy, welches einfach bis fünffach unabhängig voneinander gleich oder verschiedenen durch folgende Reste substituiert sein kann:
Wasserstoff, Fluor, Chlor, Brom, Jod, Cyano, Nitro, Amino, Hydroxy, Mercapto, Formyl, Carboxy, Carbamoyl, Thiocarbamoyl,
jeweils geradkettiges oder verzweigtes C₁-C₆-Alkyl, C₁-C₆-Alkoxy, C₁-C₆-Alkylthio, C₁-C₆-Alkylsulfinyl oder C₁-C₆-Alkylsulfonyl,
jeweils geradkettiges oder verzweigtes C₂-C₆-Alkenyl, C₂-C₆-Alkenylamino oder C₂-C₆-Alkenyloxy,
jeweils geradkettiges oder verzweigtes C₂-C₆-Alkinyl, C₂-C₆-Alkinylamino oder C₂-C₆-Alkinyloxy,
jeweils geradkettiges oder verzweigtes C₁-C₆-Halogenalkyl, C₁-C₆-Halogenalkoxy, C₁-C₇-Halogenalkylcarbonyl, C₁-C₆-Halogenalkylthio, C₁-C₆-Halogenalkylsulfinyl oder C₁-C₆-Halogenalkylsulfonyl mit jeweils 1 bis 13 gleichen oder verschiedenen Halogenatomen,
jeweils geradkettiges oder verzweigtes C₂-C₆-Halogenalkenyl oder C₂-C₆-Halogenalkenyloxy mit jeweils 1 bis 11 gleichen oder verschiedenen Halogenatomen oder geradkettiges oder verzweigtes C₂-C₆-Halogenalkinyloxy,
jeweils geradkettiges oder verzweigtes C₁-C₆-Alkylamino, Di-(C₁-C₆-alkyl)amino, C₁-C₆-Alkylcarbonyl, C₁-C₆-Alkylcarbonyloxy, C₁-C₆-Alkylcarbonylamino, C₁-C₆-Alkoxycarbonyl, C₁-C₆-Alkoxycarbonyloxy, C₁-C₆-Alkoxycarbonylamino, C₁-C₆-Alkylaminocarbonyl, C₁-C₆-Alkylaminocarbonyloxy, C₁-C₆-Alkylaminosulfonyl, Di-(C₁-C₆-alkyl)aminocarbonyl, Di-(C₁-C₆-alkyl)aminocarbonyloxy, Di-(C₁-C₆-alkyl)aminosulfonyl, C₁-C₆-Alkylsulfonylamino, C₁-C₆-Alkylsulfonyloxy, Hydroximino-C₁-C₆-alkyl oder C₁-C₆-Alkoximino-C₁-C₆-alkyl,
unsubstituiertes oder in den Alkylteilen einfach oder mehrfach substituiertes C₃-C₈-Cycloalkyl, C₃-C₈-Cycloalkylamino, C₃-C₈-Cycloalkylaminocarbonyl, C₃-C₈-Cycloalkylaminocarbonyloxy, wobei die Substituenten unabhängig voneinander gleich oder verschieden sein können ausgewählt aus Fluor, Chlor, Methyl, Cyclopropyl oder Trifluormethyl,
jeweils geradkettiges oder verzweigtes C₁-C₆-Alkoxy-C₁-C₆-alkyl, C₁-C₆-Halogenalkoxy-C₁-C₆-alkyl, C₁-C₆-Alkylamino-C₁-C₆-alkyl, Di-(C₁-C₆-alkyl)amino-C₁-C₆-alkyl,
jeweils geradkettiges oder verzweigtes C₁-C₆-Alkoxy-C₁-C₆-alkoxy, C₁-C₆-Alkoxy-C₁-C₆-alkoxy-C₁-C₆-alkoxy, C₁-C₆-Halogenalkoxy-C₁-C₆-alkoxy, C₁-C₆-Alkylamino-C₁C₆-alkoxy, Di-(C₁-C₆-alkyl)amino-C₁C₆-alkoxy,
unsubstituiertes oder einfach bis fünffach substituiertes Phenyl, wobei die Substituenten unabhängig voneinander gleich oder verschieden sein können ausgewählt aus Fluor, Chlor, Methyl, Trifluormethyl, N-Methoxyethanimidoyl, (Methoxyimino)methyl,
- oder: zwei benachbarte Reste am Phenoxyring bilden zusammen einen fünf- bis siebengliedrigen gesättigten, teilweise gesättigten oder ungesättigten substituierten oder unsubstituierten Ring ggf. enthaltend bis zu zwei Heteroatome ausgewählt aus Stickstoff, Sauerstoff oder Schwefel, mit der Maßgabe dass zwei Sauerstoffatome nicht benachbart sein dürfen und wobei die Substituenten, unabhängig voneinander gleich oder verschieden sein können ausgewählt aus Fluor, Chlor, Methyl, Cyclopropyl oder Trifluormethyl;
oder
- R⁴ und R⁵: bilden zusammen einen drei bis zwölf Ringatome enthaltenden gesättigten oder teilweise ungesättigten substituierten oder unsubstituierten monocyclischen oder bicyclischen Ring gegebenenfalls enthaltend bis zu zwei zusätzliche Heteroatome ausgewählt aus Stickstoff, Sauerstoff, Schwefel oder Silizium, mit der Maßgabe dass zwei Sauerstoffatome nicht benachbart sein dürfen;
- R⁶ und R⁷: stehen unabhängig voneinander gleichartig oder verschieden für R³, C₁-C₆-Alkyl, C₃-C₇-Cycloalkyl, C₁-C₆-Alkoxy, C₁-C₆-Alkylcarbonyl, C₁-C₆-Alkoxycarbonyl, C₁-C₆-Alkylaminocarbonyl, Di-(C₁-C₆-Alkyl)-aminocarbonyl oder geradkettiges oder verzweigtes Phenyl-(C₁-C₆)alkyl, welches im Phenylteil unsubstituiert oder einfach oder mehrfach unabhängig voneinander gleich oder verschieden mit Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy substituiert sein kann,
oder
- R⁶ und R⁷: bilden zusammen einen drei bis zwölf Ringatome enthaltenden gesättigten oder teilweise ungesättigten substituierten oder unsubstituierten monocyclischen oder bicyclischen Ring gegebenenfalls enthaltend bis zu zwei zusätzliche Heteroatome ausgewählt aus Stickstoff, Sauerstoff, Schwefel oder Silizium, mit der Maßgabe dass zwei Sauerstoffatome nicht benachbart sein dürfen,
- R⁸: steht für R³, NR⁶R⁷, jeweils geradkettiges oder verzweigtes C₁-C₈-Alkyl, C₂-C₈-Alkenyl, C₂-C₈-Alkinyl, C₃-C₇-Cycloalkyl oder C₃-C₇-Cycloalkenyl, wobei R⁸ für den Fall, dass es ungleich R³ und NR⁶R⁷ ist, jeweils unsubstituiert oder einfach oder mehrfach unabhängig voneinander gleich oder verschieden substituiert sein kann durch Cyano, Oxo, Nitro, C₁-C₄-Alkoxycarbonyl, C₁-C₄-Alkylcarbonyl, Di-(C₁-C₄-alkyl)aminocarbonyl oder durch einen gesättigten oder teilweise ungesättigten drei- bis siebengliedrigen Ring, gegebenenfalls enthaltend bis zu drei Heteroatome ausgewählt aus Stickstoff, Sauerstoff oder Schwefel mit der Maßgabe dass zwei Sauerstoffatome nicht benachbart sein dürfen, wobei der Ring selbst gegebenenfalls einfach oder mehrfach unabhängig voneinander gleich oder verschieden durch Fluor, C₁-C₆-Alkyl oder C₁-C₆-Halogenalkyl substituiert sein kann,
oder
- R⁸: steht für jeweils geradkettiges oder verzweigtes C₁-C₆-Halogenalkyl oder C₂-C₆-Halogenalkenyl mit jeweils 1 bis 11 gleichen oder verschiedenen Halogenatomen, jeweils geradkettiges oder verzweigtes C₁-C₆Alkylamino-C₁-C₆-alkyl, Di-(C₁-C₆-alkyl)amino-C₁-C₆-alkyl, C₁-C₆-Alkoxy-C₁-C₆-alkyl, C₁-C₆-Halogenalkoxy-C₁-C₆-alkyl oder geradkettiges oder verzweigtes Phenyl-(C₁-C₆)alkyl, welches im Phenylteil unsubstituiert oder einfach oder mehrfach unabhängig voneinander gleich oder verschieden mit Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy substituiert sein kann oder für einen fünf- bis siebengliedrigen gesättigten oder teilweise ungesättigten substituierten oder unsubstituierten Ring, gegebenenfalls enthaltend bis zu drei Heteroatome ausgewählt aus Stickstoff, Sauerstoff oder Schwefel, mit der Maßgabe dass zwei Sauerstoffatome nicht benachbart sein dürfen,
- R⁹: steht für Formyl, geradkettiges oder verzweigtes Tri-(C₁-C₆-alkyl)silyl, jeweils geradkettiges oder verzweigtes C₁-C₆-Alkylcarbonyl, C₃-C₇-Cycloalkylcarbonyl, C₁-C₆-Alkoxycarbonyl, C₁-C₆-Alkylaminocarbonyl, Di-(C₁-C₆-Alkyl)aminocarbonyl, C₁-C₆-Alkylaminothiocarbonyl, Di-(C₁-C₆-alkyl)aminothiocarbonyl, C₁-C₆-Alkylsulfinyl oder C₁-C₆-Alkylsulfonyl, wobei R⁹ für den Fall, dass es ungleich Formyl ist, jeweils unsubstituiert oder einfach oder mehrfach unabhängig voneinander gleich oder verschieden substituiert sein kann durch Fluor, Chlor, Cyano, C₁-C₄-Alkoxy oder C₁-C₄-Alkyl,
oder
- R⁹: steht für jeweils geradkettiges oder verzweigtes C₁-C₆-Halogenalkylcarbonyl, C₁-C₆-Halogenalkylsulfinyl oder C₁-C₆-Halogenalkylsulfonyl mit jeweils 1 bis 13 gleichen oder verschiedenen Halogenatomen, jeweils unsubstituiertes oder einfach oder mehrfach substituiertes Phenylcarbonyl, Phenylacetyl, Phenoxycarbonyl, Phenylaminocarbonyl, Phenylaminothiocarbonyl, Phenylsulfinyl, Phenylsulfonyl, Heteroarylcarbonyl, oder Heteroarylaminocarbonyl, wobei die Substituenten im Phenylteil oder Heteroarylteil unabhängig voneinander gleichartig oder verschieden sein können ausgewählt aus Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, oder Trifluormethoxy,
- R¹⁰: steht für jeweils geradkettiges oder verzweigtes C₁-C₆-Alkylcarbonyl, C₃-C₇-Cycloalkylcarbonyl, C₁-C₆-Alkoxycarbonyl, C₁-C₆-Alkylaminocarbonyl oder Di-(C₁-C₆-alkyl)aminocarbonyl, welches jeweils unsubstituiert oder einfach oder mehrfach unabhängig voneinander durch Fluor, Chlor, Cyano, C₁-C₄-Alkoxy oder C₁-C₄-Alkyl substituiert sein kann, geradkettiges oder verzweigtes Tri-(C₁-C₆-alkyl)silyl, jeweils unsubstituiertes oder einfach oder mehrfach substituiertes Phenylcarbonyl, Phenylacetyl, Phenylaminocarbonyl, Phenylaminothiocarbonyl oder Heteroarylcarbonyl, wobei die Substituenten im Phenylteil unabhängig voneinander gleichartig oder verschieden sein können ausgewählt aus Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy,
sowie agrochemisch wirksame Salze davon.

Verbindungen der Formel (I) eignen sich sehr gut zur Bekämpfung von unerwünschten Mikroorganismen. Sie zeigen vor allem eine starke fungizide Wirksamkeit und lassen sich sowohl im Pflanzenschutz als auch im Materialschutz verwenden.

Die Verbindungen der Formel (I) können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen Verbindungen sind durch die Formel (I) allgemein definiert.
- R¹: steht bevorzugt für Wasserstoff, Cyclopropylmethyl, Cyclopropylethyl, Cyclobutylmethyl, Cyclobutylethyl, Allyl, But-3-enyl, Propargyl, But-2-inyl, Methoxymethyl, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2,2-Trifluorethyl, jeweils geradkettiges oder verzweigtes C₁-C₆-Alkyl oder C₃-C₇-Cycloalkyl, welches jeweils durch einen bis drei gleiche oder verschiedene Reste substituiert sein kann ausgewählt aus Halogen, Cyano, Oxo, C₁-C₂-Alkylamino, Di-(C₁-C₂-)alkylamino, C₁-C₃-Alkyl oder C₁-C₃-Alkoxy.
- R¹: steht besonders bevorzugt für Wasserstoff, Methyl, Ethyl, n-Propyl, Isopropyl, Cyclopropyl, n-Butyl, Isobutyl, sek-Butyl, tert-Butyl, Cyclobutyl, Cyclopentyl, Cyclopropylmethyl, Cyclopropylethyl, Allyl, But-3-enyl, Propargyl, But-2-inyl, Methoxymethyl, Methoxyethyl, Ethoxymethyl, Ethoxyethyl, Cyanoethyl, Chlordifluormethyl, Dichlorfluormethyl, Chlorfluormethyl, Fluormethyl, Difluormethyl, Trifluormethyl oder 2,2,2-Trifluorethyl.
- R¹: steht ganz besonders bevorzugt für Wasserstoff, Methyl, Ethyl, n-Propyl, Isopropyl, Cyclopropyl, Cyclopropylmethyl, n-Butyl, Allyl, Propargyl, Methoxymethyl, Fluormethyl, Difluormethyl, Trifluormethyl oder 2,2,2-Trifluorethyl.
- R¹: steht insbesondere bevorzugt für Methyl.
- R²: steht bevorzugt für Fluor, Chlor oder Brom, Cyano, Propargyloxy, Methylsulfinyl, Ethylsulfinyl, Methylsulfonyl, Ethylsulfonyl, Trifluormethansulfonyl, Fluormethoxy, Difluormethoxy, Trifluormethoxy, Trifluormethylthio, 2,2,2-Trifluorethoxy, C₁-C₄-Alkoxy, C₃-C₆-Alkenyloxy oder C₁-C₄-Alkylthio, wobei die genannten Reste durch einen bis drei gleiche oder verschiedene Reste ausgewählt aus C₁-C₂-Alkylamino, C₁-C₂-Dialkylamino, Cyano, Oxo, C₁-C₄-Alkyl, C₁-C₄-Alkoxy oder unsubstituiertem oder durch Halogen, C₁-C₄-Alkyl oder C₁-C₄-Halogenalkyl substituiertem C₃-C₆-Cycloalkyl substituiert sein können, oder
jeweils unsubstituiertes oder im Phenylteil ein- bis zweifach substituiertes Phenoxy oder Benzyloxy, wobei die Substituenten im Phenylteil unabhängig voneinander gleichartig oder verschieden sein können ausgewählt aus Fluor, Chlor, Brom, Cyano, Nitro, Methyl, Methoxy, Dimethylamino, Trifluormethyl oder Trifluormethoxy.
- R²: steht besonders bevorzugt für Fluor, Chlor, Brom, Cyano, Methoxy, Ethoxy, n-Propoxy, Isopropoxy, n-Butoxy, Methoxyethoxy, Allyloxy, Propargyloxy, Phenoxy, 4-Chlorphenoxy, 4-Chlorbenzyloxy Methylthio, Ethylthio, n-Propylthio, Isopropylthio, Methylsulfinyl, Ethylsulfinyl, Methylsulfonyl, Ethylsulfonyl, Fluormethoxy, Difluormethoxy, Trifluormethoxy, Trifluormethylthio oder 2,2,2-Trifluorethoxy.
- R²: steht ganz besonders bevorzugt für Fluor, Chlor, Brom, Cyano, Methoxy, Ethoxy, 2,2,2-Trifluorethoxy, Methylthio, Ethylthio, Methylsulfinyl, Ethylsulfinyl, Methylsulfonyl oder Ethylsulfonyl.
- R²: steht insbesondere bevorzugt für Chlor, Brom oder Methoxy.
- R³: steht bevorzugt für einen Phenylring oder einen ungesättigten Heterocyclus mit fünf bis sechs Ringgliedern und ein bis vier Heteroatomen ausgewählt aus der Gruppe Stickstoff, Sauerstoff und Schwefel, mit der Maßgabe dass zwei Sauerstoffatome nicht benachbart sein dürfen, gegebenenfalls annelliert mit einem weiteren fünf- bis sechsgliedrigen gesättigten oder ganz oder teilweise ungesättigten carbocyclischen oder heterocyclischen Ring, wobei der zusätzliche heterocyclische Ring gegebenenfalls ein bis zwei zusätzliche Heteroatome ausgewählt aus der Gruppe Stickstoff, Sauerstoff und Schwefel enthalten kann, mit der Maßgabe dass zwei Sauerstoffatome nicht benachbart sein dürfen;
welche jeweils einfach oder mehrfach unabhängig voneinander durch folgende gleiche oder verschiedenene Reste substituiert sein können:
Wasserstoff, Fluor, Chlor, Brom, Jod, Cyano, Nitro, Amino, Hydroxy, Mercapto, Formyl, Carboxy, Carbamoyl, Thiocarbamoyl, Methylsulfinyl, Ethylsulfinyl, Methylsulfonyl, Ethylsulfonyl, Methylaminosulfonyl, Ethylaminosulfonyl, Methylethylaminosulfonyl, Dimethylaminosulfonyl, Diethylaminosulfonyl, Methylsulfonylamino, Ethylsulfonylamino, Methylsulfonyloxy, Ethylsulfonyloxy, Trifluormethylsulfinyl, 2,2,2-Trifluorethylsulfinyl, Trifluormethylsulfonyl, 2,2,2-Trifluorethylsulfonyl, Trifluorethinyloxy, Chlorallyloxy;
jeweils geradkettiges oder verzweigtes C₁-C₆-Alkyl, C₁-C₆-Alkoxy oder C₁-C₆-Alkylthio,
jeweils geradkettiges oder verzweigtes C₂-C₆-Alkenyl, C₂-C₆-Alkenylamino oder C₂-C₆-Alkenyloxy,
jeweils geradkettiges oder verzweigtes C₂-C₆-Alkinyl, C₂-C₆-Alkinylamino oder C₂-C₆-Alkinyloxy,
jeweils geradkettiges oder verzweigtes C₁-C₆-Halogenalkyl, C₁-C₆-Halogenalkoxy, C₁-C₇-Halogenalkylcarbonyl oder C₁-C₆-Halogenalkylthio mit jeweils 1 bis 13 gleichen oder verschiedenen Halogenatomen,
jeweils geradkettiges oder verzweigtes C₁-C₆-Alkylamino, Di-(C₁-C₆-alkyl)amino, C₁-C₆-Alkylcarbonyl, C₁-C₆-Alkylcarbonyloxy, C₁-C₆-Alkylcarbonylamino, C₁-C₆-Alkoxycarbonyl, C₁-C₆-Alkoxycarbonyloxy, C₁-C₆-Alkoxycarbonylamino, C₁-C₆-Alkylaminocarbonyl, C₁-C₆-Alkylaminocarbonyloxy, Di-(C₁-C₆-alkyl)aminocarbonyl, Di-(C₁-C₆-alkyl)aminocarbonyloxy, Hydroximino-C₁-C₆-alkyl oder C₁-C₆-Alkoximino-C₁-C₆-alkyl,
unsubstituiertes oder in den Alkylteilen einfach oder mehrfach substituiertes C₃-C₈-Cycloalkyl, C₃-C₈-Cycloalkylamino, C₃-C₈-Cycloalkylaminocarbonyl, C₃-C₈-Cycloalkylaminocarbonyloxy, wobei die Substituenten unabhängig voneinander gleich oder verschieden sein können ausgewählt aus Fluor, Chlor, Methyl, Cyclopropyl oder Trifluormethyl,
jeweils geradkettiges oder verzweigtes C₁-C₆-Alkoxy-C₁-C₆-alkyl, C₁-C₆-Halogenalkoxy-C₁-C₆-alkyl, C₁-C₆-Alkylamino-C₁-C₆-alkyl, Di-(C₁-C₆-alkyl)amino-C₁-C₆-alkyl,
jeweils geradkettiges oder verzweigtes C₁-C₄-Alkoxy-C₁-C₄-alkoxy, C₁-C₄-Alkoxy-C₁-C₄-alkoxy-C₁-C₄-alkoxy, C₁-C₄-Halogenalkoxy-C₁-C₄-alkoxy, C₁-C₄-Alkylamino-C₁C₄-alkoxy, Di-(C₁-C₄-alkyl)amino-C₁C₄-alkoxy,
unsubstituiertes oder einfach bis dreifach substituiertes Phenyl, wobei die Substituenten unabhängig voneinander gleich oder verschieden sein können ausgewählt aus Fluor, Chlor, Methyl, Trifluormethyl, N-Methoxyethanimidoyl, (Methoxyimino)methyl,
- oder: zwei benachbarte Reste am Phenylring oder am Heterocyclus bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl;
- oder: zwei Reste am Heterocyclus bilden mit dem Kohlenstoffatom, an das sie gebundenen sind, eine Carbonyl- oder Thiocarbonylgruppe.
- R³: steht besonders bevorzugt für einen Phenylring, welcher einfach bis fünffach unabhängig voneinander gleich oder verschiedenen durch folgende Reste substituiert sein kann:
Wasserstoff, Fluor, Chlor, Brom, Iod, Cyano, Nitro, Hydroxy, Mercapto, Amino, Formyl, Methyl, Ethyl, n-Propyl, Isopropyl, n-Butyl, Isobutyl, sek-Butyl, tert-Butyl, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2-Dichlor-2-fluoroethyl, 2-Chlor-2,2-difluorethyl, 2-Chlor-2-fluorethyl, 2,2,2-Trichlorethyl, 2-Fluorethyl, 2,2-Difluorethyl, 2,2,2-Trifluorethyl, Vinyl, Ethinyl, Allyl, Propargyl, Methoxy, Ethoxy, n-oder i-Propoxy, Methylthio, Ethylthio, n-Propylthio, Isopropylthio, Methylsulfinyl, Ethylsulfinyl, Methylsulfonyl, Ethylsulfonyl, Allyloxy, Propargyloxy, Difluormethoxy, Trifluormethoxy, Difluorchlormethoxy, 2,2,2-Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Methylamino, Ethylamino, n-Propylamino, Isopropylamino, Cyclopropylamino, Dimethylamino, Diethylamino, Acetyl, Propionyl, Acetyloxy, Methoxycarbonyl, Ethoxycarbonyl, Methoxymethyl, Ethoxymethyl, 2-Methoxyethoxy, 2-Ethoxyethoxy, (2-Methoxyethoxy)ethoxy, 2-(Dimethylamino)ethoxy, 2-Morpholin-4-ylethoxy, 3-(Dimethylamino)propoxy, 3-Morpholin-4-ylpropoxy, Hydroximinomethyl, Hydroximinoethyl, Methoximinomethyl, Ethoximinomethyl, N-Methoxyethanimidoyl, N-Ethoxyethanimidoyl, Cyclopropyl, Cyclobutyl, Cyclopentyl, Cyclohexyl, Phenyl oder Phenoxy;
- oder: zwei benachbarte Reste am Phenylring bilden besonders bevorzugt zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl;
oder
- R³: steht besonders bevorzugt für 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4- Triazol-1-yl, 1,2,4-Triazol-3-yl, 1,2,4-Triazol-4-yl, 1,2,4-Triazol-5-yl, 1,2,3-Triazol-1-yl, 1,2,3-Triazol-2-yl, 1,2,3-Triazol-4-yl, Tetrazol-1-yl, Tetrazol-2-yl, Tetrazol-5-yl, Indol-1-yl, Indol-2-yl, Indol-3-yl, Isoindol-1-yl, Isoindol-2-yl, Benzofur-2-yl, Benzothiophen-2-yl, Benzofur-3-yl, Benzothiophen-3-yl, Benzoxazol-2-yl, Benzothiazol-2-yl, Benzimidazol-2-yl, Indazol-1-yl, Indazol-2-yl, Indazol-3-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl oder 1,2,4-Triazin-6-yl, welches jeweils einfach oder mehrfach unabhängig voneinander gleich oder verschieden durch folgende Reste substituiert sein kann ausgewählt aus Wasserstoff, Fluor, Chlor, Brom, Iod, Cyano, Hydroxy, Mercapto, Amino, Methyl, Ethyl, n-Propyl, Isopropyl, n-Butyl, Isobutyl, sek-Butyl, tert-Butyl, Cyclopropyl, 1-Chlorcyclopropyl, Vinyl, Ethinyl, Methoxy, Ethoxy, Isopropoxy, Methylthio, Ethylthio, Trifluormethylthio, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2,2-Trifluorethyl, Trifluormethoxy, Trifluormethylthio, 2,2,2-Trifluorethoxy, 2,2-Dichlor-2-fluorethyl, 2,2-Difluor-2-chlorethyl, 2-Chlor-2-fluorethyl, 2,2,2-Trichlorethyl, 2,2,2-Trifluorethyl, 2-Fluorethyl, 2,2-Difluorethyl, 2-Methoxyethoxy, Acetyl, Propionyl, Methoxycarbonyl, Ethoxycarbonyl, N-Methylamino, N,N-Dimethylamino, N-Ethylamino, N,N-Diethylamino, Aminocarbonyl, Methylaminocarbonyl, Dimethylaminocarbonyl, Dimethylcarbamoylamino, Methoxycarbonylamino, Methoxycarbonyloxy, Ethoxycarbonylamino, Ethoxycarbonyloxy, Methylsulfamoyl, Dimethylsulfamoyl, Phenyl oder Phenoxy,
- oder: zwei benachbarte Reste am Heterocyclus bilden besonders bevorzugt zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl,
- oder: zwei Reste am Heterocyclus bilden besonders bevorzugt mit dem Kohlenstoffatom, an das sie gebundenen sind, eine Carbonyl- oder Thiocarbonylgruppe.
- R³: steht ganz besonders bevorzugt für einen Phenylring, welcher einfach bis dreifach unabhängig voneinander gleich oder verschieden durch folgende Reste substituiert sein kann:
Chlor, Brom, Iod, Fluor, Fluormethyl, Difluormethyl, Trifluormethyl, Trifluormethoxy, Trifluormethylthio, Hydroxy, Methoxy, Methoxyethoxy, Nitro, Cyano, Methyl, Ethyl, Amino, Methylamino, Dimethylamino, 2-(Dimethylamino)ethoxy, 2-Morpholin-4-ylethoxy, 3-(Dimethylamino)propoxy oder 3-Morpholin-4-ylpropoxy,

- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach, unabhängig voneinander gleich oder verschieden substituiert sein können durch Fluor, Chlor, Methyl, Cyclopropyl und/oder Trifluormethyl;
oder
- R³: steht ganz besonders bevorzugt für 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4-Triazol-1-yl, 1,2,4-Triazol-3-yl, 1,2,4-Triazol-4-yl, 1,2,4-Triazol-5-yl, 1,2,3-Triazol-1-yl, 1,2,3-Triazol-2-yl, 1,2,3-Triazol-4-yl, Tetrazol-1-yl, Tetrazol-2-yl, Tetrazol-5-yl, Indol-1-yl, Indol-2-yl, Indol-3-yl, Isoindol-1-yl, Isoindol-2-yl, Benzofur-2-yl, Benzothiophen-2-yl, Benzofur-3-yl, Benzothiophen-3-yl, Benzoxazol-2-yl, Benzothiazol-2-yl, Benzimidazol-2-yl, Indazol-1-yl, Indazol-2-yl, Indazol-3-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl oder 1,2,4-Triazin-6-yl, welches jeweils einfach oder mehrfach unabhängig voneinander gleich oder verschieden durch folgende Reste substituiert sein kann ausgewählt aus Wasserstoff, Fluor, Chlor, Brom, Iod, Cyano, Hydroxy, Mercapto, Amino, Methyl, Ethyl, n-Propyl, Isopropyl, n-Butyl, Isobutyl, sek-Butyl, tert-Butyl, Cyclopropyl, 1-Chlorcyclopropyl, Vinyl, Ethinyl, Methoxy, Ethoxy, Isopropoxy, Methylthio, Ethylthio, Trifluormethylthio, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2,2-Trifluorethyl, Trifluormethoxy, Trifluormethylthio, 2-Methoxyethoxy, Acetyl, Propionyl, Methoxycarbonyl, N-Methylamino, N,N-Dimethylamino, Aminocarbonyl, Methylaminocarbonyl, Dimethylaminocarbonyl, Dimethylcarbamoylamino, Methoxycarbonylamino, Methoxycarbonyloxy, Methylsulfamoyl, Dimethylsulfamoyl, Phenyl oder Phenoxy,
- oder: zwei benachbarte Reste am Heterocyclus bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl,
- oder: zwei Reste am Heterocyclus bilden ganz besonders bevorzugt mit dem Kohlenstoffatom, an das sie gebundenen sind, eine Carbonyl- oder Thiocarbonylgruppe,
- R³: steht weiterhin ganz besonders bevorzugt für einen Phenylring, welcher in 2-Position monosubstituiert, in 2,4-, 2,5- oder 2,6-Position bissubstituiert oder in 2,4,6-, 2,3,4- oder 2,4,5-Position trisubstituiert ist unabhängig voneinander durch folgende gleiche oder verschiedene Reste substituiert sein kann:
Chlor, Brom, Fluor, Trifluormethyl, Trifluormethoxy, Trifluormethylthio, Hydroxy, Methoxy, Methoxyethoxy, Nitro, Cyano, Methyl, Amino, Methylamino, Dimethylamino, 2-(Dimethylamino)ethoxy, 2-Morpholin-4-ylethoxy, 3-(Dimethylamino)propoxy oder 3-Morpholin-4-ylpropoxy;
- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach, unabhängig voneinander gleich oder verschieden substituiert sein können durch Fluor, Chlor, Methyl, Cyclopropyl und/oder Trifluormethyl;
oder
- R³: steht weiterhin ganz besonders bevorzugt 2-Thienyl, 3-Thienyl, 2-Thiazolyl, 4-Thiazolyl, 2-Pyridinyl, 4-Pyridinyl, 3-Pyridazinyl, 2-Pyrazinyl, 2-Pyrimidinyl, 4-Pyrimidinyl, welches jeweils einfach bis dreifach unabhängig voneinander durch gleiche oder verschiedenene Reste substituiert sein kann ausgewählt aus Fluor, Chlor, Brom, Methyl, Methoxy, Trifluormethyl, Trifluormethoxy, Trifluormethylthio, 2-Methoxyethoxy, Acetyl, Methoxycarbonyl, N,N-Dimethylamino, Methoxycarbonylamino,
- oder: zwei benachbarte Reste am Heterocyclus bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl,
- oder: zwei Reste am Heterocyclus bilden ganz besonders bevorzugt mit dem Kohlenstoffatom, an das sie gebundenen sind, eine Carbonyl- oder Thiocarbonylgruppe.
- R³: steht insbesondere bevorzugt für 2,6-Difluorphenyl oder 2-Chlor-6-Fluorphenyl.
- R⁴: steht bevorzugt für R³, Wasserstoff oder jeweils geradkettiges oder verzweigtes C₁-C₈-Alkyl, C₂-C₈-Alkenyl, C₂-C₆-Alkinyl oder C₃-C₇-Cycloalkyl, wobei R⁴, für den Fall, dass es ungleich R³ oder Wasserstoff ist, jeweils unsubstituiert oder einfach oder mehrfach gleich oder verschieden unabhängig voneinander substituiert sein kann durch Cyano, Oxo, Nitro, C₁-C₄-Alkoxycarbonyl, C₁-C₄-Alkylcarbonyl, Di-(C₁-C₄-Alkyl)aminocarbonyl oder einen gesättigten oder teilweise ungesättigten drei- bis siebengliedrigen Ring, gegebenenfalls enthaltend bis zu drei Heteroatome ausgewählt aus Stickstoff, Sauerstoff oder Schwefel, mit der Maßgabe dass zwei Sauerstoffatome nicht benachbart sein dürfen, wobei der Ring selbst gegebenenfalls einfach oder mehrfach gleich oder verschieden unabhängig voneinander durch Fluor, C₁-C₄-Alkyl oder C₁-C₄-Halogenalkyl substituiert sein kann,
oder
- R⁴: steht für jeweils geradkettiges oder verzweigtes C₁-C₆-Halogenalkyl oder C₂-C₆-Halogenalkenyl mit jeweils 1 bis 11 gleichen oder verschiedenen Halogenatomen, jeweils geradkettiges oder verzweigtes C₁-C₆-Alkylamino-C₁-C₆-alkyl, Di-(C₁-C₆-alkyl)amino-C₁-C₆-alkyl, C₁-C₆-Alkoxy-C₁-C₆-alkyl oder C₁-C₆-Halogenalkoxy-C₁-C₆-alkyl, geradkettiges oder verzweigtes Phenyl-(C₁-C₄)alkyl, welches im Phenylteil unsubstituiert oder einfach oder mehrfach unabhängig voneinander gleich oder verschieden substituiert sein kann mit Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl oder Trifluormethoxy oder für einen fünf- bis siebengliedriger gesättigten oder teilweise ungesättigten substituierten oder unsubstituierten Ring, gegebenenfalls enthaltend bis zu drei Heteroatome ausgewählt aus Stickstoff, Sauerstoff oder Schwefel, mit der Maßgabe dass zwei Sauerstoffatome nicht benachbart sein dürfen.
- R⁵: steht bevorzugt für NR⁶R⁷, Wasserstoff, geradkettiges oder verzweigtes C₁-C₆-Halogenalkyl mit jeweils 1 bis 11 gleichen oder verschiedenen Halogenatomen, jeweils geradkettiges oder verzweigtes C₁-C₆-Alkyl, C₂-C₆-Alkenyl oder C₃-C₇-Cycloalkyl, jeweils geradkettiges oder verzweigtes C₁-C₆-Alkoxy, C₂-C₆-Alkenyloxy, C₂-C₆-Alkinyloxy oder C₃-C₇-Cycloalkoxy, wobei R⁵, falls es ungleich NR⁶R⁷ oder Wasserstoff ist, jeweils unsubstituiert oder einfach oder mehrfach unabhängig voneinander gleich oder verschieden substituiert sein kann durch Cyano, Oxo, Nitro, C₁-C₄-Alkoxycarbonyl, C₁-C₄-Alkylcarbonyl, Di-(C₁-C₄-Alkyl)aminocarbonyl oder durch einen gesättigten oder teilweise ungesättigten drei- bis siebengliedrigen Ring, gegebenenfalls enthaltend bis zu drei Heteroatome ausgewählt aus Stickstoff, Sauerstoff oder Schwefel mit der Maßgabe dass zwei Sauerstoffatome nicht benachbart sein dürfen, wobei der Ring selbst gegebenenfalls einfach oder mehrfach gleich oder verschieden unabhängig voneinander durch Fluor, C₁-C₆-Alkyl oder C₁-C₆-Halogenalkyl substituiert sein kann,
oder
- R⁵: steht für jeweils geradkettiges oder verzweigtes C₁-C₆-Halogenalkoxy oder C₂-C₆-Halogenalkenyloxy mit jeweils 1 bis 11 gleichen oder verschiedenen Halogenatomen, jeweils geradkettiges oder verzweigtes C₁-C₆₋Alkylamino-C₁-C₆-alkoxy, Di-(C₁-C₆-alkyl)amino-C₁-C₆-alkoxy, C₁-C₆-Alkoxy-C₁-C₆-alkoxy oder C₁-C₆-Halogenalkoxy-C₁-C₆-alkoxy, geradkettiges oder verzweigtes Phenyl-(C₁-C₆)alkoxy, welches im Phenylteil unsubstituiert oder einfach oder mehrfach unabhängig voneinander gleich oder verschieden mit Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy substituiert sein kann,
oder
- R⁵: steht bevorzugt für einen Phenoxyring, welcher einfach bis fünffach unabhängig voneinander gleich oder verschieden durch folgende Reste substituiert sein kann:
Wasserstoff, Fluor, Chlor, Brom, Jod, Cyano, Nitro, Amino, Hydroxy, Mercapto, Formyl, Carboxy, Carbamoyl, Thiocarbamoyl, Methylsulfinyl, Ethylsulfinyl, Methylsulfonyl, Ethylsulfonyl, Methylaminosulfonyl, Ethylaminosulfonyl, Methylethylaminosulfonyl, Dimethylaminosulfonyl, Diethylaminosulfonyl, Methylsulfonylamino, Ethylsulfonylamino, Methylsulfonyloxy, Ethylsulfonyloxy, Trifluormethylsulfinyl, 2,2,2-Trifluorethylsulfinyl, Trifluormethylsulfonyl, 2,2,2-Trifluorethylsulfonyl, Trifluorethinyloxy, Chlorallyloxy,
jeweils geradkettiges oder verzweigtes C₁-C₆-Alkyl, C₁-C₆-Alkoxy oder C₁-C₆-Alkylthio,
jeweils geradkettiges oder verzweigtes C₂-C₆-Alkenyl, C₂-C₆-Alkenylamino oder C₂-C₆-Alkenyloxy,
jeweils geradkettiges oder verzweigtes C₂-C₆-Alkinyl, C₂-C₆-Alkinylamino oder C₂-C₆-Alkinyloxy,
jeweils geradkettiges oder verzweigtes C₁-C₆-Halogenalkyl, C₁-C₆-Halogenalkoxy, C₁-C₇-Halogenalkylcarbonyl oder C₁-C₆-Halogenalkylthio mit jeweils 1 bis 13 gleichen oder verschiedenen Halogenatomen,
jeweils geradkettiges oder verzweigtes C₁-C₆-Alkylamino, Di-(C₁-C₆-alkyl)amino, C₁-C₆-Alkylcarbonyl, C₁-C₆-Alkylcarbonyloxy, C₁-C₆-Alkylcarbonylamino, C₁-C₆-Alkoxycarbonyl, C₁-C₆-Alkoxycarbonyloxy, C₁-C₆-Alkoxycarbonylamino, C₁-C₆-Alkylaminocarbonyl, C₁-C₆-Alkylaminocarbonyloxy, Di-(C₁-C₆-alkyl)aminocarbonyl, Di-(C₁-C₆-alkyl)aminocarbonyloxy, Hydroximino-C₁-C₆-alkyl oder C₁-C₆-Alkoximino-C₁-C₆-alkyl,
unsubstituiertes oder in den Alkylteilen einfach oder mehrfach substituiertes C₃-C₈-Cycloalkyl, C₃-C₈-Cycloalkylamino, C₃-C₈-Cycloalkylaminocarbonyl, C₃-C₈-Cycloalkylaminocarbonyloxy, wobei die Substituenten unabhängig voneinander gleich oder verschieden sein können ausgewählt aus Fluor, Chlor, Methyl, Cyclopropyl oder Trifluormethyl,
jeweils geradkettiges oder verzweigtes C₁-C₆-Alkoxy-C₁-C₆-alkyl, C₁-C₆-Halogenalkoxy-C₁-C₆-alkyl, C₁-C₆-Alkylamino-C₁-C₆-alkyl, Di-(C₁-C₆-alkyl)amino-C₁-C₆-alkyl,
jeweils geradkettiges oder verzweigtes C₁-C₄-Alkoxy-C₁-C₄-alkoxy, C₁-C₄-Alkoxy-C₁-C₄-alkoxy-C₁-C₄-alkoxy, C₁-C₄-Halogenalkoxy-C₁-C₄-alkoxy, C₁-C₄-Alkylamino-C₁C₄-alkoxy, Di-(C₁-C₄-alkyl)amino-C₁C₄-alkoxy,
unsubstituiertes oder einfach bis dreifach substituiertes Phenyl, wobei die Substituenten unabhängig voneinander gleich oder verschieden sein können ausgewählt aus Fluor, Chlor, Methyl, Trifluormethyl, N-Methoxyethanimidoyl, (Methoxyimino)methyl,
- oder: zwei benachbarte Reste am Phenoxyring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl,
oder
- R⁴ und R⁵: bilden zusammen einen drei bis zwölf Ringatome enthaltenden gesättigten oder teilweise ungesättigten substituierten oder unsubstituierten monocyclischen oder bicyclischen Ring ggf. enthaltend bis zu zwei zusätzliche Heteroatome ausgewählt aus Stickstoff, Sauerstoff, Schwefel oder Silizium, mit der Maßgabe dass zwei Sauerstoffatome nicht benachbart sein dürfen.
- R⁴: steht besonders bevorzugt für Wasserstoff, oder Phenyl, 2-Furyl, 3-Furyl, 2-Thienyl, 3-Thienyl, 1-Pyrrolyl, 2-Pyrrolyl, 3-Pyrrolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4-Triazol-1-yl, 1,2,4-Triazol-3-yl, 1,2,4-Triazol-4-yl, 1,2,4-Triazol-5-yl, Indol-1-yl, Indol-2-yl, Indol-3-yl, Isoindol-1-yl, Isoindol-2-yl, Benzofur-2-yl, Benzothiophen-2-yl, Benzofur-3-yl, Benzothiophen-3-yl, Benzoxazol-2-yl, Benzothiazol-2-yl, Benzimidazol-2-yl, Indazol-1-yl, Indazol-2-yl, Indazol-3-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl oder 1,2,4-Triazin-6-yl,welches jeweils einfach oder mehrfach unabhängig voneinander durch gleiche oder verschiedenene Reste substituiert sein kann ausgewählt aus Wasserstoff, Fluor, Chlor, Brom, Iod, Cyano, Hydroxy, Mercapto, Amino, Methyl, Ethyl, n-Propyl, Isopropyl, n-Butyl, Isobutyl, sek-Butyl, tert-Butyl, Cyclopropyl, Vinyl, Ethinyl, Methoxy, Ethoxy, Isopropoxy, Methylthio, Ethylthio, Trifluormethylthio, Difluormethyl, Trifluormethyl, 2,2,2-Trifluorethyl, Trifluormethoxy, Trifluormethylthio, 2,2,2-Trifluorethoxy, 2-Methoxyethoxy, Acetyl, Propionyl, Methoxycarbonyl, Ethoxycarbonyl, N-Methylamino, N,N-Dimethylamino, N-Ethylamino, N,N-Diethylamino, Aminocarbonyl, Methylaminocarbonyl, Dimethylaminocarbonyl, Dimethylcarbamoylamino, Methoxycarbonylamino, Methoxycarbonyloxy, Ethoxycarbonylamino, Ethoxycarbonyloxy, Methylsulfamoyl, Dimethylsulfamoyl, Phenyl oder Phenoxy,
- oder: zwei benachbarte Reste am Phenylring oder am Heterocyclus bilden besonders bevorzugt zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl,
- oder: zwei Reste am Heterocyclus bilden besonders bevorzugt mit dem Kohlenstoffatom, an das sie gebundenen sind, eine Carbonyl- oder Thiocarbonylgruppe;
oder
- R⁴: steht besonders bevorzugt für jeweils geradkettiges oder verzweigtes C₁-C₆-Alkyl, C₂-C₆-Alkenyl, C₂-C₆-Alkinyl oder C₃-C₇-Cycloalkyl, welches jeweils unsubstituiert oder einfach oder mehrfach unabhängig voneinander gleich oder verschieden substituiert sein kann durch Cyano, Oxo, Nitro, C₁-C₄-Alkoxycarbonyl, C₁-C₄-Alkylcarbonyl, Di-(C₁-C₄-Alkyl)aminocarbonyl oder durch einen gesättigten oder teilweise ungesättigten drei- bis siebengliedrigen Ring, gegebenenfalls enthaltend bis zu zwei Heteroatome ausgewählt aus Stickstoff, Sauerstoff oder Schwefel mit der Maßgabe dass zwei Sauerstoffatome nicht benachbart sein dürfen, wobei der Ring selbst gegebenenfalls einfach oder mehrfach unabhängig voneinander gleich oder verschieden durch Fluor, C₁-C₆-Alkyl oder C₁-C₆-Halogenalkyl substituiert sein kann,
oder
- R⁴: steht für jeweils geradkettiges oder verzweigtes C₁-C₆-Halogenalkyl oder C₂-C₆-Halogenalkenyl mit jeweils 1 bis 11 gleichen oder verschiedenen Halogenatomen, jeweils geradkettiges oder verzweigtes C₁-C₆-Alkylamino-C₁-C₆-alkyl, Di-(C₁-C₆-alkyl)amino-C₁-C₆-alkyl, C₁-C₆-Alkoxy-C₁-C₆-alkyl oder C₁-C₆-Halogenalkoxy-C₁-C₆-alkyl, geradkettiges oder verzweigtes Phenyl-(C₁-C₄)alkyl, welches im Phenylteil unsubstituiert oder einfach bis dreifach unabhängig voneinander gleich oder verschieden substituiert sein kann mit Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl oder Trifluormethoxy, oder für einen fünf- bis siebengliedrigen gesättigten oder teilweise ungesättigten substituierten oder unsubstituierten Ring, gegebenenfalls enthaltend bis zu drei Heteroatome ausgewählt aus Stickstoff, Sauerstoff oder Schwefel, mit der Maßgabe dass zwei Sauerstoffatome nicht benachbart sein dürfen.
- R⁵: steht besonders bevorzugt für Wasserstoff, NR⁶R⁷, jeweils geradkettiges oder verzweigtes C₁-C₆-Alkyl, C₂-C₆-Alkenyl, C₃-C₇-Cycloalkyl, C₁-C₆-Alkoxy; jeweils geradkettiges oder verzweigtes C₁-C₆-Halogenalkyl oder C₂-C₆-Halogenalkoxy mit jeweils 1 bis 11 gleichen oder verschiedenen Halogenatomen, oder Phenoxy welches einfach bis dreifach unabhängig voneinander durch folgende gleiche oder verschiedenene Reste substituiert sein kann:
Fluor, Chlor, Brom, Iod, Cyano, Hydroxy, Mercapto, Amino, Methyl, Ethyl, n-Propyl, Isopropyl, n-Butyl, Isobutyl, sek-Butyl, tert-Butyl, Cyclopropyl, 1-Chlorcyclopropyl, Vinyl, Ethinyl, Methoxy, Ethoxy, Isopropoxy, Methylthio, Ethylthio, Trifluormethylthio, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2,2-Trifluorethyl, Trifluormethoxy, Trifluormethylthio, 2-Methoxyethoxy, Acetyl, Propionyl, Methoxycarbonyl, N-Methylamino, N,N-Dimethylamino, Aminocarbonyl, Methylaminocarbonyl, Dimethylaminocarbonyl, Dimethylcarbamoylamino, Methoxycarbonylamino, Methoxycarbonyloxy, Methylsulfamoyl, Dimethylsulfamoyl, Phenyl oder Phenoxy,
- oder: zwei benachbarte Reste am Phenoxyrest bilden besonders bevorzugt zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl;
oder
- R⁴ und R⁵: bilden besonders bevorzugt zusammen einen drei bis acht Ringatome enthaltenden gesättigten oder teilweise ungesättigten substituierten oder unsubstituierten monocyclischen oder bicyclischen Ring ggf. enthaltend bis zu zwei zusätzliche Heteroatome ausgewählt aus Stickstoff, Sauerstoff, Schwefel oder Silizium, mit der Maßgabe dass zwei Sauerstoffatome nicht benachbart sein dürfen.
- R⁴: ist ganz besonders bevorzugt Wasserstoff oder ein unsubstituierter oder einfach bis dreifach substituierter Phenylring, dessen Substituenten unabhängig voneinander gleichartig oder verschieden sein können ausgewählt aus Chlor, Brom, Iod, Fluor, Fluormethyl, Difluormethyl, Trifluormethyl, Trifluormethoxy, Trifluormethylthio, Hydroxy, Methoxy, Methoxyethoxy, Cyano, Methyl, Ethyl, Amino, Methylamino, Dimethylamino, Acetyl, Methoxycarbonyl, N,N-Dimethylcarbamoyl,
oder
- R⁴: ist ganz besonders bevorzugt ein heteroaromatischer Rest ausgewählt aus 2-Furyl, 3-Furyl, 2-Thienyl, 3-Thienyl, 2-Oxazolyl, 4-Oxazolyl, 5-Oxazolyl, 2-Thiazolyl, 4-Thiazolyl, 5-Thiazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, Benzofur-2-yl, Benzothiophen-2-yl, Benzofur-3-yl, Benzothiophen-3-yl, 2-Pyridinyl, 3-Pyridinyl, 4-Pyridinyl, 2-Pyrimidinyl, 4-Pyrimidinyl oder 5-Pyrimidinyl, welcher jeweils unsubstituiert oder einfach oder mehrfach substituiert ist unabhängig voneinander durch gleiche oder verschiedenene Reste ausgewählt aus Fluor, Chlor, Brom, Iod, Cyano, Hydroxy, Mercapto, Methyl, Ethyl, tert-Butyl, Cyclopropyl, Vinyl, Ethinyl, Methoxy, Ethoxy, Trifluormethyl, Trifluormethoxy, 2-Methoxyethoxy, Acetyl, N-Methylamino, N,N-Dimethylamino, N-Methylcarbamoyl oder N,N-Dimethylcarbamoyl ,
oder
- R⁴: ist ganz besonders bevorzugt eine Gruppe ausgewählt aus 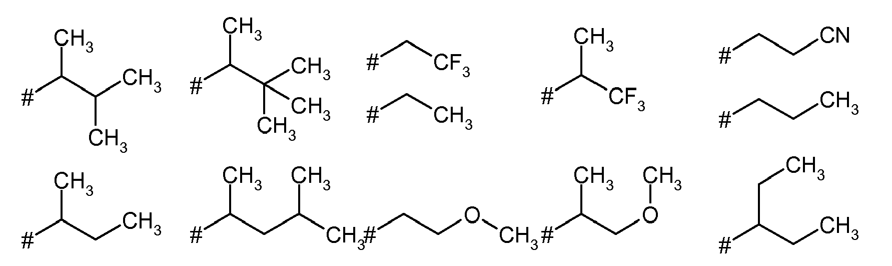 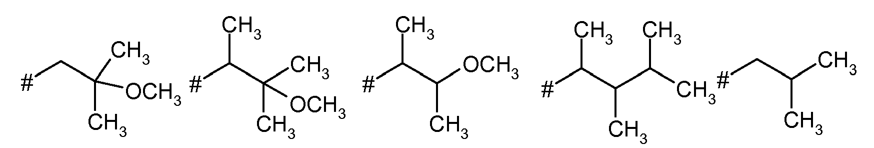 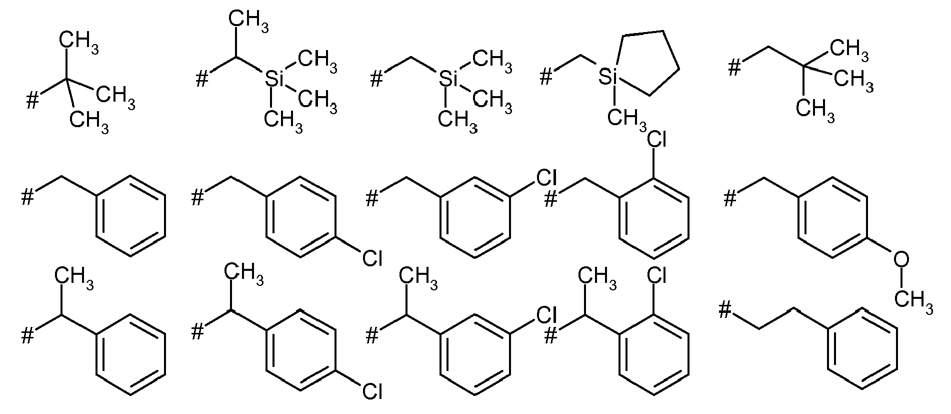 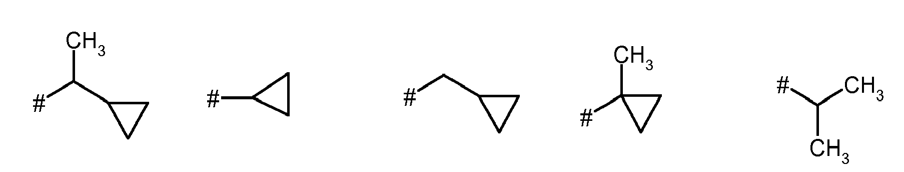 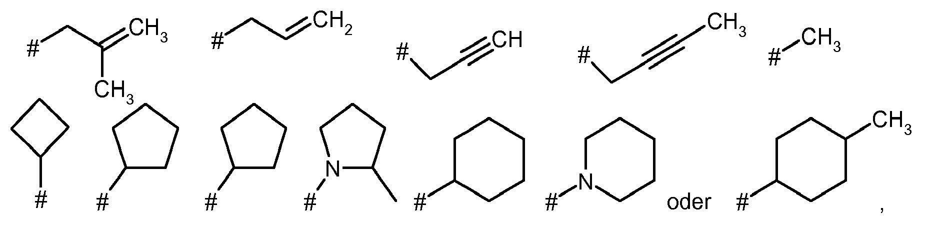
wobei "#" die Anknüpfungsstelle markiert.
- R⁴: steht insbesondere bevorzugt für Cyclopropyl, 2-Methoxy-1-methylethyl, Isopropyl, Cyclopropylmethyl, 2-Butyl, 2-Methylpropyl, Benzyl, rac-(1,2-Dimethylpropyl), R-(1,2-Dimethylpropyl, 2,2-Dimethylpropyl, 2-Methylpropyl, 2-Fluorphenyl, 3-Methoxyphenyl, 4-Fluorphenyl, 3-Fluorphenyl, 3-Methylphenyl, 3-(Trifluormethyl)phenyl, 4-Chlorphenyl, 3-(Trifluormethoxy)phenyl oder 2,4-Dichlorphenyl.

- R⁵: steht ganz besonders bevorzugt für Wasserstoff, Methyl, Ethyl, n-Propyl, Isopropyl, n-Butyl, Isobutyl, Pentyl, 2-Methylbutyl, 3-Methylbutyl, 2,2-Di-methylpropyl, Cyclopropyl, Cyclobutyl, 2,2,2-Trifluorethyl, 1-(Trifluormethyl-)propyl, 2-Cyanoethyl, 2-Methoxyethyl, 2-Methoxy-2-methylpropyl, Cyclopropylmethyl, 2-Methylprop-2-enyl, Allyl, Propargyl, 3-Methylpropargyl, Amino, Methylamino, Dimethylamino, Ethylamino, Diethylamino, Methylethylamino, Cyclopropylamino, Isopropylamino, N-Methyl-N-isopropylamino, Phenylamino, 4-Chlorphenylamino, 3-Chlorphenylamino, 2-Chlorphenylamino, N-Methyl-N-phenylamino, N-Methyl-N-(4-chlorphenyl)amino, N-Methyl-N-(3-chlorphenyl)amino, N-Methyl-N-(2-chlorphenyl)amino, Methoxy, Ethoxy, Isopropoxy, 1,1,1-Trifluorisopropoxy, Phenoxy, 2-Chlorphenoxy, 3-Chlorphenoxy oder 4-Chlorphenoxy,
oder
- R⁴ und R⁵: bilden ganz besonders bevorzugt zusammen mit dem Stickstoffatom, an das sie gebunden sind, einen Ring ausgewählt aus 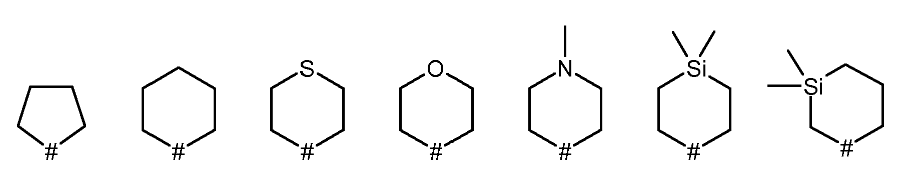 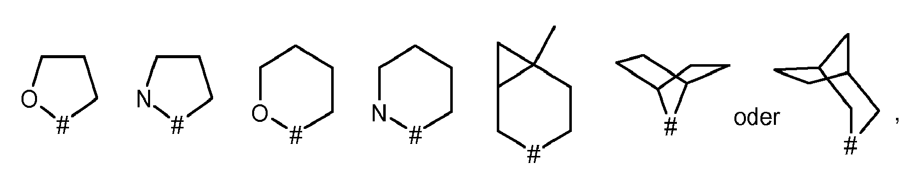
wobei der Ring unsubstituiert oder einfach bis dreifach unabhängig voneinander gleichartig oder verschieden durch Methyl, Fluor oder Trifluormethyl substituiert sein kann und "#" das Stickstoffatom symbolisiert über welches der Ring gebunden ist.
- R⁵: steht insbesondere bevorzugt für Wasserstoff oder Methyl,
oder
- R⁴ und R⁵: stehen zusammen mit dem Stickstoff an den sie gebunden sind insbesondere bevorzugt für Morpholin-4-yl, Pyrrolidin-1-yl, 2-Methylpyrrolidin-1-yl, 4-Methylpiperidin-1-yl, 4,4-Dimethyl-1,4-azasilinan-1-yl oder 6-Methyl-3-azabicyclo[4.1.0]hept-3-yl.
- R⁶ und R⁷: stehen bevorzugt und unabhängig voneinander gleichartig oder verschieden für R³, C₁-C₄-Alkyl, C₃-C₇-Cycloalkyl, C₁-C₄-Alkoxy, C₁-C₄-Alkylcarbonyl, C₁-C₄-Alkoxycarbonyl, C₁-C₄-Alkylaminocarbonyl oder Di-(C₁-C₄-Alkyl)-aminocarbonyl, geradkettiges oder
verzweigtes Phenyl-(C₁-C₄)alkyl, welches im Phenylteil unsubstituiert oder einfach oder mehrfach unabhängig voneinander gleich oder verschieden mit Fluor, Chlor, Brom, Cyano, Nitro, Methyl, 1,1-Dimethylethyl, Methoxy, Methylamino, Dimethylamino, Fluormethyl, Difluormethyl, Trifluormethyl oder Trifluormethoxy substituiert sein kann,
oder
- R⁶ und: R⁷ bilden bevorzugt zusammen einen drei bis sechs Ringatome enthaltenden gesättigten oder teilweise ungesättigten substituierten oder unsubstituierten monocyclischen Ring gegebenenfalls enthaltend bis zu zwei zusätzliche Heteroatome ausgewählt aus Stickstoff, Sauerstoff, Schwefel oder Silizium, mit der Maßgabe dass zwei Sauerstoffatome nicht benachbart sein dürfen.
- R⁶ und R⁷: stehen besonders bevorzugt und unabhängig voneinander gleichartig oder verschieden für Phenyl, 2-Furyl, 3-Furyl, 2-Thienyl, 3-Thienyl, 1-Pyrrolyl, 2-Pyrrolyl, 3-Pyrrolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4-Triazol-1-yl, 1,2,4-Triazol-3-yl, 1,2,4-Triazol-4-yl, 1,2,4-Triazol-5-yl, Indol-1-yl, Indol-2-yl, Indol-3-yl, Isoindol-1-yl, Isoindol-2-yl, Benzofur-2-yl, Benzothiophen-2-yl, Benzofur-3-yl, Benzothiophen-3-yl, Benzoxazol-2-yl, Benzothiazol-2-yl, Benzimidazol-2-yl, Indazol-1-yl, Indazol-2-yl, Indazol-3-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl oder 1,2,4-Triazin-6-yl,
welches jeweils einfach oder mehrfach unabhängig voneinander durch gleiche oder verschiedenene Reste substituiert sein kann ausgewählt aus Wasserstoff, Fluor, Chlor, Brom, Iod, Cyano, Hydroxy, Mercapto, Amino, Methyl, Ethyl, n-Propyl, Isopropyl, n-Butyl, Isobutyl, sek-Butyl, tert-Butyl, Cyclopropyl, Vinyl, Ethinyl, Methoxy, Ethoxy, Isopropoxy, Methylthio, Ethylthio, Trifluormethylthio, Difluormethyl, Trifluormethyl, 2,2,2-Trifluorethyl, Trifluormethoxy, Trifluormethylthio, 2,2,2-Trifluorethoxy, 2-Methoxyethoxy, Acetyl, Propionyl, Methoxycarbonyl, Ethoxycarbonyl, N-Methylamino, N,N-Dimethylamino, N-Ethylamino, N,N-Diethylamino, Aminocarbonyl, Methylaminocarbonyl, Dimethylaminocarbonyl, Dimethylcarbamoylamino, Methoxycarbonylamino, Methoxycarbonyloxy, Ethoxycarbonylamino, Ethoxycarbonyloxy, Methylsulfamoyl, Dimethylsulfamoyl, Phenyl oder Phenoxy,
- oder: zwei benachbarte Reste am Phenylring oder am Heterocyclus bilden besonders bevorzugt zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl,
- oder: zwei Reste am Heterocyclus bilden besonders bevorzugt mit dem Kohlenstoffatom, an das sie gebundenen sind, eine Carbonyl- oder Thiocarbonylgruppe,
oder
- R⁶ und R⁷: stehen unabhängig voneinander gleichartig oder verschieden für C₁-C₆-Alkyl, C₃-C₇-Cycloalkyl, C₁-C₆-Alkoxy, C₁-C₆-Alkylcarbonyl, C₁-C₆-Alkoxycarbonyl, C₁-C₆-Alkylaminocarbonyl oder Di-(C₁-C₆-Alkyl)-aminocarbonyl, geradkettiges oder verzweigtes Phenyl-(C₁-C₆)alkyl, welches im Phenylteil unsubstituiert oder einfach oder mehrfach unabhängig voneinander gleich oder verschieden substituiert sein kann mit Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy;
oder
- R⁶ und R⁷: bilden besonders bevorzugt zusammen einen drei bis acht Ringatome enthaltenden gesättigten oder teilweise ungesättigten substituierten oder unsubstituierten monocyclischen Ring gegebenenfalls enthaltend bis zu ein zusätzliches Heteroatom ausgewählt aus Stickstoff, Sauerstoff oder Schwefel, mit der Maßgabe dass zwei Sauerstoffatome nicht benachbart sein dürfen.
- R⁸: steht bevorzugt für R³, NR⁶R⁷,jeweils geradkettiges oder verzweigtes C₁-C₆-Alkyl, C₂-C₆-Alkenyl, C₂-C₆-Alkinyl, C₃-C₇-Cycloalkyl oder C₃-C₇-Cycloalkenyl, wobei R⁸ für den Fall, dass es ungleich R³ und NR⁶R⁷ ist, jeweils unsubstituiert oder einfach oder mehrfach unabhängig voneinander gleich oder verschieden substituiert sein kann durch Cyano, Oxo, Nitro, C₁-C₄-Alkoxycarbonyl, C₁-C₄-Alkylcarbonyl, Di-(C₁-C₄-alkyl)aminocarbonyl oder durch einen gesättigten oder teilweise ungesättigten drei- bis siebengliedrigen Ring, gegebenenfalls enthaltend bis zu drei Heteroatome ausgewählt aus Stickstoff, Sauerstoff oder Schwefel mit der Maßgabe dass zwei Sauerstoffatome nicht benachbart sein dürfen, wobei der Ring selbst gegebenenfalls einfach oder mehrfach unabhängig voneinander gleich oder verschieden durch Fluor, C₁-C₆-Alkyl oder C₁-C₆-Halogenalkyl substituiert sein kann,
oder
- R⁸: steht für jeweils geradkettiges oder verzweigtes C₁-C₆-Halogenalkyl oder C₂-C₆-Halogenalkenyl mit jeweils 1 bis 11 gleichen oder verschiedenen Halogenatomen, jeweils geradkettiges oder verzweigtes C₁-C₆Alkylamino-C₁-C₆-alkyl, Di-(C₁-C₆-alkyl)amino-C₁-C₆-alkyl, C₁-C₆-Alkoxy-C₁-C₆-alkyl oder C₁-C₆-Halogenalkoxy-C₁-C₆-alkyl, geradkettiges oder verzweigtes Phenyl-(C₁-C₆)alkyl, welches im Phenylteil unsubstituiert oder einfach oder mehrfach unabhängig voneinander gleich oder verschieden mit Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy substituiert sein kann, oder für einen fünf- bis siebengliedrigen gesättigten oder teilweise ungesättigten substituierten oder unsubstituierten Ring, gegebenenfalls enthaltend bis zu drei Heteroatome ausgewählt aus Stickstoff, Sauerstoff oder Schwefel, mit der Maßgabe dass zwei Sauerstoffatome nicht benachbart sein dürfen.
- R⁸: steht besonders bevorzugt für einen einfach bis dreifach substituierten Phenylring, dessen Substituenten unabhängig voneinander gleichartig oder verschieden sein können ausgewählt aus Chlor, Brom, Iod, Fluor, Fluormethyl, Difluormethyl, Trifluormethyl, Trifluormethoxy, Trifluormethylthio, Hydroxy, Methoxy, Methoxyethoxy, Nitro, Cyano, Methyl, Ethyl, Amino, Methylamino, Dimethylamino, Acetyl, Methoxycarbonyl, N,N-Dimethylcarbamoyl, Phenyl oder Phenoxy,
- oder: zwei benachbarte Substituenten des Phenylrings bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach, gleichartig oder verschieden substituiert sein können durch Fluor, Chlor, Methyl, Ethyl, n-Propyl, Isopropyl und/oder Trifluormethyl,
oder
- R⁸: steht besonders bevorzugt für 2-Furyl, 3-Furyl, 2-Thienyl, 3-Thienyl, 1-Pyrrolyl, 2-Pyrrolyl, 3-Pyrrolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4-Triazol-1-yl, 1,2,4-Triazol-3-yl, 1,2,4-Triazol-4-yl, 1,2,4-Triazol-5-yl, Indol-1-yl, Indol-2-yl, Indol-3-yl, Isoindol-1-yl, Isoindol-2-yl, Benzofur-2-yl, Benzothiophen-2-yl, Benzofur-3-yl, Benzothiophen-3-yl, Benzoxazol-2-yl, Benzothiazol-2-yl, Benzimidazol-2-yl, Indazol-1-yl, Indazol-2-yl, Indazol-3-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl oder 1,2,4-Triazin-6-yl,
welches jeweils einfach oder mehrfach unabhängig voneinander durch gleiche oder verschiedenene Reste substituiert sein kann ausgewählt aus Wasserstoff, Fluor, Chlor, Brom, Iod, Cyano, Hydroxy, Mercapto, Amino, Methyl, Ethyl, n-Propyl, Isopropyl, n-Butyl, Isobutyl, sek-Butyl, tert-Butyl, Cyclopropyl, Vinyl, Ethinyl, Methoxy, Ethoxy, Isopropoxy, Methylthio, Ethylthio, Trifluormethylthio, Difluormethyl, Trifluormethyl, 2,2,2-Trifluorethyl, Trifluormethoxy, Trifluormethylthio, 2,2,2-Trifluorethoxy, 2-Methoxyethoxy, Acetyl, Propionyl, Methoxycarbonyl, Ethoxycarbonyl, N-Methylamino, N,N-Dimethylamino, N-Ethylamino, N,N-Diethylamino, Aminocarbonyl, Methylaminocarbonyl, Dimethylaminocarbonyl, Dimethylcarbamoylamino, Methoxycarbonylamino, Methoxycarbonyloxy, Ethoxycarbonylamino, Ethoxycarbonyloxy, Methylsulfamoyl, Dimethylsulfamoyl, Phenyl oder Phenoxy,
- oder: zwei benachbarte Reste am Heterocyclus bilden besonders bevorzugt zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl,
- oder: zwei Reste am Heterocyclus bilden besonders bevorzugt mit dem Kohlenstoffatom, an das sie gebundenen sind, eine Carbonyl- oder Thiocarbonylgruppe;
oder
- R⁸: steht besonders bevorzugt für NR⁶R⁷, jeweils geradkettiges oder verzweigtes C₁-C₆-Alkyl, C₂-C₆-Alkenyl, C₂-C₆-Alkinyl, C₃-C₇-Cycloalkyl oder C₃-C₇-Cycloalkenyl, wobei R⁸ für den Fall, dass es ungleich NR⁶R⁷ ist, jeweils unsubstituiert oder einfach oder mehrfach unabhängig voneinander gleich oder verschieden substituiert sein kann durch Cyano, Oxo, Nitro, C₁-C₄-Alkoxycarbonyl, C₁-C₄-Alkylcarbonyl, Di-(C₁-C₄-alkyl)aminocarbonyl oder durch einen gesättigten oder teilweise ungesättigten drei- bis siebengliedrigen Ring, gegebenenfalls enthaltend bis zu drei Heteroatome ausgewählt aus Stickstoff, Sauerstoff oder Schwefel mit der Maßgabe dass zwei Sauerstoffatome nicht benachbart sein dürfen, wobei der Ring selbst gegebenenfalls einfach oder mehrfach unabhängig voneinander gleich oder verschieden durch Fluor, C₁-C₆-Alkyl oder C₁-C₆-Halogenalkyl substituiert sein kann,
oder
- R⁸: steht besonders bevorzugt für jeweils geradkettiges oder verzweigtes C₁-C₆-Halogenalkyl oder C₂-C₆-Halogenalkenyl mit jeweils 1 bis 11 gleichen oder verschiedenen Halogenatomen, jeweils geradkettiges oder verzweigtes C₁-C₄-Alkylamino-C₁-C₄-alkyl, Di-(C₁-C₄-alkyl)amino-C₁-C₄-alkyl, C₁-C₄-Alkoxy-C₁-C₄-alkyl oder C₁-C₄-Halogenalkoxy-C₁-C₄-alkyl, geradkettiges oder verzweigtes Phenyl-(C₁-C₄)alkyl, welches im Phenylteil unsubstituiert oder einfach bis dreifach unabhängig voneinander gleich oder verschieden mit Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy substituiert sein kann oder für einen fünf- bis siebengliedrigen gesättigten oder teilweise ungesättigten substituierten oder unsubstituierten Ring, gegebenenfalls enthaltend bis zu drei Heteroatome ausgewählt aus Stickstoff, Sauerstoff oder Schwefel, mit der Maßgabe dass zwei Sauerstoffatome nicht benachbart sein dürfen.
- R⁸: ist ganz besonders bevorzugt ein einfach bis dreifach substituierter Phenylring, dessen Substituenten unabhängig voneinander gleichartig oder verschieden sein können ausgewählt aus Chlor, Brom, Iod, Fluor, Fluormethyl, Difluormethyl, Trifluormethyl, Trifluormethoxy, Trifluormethylthio, Hydroxy, Methoxy, Methoxyethoxy, Nitro, Cyano, Methyl, Ethyl, Amino, Methylamino, Dimethylamino, Acetyl, Methoxycarbonyl, N,N-Dimethylcarbamoyl, Phenyl oder Phenoxy;
oder
- R⁸: ist ganz besonders bevorzugt ein heteroaromatischer Rest ausgewählt aus 2-Furyl, 3-Furyl, 2-Thienyl, 3-Thienyl, 2-Oxazolyl, 4-Oxazolyl, 5-Oxazolyl, 2-Thiazolyl, 4-Thiazolyl, 5-Thiazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, Benzofur-2-yl, Benzothiophen-2-yl, Benzofur-3-yl, Benzothiophen-3-yl, 2-Pyridinyl, 3-Pyridinyl, 4-Pyridinyl, 2-Pyrimidinyl, 4-Pyrimidinyl oder 5-Pyrimidinyl, welcher jeweils unsubstituiert oder einfach oder mehrfach durch gleiche oder verschiedenene Reste ausgewählt aus Fluor, Chlor, Brom, Iod, Cyano, Hydroxy, Mercapto, Methyl, Ethyl, tert-Butyl, Cyclopropyl, Vinyl, Ethinyl, Methoxy, Ethoxy, Trifluormethyl, Trifluormethoxy, 2-Methoxyethoxy, Acetyl, N-Methylamino, N,N-Dimethylamino, N-Methylcabamoyl oder N,N-Dimethylcarbamoyl substituiert sein kann;
oder
- R⁸: ist jeweils unsubstituiertes oder im Phenylteil einfach bis dreifach substituiertes Benzyl, 1-Phenylethyl oder 2-Phenylethyl, wobei die Substituenten im Phenylteil unabhängig voneinander gleichartig oder verschieden sein können ausgewählt aus Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy;
oder
- R⁸: ist ganz besonders bevorzugt gleich Benzyl, 2-Chlorbenzyl, 3-Chlorbenzyl, 4-Chlorbenzyl, 4-Methoxybenzyl, 3,4-Dimethoxybenzyl, 1-(Phenyl)ethyl, 1-(2-Chlorphenyl)ethyl, 1-(3-Chlorphenyl)ethyl, 1-(4-Chlorphenyl)ethyl, Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; Cyclopropyl, 1-Methoxycyclopropyl, 1-Chlorcyclopropyl, 1-Methylcyclopropyl, Cyclobutyl, Cyclopentyl, Cyclohexyl, 4-Methylcyclohexyl, 2,2,2-Trifluorethyl, 1-(Trifluormethyl-)propyl, 2-Cyanoethyl, 1,3-Dimethylbutyl, 2-Methoxyethyl, 1-Methyl-2-methoxypropyl, 2-Methoxy-2-methylpropyl, 2-Methoxy-1,2-dimethylpropyl, 2-Methoxy-1-methylpropyl, 1-(Cyclopropyl)ethyl, Cyclopropylmethyl, 2-Methylprop-2-enyl, Allyl, Propargyl, 3-Methylpropargyl, Methylamino, Dimethylamino, Ethylamino, Diethylamino, Methylethylamino, Cyclopropylamino, 1-Piperidinyl, 1-Pyrrolidinyl, Phenylamino, 4-Chlorphenylamino, N-Phenyl-N-methylamino, 1,1-Dimethylethylamino, Cyclohexylamino oder Isopropylamino.
- R⁸: steht insbesondere bevorzugt für Propargyl, Cyclopropylmethyl, 4-Methoxybenzyl, (1-Methylcyclopropyl)methyl, Isobutyl, Cyclohexyl, 4-Chlorphenyl, 2-(2-Methoxyethoxy)ethyl, 2-Methoxyethyl, 4-Oxacyclohexyl, 3-Cyanophenyl, 4-Cyanophenyl, 2-Fluorphenyl, 4-Fluorphenyl, 3-Methoxyphenyl, 3-Fluorphenyl, 3-(N,N-Dimethylamino)phenyl, 3-Methylphenyl, 3-Chlorphenyl, 3-(Trifluormethyl)phenyl, 3-Chlor-5-methoxyphenyl, 2,5-Dichlorphenyl, 3-(Trifluormethoxy)phenyl, 3,4-Dichlorphenyl, 2,4-Dichlorphenyl, 3,5-Dichlorphenyl, 3-(tert-Butyl-)phenyl oder 4-(4-Chlorphenoxy)phenyl.
- R⁹: steht bevorzugt für Formyl, geradkettiges oder verzweigtes Tri-(C₁-C₆-alkyl)silyl, geradkettiges oder verzweigtes C₁-C₆-Alkylcarbonyl, C₃-C₇-Cycloalkylcarbonyl, C₁-C₆-Alkoxycarbonyl, C₁-C₆-Alkylaminocarbonyl, Di-(C₁-C₆-Alkyl)aminocarbonyl, C₁-C₆-Alkylaminothiocarbonyl, Di-(C₁-C₆-alkyl)aminothiocarbonyl, C₁-C₆-Alkylsulfinyl oder C₁-C₆-Alkylsulfonyl, wobei R⁹ für den Fall, dass es ungleich Formyl ist, jeweils unsubstituiert
oder einfach oder mehrfach unabhängig voneinander gleich oder verschieden substituiert sein kann durch Fluor, Chlor, Cyano, C₁-C₄-Alkoxy oder C₁-C₄-Alkyl,
oder
- R⁹: steht für jeweils geradkettiges oder verzweigtes C₁-C₆-Halogenalkylcarbonyl, C₁-C₆-Halogenalkylsulfinyl oder C₁-C₆-Halogenalkylsulfonyl mit jeweils 1 bis 13 gleichen oder verschiedenen Halogenatomen, jeweils unsubstituiertes oder einfach bis dreifach substituiertes Phenylcarbonyl, Phenylacetyl, Phenoxycarbonyl, Phenylaminocarbonyl, Phenylaminothiocarbonyl, Phenylsulfinyl, Phenylsulfonyl, 2-Furylcarbonyl, 3-Furylcarbonyl, 2-Thienylcarbonyl, 3-Thienylcarbonyl, 2-Pyrrolylcarbonyl, 3-Pyrrolylcarbonyl, 3-Pyrazolylcarbonyl, 4-Pyrazolylcarbonyl, 5-Pyrazolylcarbonyl, 4-Oxazolylcarbonyl, 5-Oxazolylcarbonyl, 4-Thiazolylcarbonyl, 5-Thiazolylcarbonyl, 2-Imidazolylcarbonyl, 4-Imidazolylcarbonyl, 5-Imidazolylcarbonyl, 1,2,4-Oxadiazol-3-ylcarbonyl, 1,2,4-Oxadiazol-5-ylcarbonyl, 1,2,4-Thiadiazol-3-ylcarbonyl, 1,2,4-Thiadiazol-5-ylcarbonyl, 1,3,4-Oxadiazol-2-ylcarbonyl, 1,3,4-Thiadiazol-2-ylcarbonyl, 1,2,4-Triazol-3-ylcarbonyl, 1,2,4-Triazol-5-ylcarbonyl, 1,2,3-Triazol-4-ylcarbonyl, Indol-2-ylcarbonyl, Indol-3-ylcarbonyl, Isoindol-1-ylcarbonyl, Benzofur-2-ylcarbonyl, Benzothiophen-2-ylcarbonyl, Benzofur-3-ylcarbonyl, Benzothiophen-3-ylcarbonyl, Benzoxazol-2-ylcarbonyl, Benzothiazol-2-ylcarbonyl, Benzimidazol-2-ylcarbonyl, 2-Pyridinylcarbonyl, 3-Pyridinylcarbonyl, 4-Pyridinylcarbonyl, 3-Pyridazinylcarbonyl, 4-Pyridazinylcarbonyl, 2-Pyrimidinylcarbonyl, 4-Pyrimidinylcarbonyl, 5-Pyrimidinylcarbonyl oder 2-Pyrazinylcarbonyl, wobei die Substituenten im Phenylteil oder Heteroarylteil unabhängig voneinander gleichartig oder verschieden sein können ausgewählt aus Fluor, Chlor, Brom, Iod, Cyano, Methyl, 1,1-Dimethylethyl, Methoxy, Fluormethyl, Difluormethyl, Trifluormethyl oder Trifluormethoxy.
- R⁹: steht besonders bevorzugt für Formyl, geradkettiges oder verzweigtes Tri-(C₁-C₄-alkyl)silyl, jeweils geradkettiges oder verzweigtes C₁-C₆-Alkylcarbonyl, C₃-C₇-Cycloalkylcarbonyl, C₁-C₆-Alkoxycarbonyl, C₁-C₆-Alkylsulfonyl, C₁-C₆-Alkylaminocarbonyl oder Di-(C₁-C₆-alkyl)aminocarbonyl, welches jeweils unsubstituiert oder einfach oder mehrfach unabhängig voneinander substituiert sein kann durch Fluor, Chlor, Cyano, C₁-C₄-Alkoxy oder C₁-C₄-Alkyl,
oder
- R⁹: steht für jeweils geradkettiges oder verzweigtes C₁-C₄-Halogenalkylcarbonyl oder C₁-C₄-Halogenalkylsulfonyl mit jeweils 1 bis 9 gleichen oder verschiedenen Halogenatomen, jeweils unsubstituiertes oder einfach bis dreifach substituiertes Phenylcarbonyl, Phenylacetyl, Phenylaminocarbonyl, Phenylsulfonyl, 2-Furylcarbonyl, 3-Furylcarbonyl, 2-Thienylcarbonyl, 3-Thienylcarbonyl, 3-Pyrazolylcarbonyl, 4-Pyrazolylcarbonyl, 5-Pyrazolylcarbonyl, 4-Thiazolylcarbonyl, 5-Thiazolylcarbonyl, 1,2,4-Oxadiazol-3-ylcarbonyl, 1,2,4-Oxadiazol-5-ylcarbonyl, 1,2,4-Thiadiazol-3-ylcarbonyl, 1,2,4-Thiadiazol-5-ylcarbonyl, 1,3,4-Oxadiazol-2-ylcarbonyl, 1,3,4-Thiadiazol-2-ylcarbonyl, 2-Pyridinylcarbonyl, 3-Pyridinylcarbonyl, 4-Pyridinylcarbonyl, 2-Pyrimidinylcarbonyl, 4-Pyrimidinylcarbonyl, 5-Pyrimidinylcarbonyl oder 2-Pyrazinylcarbonyl, wobei die Substituenten im Phenylteil oder Heteroarylteil unabhängig voneinander gleichartig oder verschieden sein können ausgewählt aus Fluor, Chlor, Brom, Cyano, Methyl, 1,1-Dimethylethyl, Methoxy, Trifluormethyl oder Trifluormethoxy.
- R⁹: steht ganz besonders bevorzugt für Formyl, Acetyl, 2-Chloracetyl, 2-Methoxyacetyl, n-Propionyl, i-Propionyl, 2-Methoxypropionyl, Pivaloyl, Phenylacetyl, 4-Chlorphenylacetyl, Methoxycarbonyl, Ethoxycarbonyl, n-Propoxycarbonyl, i-Propoxycarbonyl, tert.-Butoxycarbonyl, N,N-Dimethylcarbamoyl, Methylsulfonyl, Ethylsulfonyl, Benzoyl, 2-Chlorbenzoyl, 3-Chlorbenzoyl, 4-Chlorbenzoyl, Phenylcarbamoyl, 2-Chlorphenylcarbamoyl, 3-Chlorphenylcarbamoyl, 4-Chlorphenylcarbamoyl, 2-Methoxyphenylcarbamoyl, 3-Methoxyphenylcarbamoyl, 4-Methoxyphenylcarbamoyl, Phenylthiocarbamoyl, 2-Methoxyphenylthiocarbamoyl, 3-Methoxyphenylthiocarbamoyl, 4-Methoxyphenylthiocarbamoyl, Phenylsulfonyl, 4-Methylphenylsulfonyl, Trimethylsilyl, Triethylsilyl, tert-Butyldimethylsilyl oder Triisopropylsilyl.
- R¹⁰: steht bevorzugt für jeweils geradkettiges oder verzweigtes C₁-C₆-Alkylcarbonyl, C₃-C₇-Cycloalkylcarbonyl, C₁-C₆-Alkoxycarbonyl, C₁-C₆-Alkylaminocarbonyl oder Di-(C₁-C₆-alkyl)aminocarbonyl, welches jeweils unsubstituiert oder einfach oder mehrfach unabhängig voneinander substituiert sein kann durch Fluor, Chlor, Cyano, C₁-C₄-Alkoxy oder C₁-C₄-Alkyl,
oder
- R¹⁰: steht für geradkettiges oder verzweigtes Tri-(C₁-C₆-alkyl)silyl, jeweils unsubstituiertes oder einfach bis dreifach substituiertes Phenylcarbonyl, Phenylacetyl, Phenylacetyl, Phenylaminocarbonyl, Phenylaminothiocarbonyl, 2-Furylcarbonyl, 3-Furylcarbonyl, 2-Thienylcarbonyl, 3-Thienylcarbonyl, 2-Pyrrolylcarbonyl, 3-Pyrrolylcarbonyl, 3-Pyrazolylcarbonyl, 4-Pyrazolylcarbonyl, 5-Pyrazolylcarbonyl, 4-Oxazolylcarbonyl, 5-Oxazolylcarbonyl, 4-Thiazolylcarbonyl, 5-Thiazolylcarbonyl, 2-Imidazolylcarbonyl, 4-Imidazolylcarbonyl, 5-Imidazolylcarbonyl, 1,2,4-Oxadiazol-3-ylcarbonyl, 1,2,4-Oxadiazol-5-ylcarbonyl, 1,2,4-Thiadiazol-3-ylcarbonyl, 1,2,4-Thiadiazol-5-ylcarbonyl, 1,3,4-Oxadiazol-2-ylcarbonyl, 1,3,4-Thiadiazol-2-ylcarbonyl, 1,2,4-Triazol-3-ylcarbonyl, 1,2,4- Triazol-5-ylcarbonyl, 1,2,3-Triazol-4-ylcarbonyl, Indol-2-ylcarbonyl, Indol-3-ylcarbonyl, Isoindol-1-ylcarbonyl, Benzofur-2-ylcarbonyl, Benzothiophen-2-ylcarbonyl, Benzofur-3-ylcarbonyl, Benzothiophen-3-ylcarbonyl, Benzoxazol-2-ylcarbonyl, Benzothiazol-2-ylcarbonyl, Benzimidazol-2-ylcarbonyl, 2-Pyridinylcarbonyl, 3-Pyridinylcarbonyl, 4-Pyridinylcarbonyl, 3-Pyridazinylcarbonyl, 4-Pyridazinylcarbonyl, 2-Pyrimidinylcarbonyl, 4-Pyrimidinylcarbonyl, 5-Pyrimidinylcarbonyl oder 2-Pyrazinylcarbonyl, wobei die Substituenten im Phenylteil oder Heteroarylteil unabhängig voneinander gleichartig oder verschieden sein können ausgewählt aus Fluor, Chlor, Brom, Iod, Cyano, Methyl, 1,1-Dimethylethyl, Methoxy, Fluormethyl, Difluormethyl, Trifluormethyl oder Trifluormethoxy.
- R¹⁰: steht besonders bevorzugt für jeweils geradkettiges oder verzweigtes C₁-C₄-Alkylcarbonyl, C₃-C₇-Cycloalkylcarbonyl, C₁-C₄-Alkoxycarbonyl, C₁-C₄-Alkylaminocarbonyl oder Di-(C₁-C₄-alkyl)aminocarbonyl, welches jeweils unsubstituiert oder einfach oder mehrfach unabhängig voneinander substituiert sein kann durch Fluor, Cyano, C₁-C₄-Alkoxy oder C₁-C₄-Alkyl, geradkettiges oder verzweigtes Tri-(C₁-C₄-alkyl)silyl, jeweils unsubstituiertes oder einfach bis dreifach substituiertes Phenylcarbonyl, Phenylacetyl, Phenylaminocarbonyl, 2-Furylcarbonyl, 3-Furylcarbonyl, 2-Thienylcarbonyl, 3-Thienylcarbonyl, 3-Pyrazolylcarbonyl, 4-Pyrazolylcarbonyl, 5-Pyrazolylcarbonyl, 4-Thiazolylcarbonyl, 5-Thiazolylcarbonyl, 1,2,4-Oxadiazol-3-ylcarbonyl, 1,2,4-Oxadiazol-5-ylcarbonyl, 1,2,4-Thiadiazol-3-ylcarbonyl, 1,2,4-Thiadiazol-5-ylcarbonyl, 1,3,4-Oxadiazol-2-ylcarbonyl, 1,3,4-Thiadiazol-2-ylcarbonyl, 2-Pyridinylcarbonyl, 3-Pyridinylcarbonyl, 4-Pyridinylcarbonyl, 2-Pyrimidinylcarbonyl, 4-Pyrimidinylcarbonyl, 5-Pyrimidinylcarbonyl oder 2-Pyrazinylcarbonyl, wobei die Substituenten im Phenylteil oder Heteroarylteil unabhängig voneinander gleichartig oder verschieden sein können ausgewählt aus Fluor, Chlor, Brom, Cyano, Methyl, 1,1-Dimethylethyl, Methoxy, Trifluormethyl oder Trifluormethoxy.
- R¹⁰: steht ganz besonders bevorzugt für Formyl, Acetyl, 2-Chloracetyl, 2-Methoxyacetyl, n-Propionyl, i-Propionyl, Pivaloyl, Phenylacetyl, 2-Chlorphenylacetyl, 3-Chlorphenylacetyl, 4-Chlorphenylacetyl, Methoxycarbonyl, Ethoxycarbonyl, n-Propoxycarbonyl, i-Propoxycarbonyl, Cyclopropylcarbonyl, tert.-Butoxycarbonyl, N,N-Dimethylcarbamoyl, Phenylcarbamoyl, Trimethylsilyl, Triethylsilyl, tert-Butyldimethylsilyl, Triisopropylsilyl, Phenylcarbamoyl, 2-Chlorphenylcarbamoyl, 4-Chlorphenylcarbamoyl, 4-Chlorphenylcarbamoyl, 2-Methoxyphenylcarbamoyl, 4-Methoxyphenylcarbamoyl, 4-Methoxyphenylcarbamoyl, Phenylcarbonyl, 2-Chlorphenylcarbonyl, 4-Chlorphenylcarbonyl, 4-Chlorphenylcarbonyl, 2-Methoxyphenylcarbonyl, 4-
Methoxyphenylcarbonyl, 4-Methoxyphenylcarbonyl, 2-Cyanophenylcarbonyl, 4-Cyanophenylcarbonyl oder 4-Cyanophenylcarbonyl.

Bevorzugt sind dabei solche Verbindungen der Formel (I), in welchen alle Reste die oben genannten bevorzugten Bedeutungen haben.

Besonders bevorzugt sind Verbindungen der Formel (I), in welchen alle Reste die oben genannten besonders bevorzugten Bedeutungen haben.

Ganz besonders bevorzugt sind Verbindungen der Formel (I), in welchen alle Reste die oben genannten ganz besonders bevorzugten Bedeutungen haben.

Insbesondere bevorzugt sind Verbindungen der Formel (I), in welchen alle Reste die oben genannten insbesondere bevorzugten Bedeutungen haben.

Darüber hinaus ganz besonders bevorzugt sind Verbindungen der Formel (I), in welchen R¹ und R³ bis R¹⁰ die oben genannten bevorzugten Bedeutungen haben, Z gleich NR⁴R⁵, NR⁴R⁹, OR⁸, OR¹⁰ oder S(O)ₙR⁴ ist, n gleich 0, 1 oder 2 sein kann und R² für Fluor, Chlor oder Brom steht.

Darüber hinaus ganz besonders bevorzugt sind Verbindungen der Formel (I), in welchen R¹ und R³ bis R¹⁰ die oben genannten besonders bevorzugten Bedeutungen haben, Z gleich NR⁴R⁵, NR⁴R⁹, OR⁸, OR¹⁰ oder S(O)ₙR⁴ ist, n gleich 0, 1 oder 2 sein kann und R² für Fluor, Chlor oder Brom steht.

Darüber hinaus ganz besonders bevorzugt sind Verbindungen der Formel (I), in welchen R¹ und R³ bis R¹⁰ die oben genannten ganz besonders bevorzugten Bedeutungen haben, Z gleich NR⁴R⁵, NR⁴R⁹, OR⁸, OR¹⁰ oder S(O)ₙR⁴ ist, n gleich 0, 1 oder 2 sein kann und R² für Fluor, Chlor oder Brom steht.

Darüber hinaus ganz besonders bevorzugt sind Verbindungen der Formel (I), in welchen R¹ und R³ bis R¹⁰ die oben genannten bevorzugten Bedeutungen haben, Z gleich NR⁴R⁵, OR⁸, oder SR⁴ ist und R² für Fluor, Chlor oder Brom steht.

Darüber hinaus ganz besonders bevorzugt sind Verbindungen der Formel (I), in welchen R¹ und R³ bis R¹⁰ die oben genannten besonders bevorzugten Bedeutungen haben, Z gleich NR⁴R⁵, OR⁸, oder SR⁴ ist und R² für Fluor, Chlor oder Brom steht.

Darüber hinaus ganz besonders bevorzugt sind Verbindungen der Formel (I), in welchen R¹ und R³ bis R¹⁰ die oben genannten ganz besonders bevorzugten Bedeutungen haben, Z gleich NR⁴R⁵, OR⁸, oder SR⁴ ist und R² für Fluor, Chlor oder Brom steht.

Darüber hinaus ganz besonders bevorzugt sind Verbindungen der Formel (I), in welchen R¹ bis R¹⁰ die oben genannten bevorzugten Bedeutungen haben und Z gleich NR⁴R⁵ ist.

Darüber hinaus ganz besonders bevorzugt sind Verbindungen der Formel (I), in welchen R¹ bis R¹⁰ die oben genannten besonders bevorzugten Bedeutungen haben und Z gleich NR⁴R⁵ ist.

Darüber hinaus ganz besonders bevorzugt sind Verbindungen der Formel (I), in welchen R¹ bis R¹⁰ die oben genannten ganz besonders bevorzugten Bedeutungen haben und Z gleich NR⁴R⁵ ist.

Darüber hinaus ganz besonders bevorzugt sind Verbindungen der Formel (I), in welchen R¹ bis R¹⁰ die oben genannten bevorzugten Bedeutungen haben und Z gleich NR⁴R⁹ ist.

Darüber hinaus ganz besonders bevorzugt sind Verbindungen der Formel (I), in welchen R¹ bis R¹⁰ die oben genannten besonders bevorzugten Bedeutungen haben und Z gleich NR⁴R⁹ ist.

Darüber hinaus ganz besonders bevorzugt sind Verbindungen der Formel (I), in welchen R¹ bis R¹⁰ die oben genannten ganz besonders bevorzugten Bedeutungen haben und Z gleich NR⁴R⁹ ist.

Darüber hinaus ganz besonders bevorzugt sind Verbindungen der Formel (I), in welchen R¹ bis R¹⁰ die oben genannten bevorzugten Bedeutungen haben, Z gleich S(O)ₙR⁴ ist und n gleich 0, 1 oder 2 sein kann.

Darüber hinaus ganz besonders bevorzugt sind Verbindungen der Formel (I), in welchen R¹ bis R¹⁰ die oben genannten besonders bevorzugten Bedeutungen haben, Z gleich S(O)ₙR⁴ ist und n gleich 0, 1 oder 2 sein kann.

Darüber hinaus ganz besonders bevorzugt sind Verbindungen der Formel (I), in welchen R¹ bis R¹⁰ die oben genannten ganz besonders bevorzugten Bedeutungen haben, Z gleich S(O)ₙR⁴ ist und n gleich 0, 1 oder 2 sein kann.

Darüber hinaus ganz besonders bevorzugt sind Verbindungen der Formel (I), in welchen R¹ bis R¹⁰ die oben genannten bevorzugten Bedeutungen haben und Z gleich OR⁸ ist.

Darüber hinaus ganz besonders bevorzugt sind Verbindungen der Formel (I), in welchen R¹ bis R¹⁰ die oben genannten besonders bevorzugten Bedeutungen haben und Z gleich OR⁸ ist.

Darüber hinaus ganz besonders bevorzugt sind Verbindungen der Formel (I), in welchen R¹ bis R¹⁰ die oben genannten ganz besonders bevorzugten Bedeutungen haben und Z gleich OR⁸ ist.

Darüber hinaus ganz besonders bevorzugt sind Verbindungen der Formel (I), in welchen R¹ bis R¹⁰ die oben genannten bevorzugten Bedeutungen haben und Z gleich OR¹⁰ ist.

Darüber hinaus ganz besonders bevorzugt sind Verbindungen der Formel (I), in welchen R¹ bis R¹⁰ die oben genannten besonders bevorzugten Bedeutungen haben und Z gleich OR¹⁰ ist.

Darüber hinaus ganz besonders bevorzugt sind Verbindungen der Formel (I), in welchen R¹ bis R¹⁰ die oben genannten ganz besonders bevorzugten Bedeutungen haben und Z gleich OR¹⁰ ist.

Insbesondere bevorzugt sind Verbindungen der Formel (I), in welchen R¹, R² und R⁴ bis R¹⁰ die oben genannten besonders bevorzugten Bedeutungen haben, Z für NR⁴R⁵ steht und
- R³: steht für einen Phenylring, welcher einfach bis fünffach unabhängig voneinander durch folgende gleiche oder verschiedenene Reste substituiert sein kann:
Wasserstoff, Fluor, Chlor, Brom, Iod, Cyano, Nitro, Hydroxy, Mercapto, Amino, Formyl, Methyl, Ethyl, n-Propyl, Isopropyl, n-Butyl, Isobutyl, sek-Butyl, tert-Butyl, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2-Dichlor-2-fluoroethyl, 2-Chlor-2,2-difluorethyl, 2-Chlor-2-fluorethyl, 2,2,2-Trichlorethyl, 2-Fluorethyl, 2,2-Difluorethyl, 2,2,2-Trifluorethyl, Vinyl, Ethinyl, Allyl, Propargyl, Methoxy, Ethoxy, n-oder i-Propoxy, Methylthio, Ethylthio, n-Propylthio, Isopropylthio, Methylsulfinyl, Ethylsulfinyl, Methylsulfonyl, Ethylsulfonyl, Allyloxy, Propargyloxy, Difluormethoxy, Trifluormethoxy, Difluorchlormethoxy, 2,2,2-Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Methylamino, Ethylamino, n-Propylamino, Isopropylamino, Cyclopropylamino, Dimethylamino, Diethylamino, Acetyl, Propionyl, Acetyloxy, Methoxycarbonyl, Ethoxycarbonyl, Methoxymethyl, Ethoxymethyl, 2-Methoxyethoxy, 2-Ethoxyethoxy, (2-Methoxyethoxy)ethoxy, 2-(Dimethylamino)ethoxy, 2-Morpholin-4-ylethoxy, 3-(Dimethylamino)propoxy, 3-Morpholin-4-ylpropoxy, Hydroximinomethyl, Hydroximinoethyl, Methoximinomethyl, Ethoximinomethyl, N-Methoxyethanimidoyl, N-Ethoxyethanimidoyl, Cyclopropyl, Cyclobutyl, Cyclopentyl, Cyclohexyl, Phenyl oder Phenoxy;
- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl.

Insbesondere bevorzugt sind Verbindungen der Formel (I), in welcher R¹, R² und R⁴ bis R¹⁰ die oben genannten besonders bevorzugten Bedeutungen haben, Z für NR⁴R⁹ steht und
- R³: steht für einen Phenylring, welcher einfach bis fünffach unabhängig voneinander durch folgende gleiche oder verschiedenene Reste substituiert sein kann:
Wasserstoff, Fluor, Chlor, Brom, Iod, Cyano, Nitro, Hydroxy, Mercapto, Amino, Formyl, Methyl, Ethyl, n-Propyl, Isopropyl, n-Butyl, Isobutyl, sek-Butyl, tert-Butyl, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2-Dichlor-2-fluoroethyl, 2-Chlor-2,2-difluorethyl, 2-Chlor-2-fluorethyl, 2,2,2-Trichlorethyl, 2-Fluorethyl, 2,2-Difluorethyl, 2,2,2-Trifluorethyl, Vinyl, Ethinyl, Allyl, Propargyl, Methoxy, Ethoxy, n-oder i-Propoxy, Methylthio, Ethylthio, n-Propylthio, Isopropylthio, Methylsulfinyl, Ethylsulfinyl, Methylsulfonyl, Ethylsulfonyl, Allyloxy, Propargyloxy, Difluormethoxy, Trifluormethoxy, Difluorchlormethoxy, 2,2,2-Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Methylamino, Ethylamino, n-Propylamino, Isopropylamino, Cyclopropylamino, Dimethylamino, Diethylamino, Acetyl, Propionyl, Acetyloxy, Methoxycarbonyl, Ethoxycarbonyl, Methoxymethyl, Ethoxymethyl, 2-Methoxyethoxy, 2-Ethoxyethoxy, (2-Methoxyethoxy)ethoxy, 2-(Dimethylamino)ethoxy, 2-Morpholin-4-ylethoxy, 3-(Dimethylamino)propoxy, 3-Morpholin-4-ylpropoxy, Hydroximinomethyl, Hydroximinoethyl, Methoximinomethyl, Ethoximinomethyl, N-Methoxyethanimidoyl, N-Ethoxyethanimidoyl, Cyclopropyl, Cyclobutyl, Cyclopentyl, Cyclohexyl, Phenyl oder Phenoxy;
- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl.

Weiterhin insbesondere bevorzugt sind Verbindungen der Formel (I), in welchen R¹, R² und R⁴ bis R¹⁰ die oben genannten besonders bevorzugten Bedeutungen haben, Z für S(O)ₙR⁴ ist, n gleich 0, 1 oder 2 sein kann und
- R³: steht für einen Phenylring, welcher einfach bis fünffach unabhängig voneinander durch folgende gleiche oder verschiedenene Reste substituiert sein kann:
Wasserstoff, Fluor, Chlor, Brom, Iod, Cyano, Nitro, Hydroxy, Mercapto, Amino, Formyl, Methyl, Ethyl, n-Propyl, Isopropyl, n-Butyl, Isobutyl, sek-Butyl, tert-Butyl, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2-Dichlor-2-fluoroethyl, 2- Chlor-2,2-difluorethyl, 2-Chlor-2-fluorethyl, 2,2,2-Trichlorethyl, 2-Fluorethyl, 2,2-Difluorethyl, 2,2,2-Trifluorethyl, Vinyl, Ethinyl, Allyl, Propargyl, Methoxy, Ethoxy, n-oder i-Propoxy, Methylthio, Ethylthio, n-Propylthio, Isopropylthio, Methylsulfinyl, Ethylsulfinyl, Methylsulfonyl, Ethylsulfonyl, Allyloxy, Propargyloxy, Difluormethoxy, Trifluormethoxy, Difluorchlormethoxy, 2,2,2-Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Methylamino, Ethylamino, n-Propylamino, Isopropylamino, Cyclopropylamino, Dimethylamino, Diethylamino, Acetyl, Propionyl, Acetyloxy, Methoxycarbonyl, Ethoxycarbonyl, Methoxymethyl, Ethoxymethyl, 2-Methoxyethoxy, 2-Ethoxyethoxy, (2-Methoxyethoxy)ethoxy, 2-(Dimethylamino)ethoxy, 2-Morpholin-4-ylethoxy, 3-(Dimethylamino)propoxy, 3-Morpholin-4-ylpropoxy, Hydroximinomethyl, Hydroximinoethyl, Methoximinomethyl, Ethoximinomethyl, N-Methoxyethanimidoyl, N-Ethoxyethanimidoyl, Cyclopropyl, Cyclobutyl, Cyclopentyl, Cyclohexyl, Phenyl oder Phenoxy;
- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl.

Weiterhin insbesondere bevorzugt sind Verbindungen der Formel (I), in welchen R¹, R² und R⁴ bis R¹⁰ die oben genannten besonders bevorzugten Bedeutungen haben, Z für OR⁸ steht und
- R³: steht für einen Phenylring, welcher einfach bis fünffach unabhängig voneinander durch folgende gleiche oder verschiedenene Reste substituiert sein kann:
Wasserstoff, Fluor, Chlor, Brom, Iod, Cyano, Nitro, Hydroxy, Mercapto, Amino, Formyl, Methyl, Ethyl, n-Propyl, Isopropyl, n-Butyl, Isobutyl, sek-Butyl, tert-Butyl, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2-Dichlor-2-fluoroethyl, 2-Chlor-2,2-difluorethyl, 2-Chlor-2-fluorethyl, 2,2,2-Trichlorethyl, 2-Fluorethyl, 2,2-Difluorethyl, 2,2,2-Trifluorethyl, Vinyl, Ethinyl, Allyl, Propargyl, Methoxy, Ethoxy, n-oder i-Propoxy, Methylthio, Ethylthio, n-Propylthio, Isopropylthio, Methylsulfinyl, Ethylsulfinyl, Methylsulfonyl, Ethylsulfonyl, Allyloxy, Propargyloxy, Difluormethoxy, Trifluormethoxy, Difluorchlormethoxy, 2,2,2-Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Methylamino, Ethylamino, n-Propylamino, Isopropylamino, Cyclopropylamino, Dimethylamino, Diethylamino, Acetyl, Propionyl, Acetyloxy, Methoxycarbonyl, Ethoxycarbonyl, Methoxymethyl, Ethoxymethyl, 2-Methoxyethoxy, 2-Ethoxyethoxy, (2-Methoxyethoxy)ethoxy, 2-(Dimethylamino)ethoxy, 2-Morpholin-4-ylethoxy, 3-(Dimethylamino)propoxy, 3-Morpholin-4-ylpropoxy, Hydroximinomethyl, Hydroximinoethyl, Methoximinomethyl, Ethoximinomethyl, N-Methoxyethanimidoyl, N-Ethoxyethanimidoyl, Cyclopropyl, Cyclobutyl, Cyclopentyl, Cyclohexyl, Phenyl oder Phenoxy;
- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl.

Weiterhin insbesondere bevorzugt sind Verbindungen der Formel (I), in welchen R¹, R² und R⁴ bis R¹⁰ die oben genannten besonders bevorzugten Bedeutungen haben, Z für OR¹⁰ steht und
- R³: steht für einen Phenylring, welcher einfach bis fünffach unabhängig voneinander durch folgende gleiche oder verschiedenene Reste substituiert sein kann:
Wasserstoff, Fluor, Chlor, Brom, Iod, Cyano, Nitro, Hydroxy, Mercapto, Amino, Formyl, Methyl, Ethyl, n-Propyl, Isopropyl, n-Butyl, Isobutyl, sek-Butyl, tert-Butyl, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2-Dichlor-2-fluoroethyl, 2-Chlor-2,2-difluorethyl, 2-Chlor-2-fluorethyl, 2,2,2-Trichlorethyl, 2-Fluorethyl, 2,2-Difluorethyl, 2,2,2-Trifluorethyl, Vinyl, Ethinyl, Allyl, Propargyl, Methoxy, Ethoxy, n-oder i-Propoxy, Methylthio, Ethylthio, n-Propylthio, Isopropylthio, Methylsulfinyl, Ethylsulfinyl, Methylsulfonyl, Ethylsulfonyl, Allyloxy, Propargyloxy, Difluormethoxy, Trifluormethoxy, Difluorchlormethoxy, 2,2,2-Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Methylamino, Ethylamino, n-Propylamino, Isopropylamino, Cyclopropylamino, Dimethylamino, Diethylamino, Acetyl, Propionyl, Acetyloxy, Methoxycarbonyl, Ethoxycarbonyl, Methoxymethyl, Ethoxymethyl, 2-Methoxyethoxy, 2-Ethoxyethoxy, (2-Methoxyethoxy)ethoxy, 2-(Dimethylamino)ethoxy, 2-Morpholin-4-ylethoxy, 3-(Dimethylamino)propoxy, 3-Morpholin-4-ylpropoxy, Hydroximinomethyl, Hydroximinoethyl, Methoximinomethyl, Ethoximinomethyl, N-Methoxyethanimidoyl, N-Ethoxyethanimidoyl, Cyclopropyl, Cyclobutyl, Cyclopentyl, Cyclohexyl, Phenyl oder Phenoxy;
- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl.

Weiterhin insbesondere bevorzugt sind Verbindungen der Formel **(I)**, in welchen R¹, R² und R⁴ bis R¹⁰ die oben genannten besonders bevorzugten Bedeutungen haben, Z für NR⁴R⁵ steht und
- R³: steht für einen Phenylring, welcher einfach bis dreifach unabhängig voneinander durch folgende gleiche oder verschiedene Reste substituiert sein kann:
Chlor, Brom, Iod, Fluor, Fluormethyl, Difluormethyl, Trifluormethyl, Trifluormethoxy, Trifluormethylthio, Hydroxy, Methoxy, Methoxyethoxy, Nitro, Cyano, Methyl, Ethyl, Amino, Methylamino, Dimethylamino, 2-(Dimethylamino)ethoxy, 2-Morpholin-4-ylethoxy, 3-(Dimethylamino)propoxy oder 3-Morpholin-4-ylpropoxy;
- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach, unabhängig voneinander gleich oder verschieden substituiert sein können durch Fluor, Chlor, Methyl, Cyclopropyl und/oder Trifluormethyl;

Weiterhin insbesondere bevorzugt sind Verbindungen der Formel **(I)**, in welchen R¹, R² und R⁴ bis R¹⁰ die oben genannten besonders bevorzugten Bedeutungen haben, Z für NR⁴R⁹ steht und
- R³: steht für einen Phenylring, welcher einfach bis dreifach unabhängig voneinander durch folgende gleiche oder verschiedene Reste substituiert sein kann:
Chlor, Brom, Iod, Fluor, Fluormethyl, Difluormethyl, Trifluormethyl, Trifluormethoxy, Trifluormethylthio, Hydroxy, Methoxy, Methoxyethoxy, Nitro, Cyano, Methyl, Ethyl, Amino, Methylamino, Dimethylamino, 2-(Dimethylamino)ethoxy, 2-Morpholin-4-ylethoxy, 3-(Dimethylamino)propoxy oder 3-Morpholin-4-ylpropoxy;
- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach, unabhängig voneinander gleich oder verschieden substituiert sein können durch Fluor, Chlor, Methyl, Cyclopropyl und/oder Trifluormethyl;

Weiterhin insbesondere bevorzugt sind Verbindungen der Formel **(I)**, in welchen R¹, R² und R⁴ bis R¹⁰ die oben genannten besonders bevorzugten Bedeutungen haben, Z für S(O)ₙR⁴ ist, n gleich 0, 1 oder 2 sein kann und
- R³: steht für einen Phenylring, welcher einfach bis dreifach unabhängig voneinander durch folgende gleiche oder verschiedene Reste substituiert sein kann:
Chlor, Brom, Iod, Fluor, Fluormethyl, Difluormethyl, Trifluormethyl, Trifluormethoxy, Trifluormethylthio, Hydroxy, Methoxy, Methoxyethoxy, Nitro, Cyano, Methyl, Ethyl, Amino, Methylamino, Dimethylamino, 2-(Dimethylamino)ethoxy, 2-Morpholin-4-ylethoxy, 3-(Dimethylamino)propoxy oder 3-Morpholin-4-ylpropoxy;
- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach, unabhängig voneinander gleich oder verschieden substituiert sein können durch Fluor, Chlor, Methyl, Cyclopropyl und/oder Trifluormethyl;

Weiterhin insbesondere bevorzugt sind Verbindungen der Formel **(I)**, in welchen R¹, R² und R⁴ bis R¹⁰ die oben genannten besonders bevorzugten Bedeutungen haben, Z für OR⁸ steht und
- R³: steht für einen Phenylring, welcher einfach bis dreifach unabhängig voneinander durch folgende gleiche oder verschiedene Reste substituiert sein kann:
Chlor, Brom, Iod, Fluor, Fluormethyl, Difluormethyl, Trifluormethyl, Trifluormethoxy, Trifluormethylthio, Hydroxy, Methoxy, Methoxyethoxy, Nitro, Cyano, Methyl, Ethyl, Amino, Methylamino, Dimethylamino, 2-(Dimethylamino)ethoxy, 2-Morpholin-4-ylethoxy, 3-(Dimethylamino)propoxy oder 3-Morpholin-4-ylpropoxy;
- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach, unabhängig voneinander gleich oder verschieden substituiert sein können durch Fluor, Chlor, Methyl, Cyclopropyl und/oder Trifluormethyl;

Weiterhin insbesondere bevorzugt sind Verbindungen der Formel **(I)**, in welchen R¹, R² und R⁴ bis R¹⁰ die oben genannten besonders bevorzugten Bedeutungen haben, Z für OR¹⁰ steht und
- R³: steht für einen Phenylring, welcher einfach bis dreifach unabhängig voneinander durch folgende gleiche oder verschiedene Reste substituiert sein kann:
Chlor, Brom, Iod, Fluor, Fluormethyl, Difluormethyl, Trifluormethyl, Trifluormethoxy, Trifluormethylthio, Hydroxy, Methoxy, Methoxyethoxy, Nitro, Cyano, Methyl, Ethyl, Amino, Methylamino, Dimethylamino, 2-(Dimethylamino)ethoxy, 2-Morpholin-4-ylethoxy, 3-(Dimethylamino)propoxy oder 3-Morpholin-4-ylpropoxy;
- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach, unabhängig voneinander gleich oder verschieden substituiert sein können durch Fluor, Chlor, Methyl, Cyclopropyl und/oder Trifluormethyl;

Weiterhin insbesondere bevorzugt sind Verbindungen der Formel **(I)**, in welchen R¹, R² und R⁴ bis R¹⁰ die oben genannten besonders bevorzugten Bedeutungen haben, Z für NR⁴R⁵ oder NR⁴R⁹ steht und
- R³: steht für 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4-Triazol-1-yl, 1,2,4-Triazol-3-yl, 1,2,4-Triazol-4-yl, 1,2,4-Triazol-5-yl, 1,2,3-Triazol-1-yl, 1,2,3-Triazol-2-yl, 1,2,3-Triazol-4-yl, Tetrazol-1-yl, Tetrazol-2-yl, Tetrazol-5-yl, Indol-1-yl, Indol-2-yl, Indol-3-yl, Isoindol-1-yl, Isoindol-2-yl, Benzofur-2-yl, Benzothiophen-2-yl, Benzofur-3-yl, Benzothiophen-3-yl, Benzoxazol-2-yl, Benzothiazol-2-yl, Benzimidazol-2-yl, Indazol-1-yl, Indazol-2-yl, Indazol-3-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl oder 1,2,4-Triazin-6-yl, welches jeweils einfach oder mehrfach unabhängig voneinander durch gleiche oder verschiedenene Reste substituiert sein kann ausgewählt aus Wasserstoff, Fluor, Chlor, Brom, Iod, Cyano, Hydroxy, Mercapto, Amino, Methyl, Ethyl, n-Propyl, Isopropyl, n-Butyl, Isobutyl, sek-Butyl, tert-Butyl, Cyclopropyl, 1-Chlorcyclopropyl, Vinyl, Ethinyl, Methoxy, Ethoxy, Isopropoxy, Methylthio, Ethylthio, Trifluormethylthio, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2,2-Trifluorethyl, Trifluormethoxy, Trifluormethylthio, 2-Methoxyethoxy, Acetyl, Propionyl, Methoxycarbonyl, N-Methylamino, N,N-Dimethylamino, Aminocarbonyl, Methylaminocarbonyl, Dimethylaminocarbonyl, Dimethylcarbamoylamino, Methoxycarbonylamino, Methoxycarbonyloxy, Methylsulfamoyl, Dimethylsulfamoyl, Phenyl oder Phenoxy,
- oder: zwei benachbarte Reste am Heterocyclus bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl,
- oder: zwei Reste am Heterocyclus bilden ganz besonders bevorzugt mit dem Kohlenstoffatom, an das sie gebundenen sind, eine Carbonyl- oder Thiocarbonylgruppe.

Weiterhin insbesondere bevorzugt sind Verbindungen der Formel **(I)**, in welchen R¹, R² und R⁴ bis R¹⁰ die oben genannten besonders bevorzugten Bedeutungen haben, Z für S(O)ₙR⁴ ist, n gleich 0, 1 oder 2 sein kann und
- R³: steht für 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4-Triazol-1-yl, 1,2,4-Triazol-3-yl, 1,2,4-Triazol-4-yl, 1,2,4-Triazol-5-yl, 1,2,3-Triazol-1-yl, 1,2,3-Triazol-2-yl, 1,2,3-Triazol-4-yl, Tetrazol-1-yl, Tetrazol-2-yl, Tetrazol-5-yl, Indol-1-yl, Indol-2-yl, Indol-3-yl, Isoindol-1-yl, Isoindol-2-yl, Benzofur-2-yl, Benzothiophen-2-yl, Benzofur-3-yl, Benzothiophen-3-yl, Benzoxazol-2-yl, Benzothiazol-2-yl, Benzimidazol-2-yl, Indazol-1-yl, Indazol-2-yl, Indazol-3-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl oder 1,2,4-Triazin-6-yl,welches jeweils einfach oder mehrfach unabhängig voneinander durch gleiche oder verschiedenene Reste substituiert sein kann ausgewählt aus Wasserstoff, Fluor, Chlor, Brom, Iod, Cyano, Hydroxy, Mercapto, Amino, Methyl, Ethyl, n-Propyl, Isopropyl, n-Butyl, Isobutyl, sek-Butyl, tert-Butyl, Cyclopropyl, 1-Chlorcyclopropyl, Vinyl, Ethinyl, Methoxy, Ethoxy, Isopropoxy, Methylthio, Ethylthio, Trifluormethylthio, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2,2-Trifluorethyl, Trifluormethoxy, Trifluormethylthio, 2-Methoxyethoxy, Acetyl, Propionyl, Methoxycarbonyl, N-Methylamino, N,N-Dimethylamino, Aminocarbonyl, Methylaminocarbonyl, Dimethylaminocarbonyl, Dimethylcarbamoylamino, Methoxycarbonylamino, Methoxycarbonyloxy, Methylsulfamoyl, Dimethylsulfamoyl, Phenyl oder Phenoxy,
- oder: zwei benachbarte Reste am Heterocyclus bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl,
- oder: zwei Reste am Heterocyclus bilden ganz besonders bevorzugt mit dem Kohlenstoffatom, an das sie gebundenen sind, eine Carbonyl- oder Thiocarbonylgruppe.

Weiterhin insbesondere bevorzugt sind Verbindungen der Formel **(I)**, in welchen R¹, R² und R⁴ bis R¹⁰ die oben genannten besonders bevorzugten Bedeutungen haben, Z für OR⁸ oder OR¹⁰ steht und
- R³: steht für 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4-Triazol-1-yl, 1,2,4-Triazol-3-yl, 1,2,4-Triazol-4-yl, 1,2,4-Triazol-5-yl, 1,2,3-Triazol-1-yl, 1,2,3-Triazol-2-yl, 1,2,3-Triazol-4-yl, Tetrazol-1-yl, Tetrazol-2-yl, Tetrazol-5-yl, Indol-1-yl, Indol-2-yl, Indol-3-yl, Isoindol-1-yl, Isoindol-2-yl, Benzofur-2-yl, Benzothiophen-2-yl, Benzofur-3-yl, Benzothiophen-3-yl, Benzoxazol-2-yl, Benzothiazol-2-yl, Benzimidazol-2-yl, Indazol-1-yl, Indazol-2-yl, Indazol-3-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl oder 1,2,4-Triazin-6-yl,welches jeweils einfach oder mehrfach unabhängig voneinander durch gleiche oder verschiedenene Reste substituiert sein kann ausgewählt aus Wasserstoff, Fluor, Chlor, Brom, Iod, Cyano, Hydroxy, Mercapto, Amino, Methyl, Ethyl, n-Propyl, Isopropyl, n-Butyl, Isobutyl, sek-Butyl, tert-Butyl, Cyclopropyl, 1-Chlorcyclopropyl, Vinyl, Ethinyl, Methoxy, Ethoxy, Isopropoxy, Methylthio, Ethylthio, Trifluormethylthio, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2,2-Trifluorethyl, Trifluormethoxy, Trifluormethylthio, 2-Methoxyethoxy, Acetyl, Propionyl, Methoxycarbonyl, N-Methylamino, N,N-Dimethylamino, Aminocarbonyl, Methylaminocarbonyl, Dimethylaminocarbonyl, Dimethylcarbamoylamino, Methoxycarbonylamino, Methoxycarbonyloxy, Methylsulfamoyl, Dimethylsulfamoyl, Phenyl oder Phenoxy,
- oder: zwei benachbarte Reste am Heterocyclus bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl,
- oder: zwei Reste am Heterocyclus bilden ganz besonders bevorzugt mit dem Kohlenstoffatom, an das sie gebundenen sind, eine Carbonyl- oder Thiocarbonylgruppe.

Weiterhin insbesondere bevorzugt sind Verbindungen der Formel **(I)**, in welchen R¹, R² und R⁴ bis R¹⁰ die oben genannten Bedeutungen haben, Z für NR⁴R⁵ oder NR⁴R⁹ steht und
- R³: steht für einen Phenylring, welcher in 2-Position monosubstituiert, in 2,4-, 2,5- oder 2,6-Position bissubstituiert oder in 2,4,6-, 2,3,4- oder 2,4,5-Position trisubstituiert ist unabhängig voneinander durch folgende gleiche oder verschiedene Reste substituiert sein kann:
Chlor, Brom, Fluor, Trifluormethyl, Trifluormethoxy, Trifluormethylthio, Hydroxy, Methoxy, Methoxyethoxy, Nitro, Cyano, Methyl, Amino, Methylamino, Dimethylamino, 2-(Dimethylamino)ethoxy, 2-Morpholin-4-ylethoxy, 3-(Dimethylamino)propoxy oder 3-Morpholin-4-ylpropoxy;
- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach, unabhängig voneinander gleich oder verschieden substituiert sein können durch Fluor, Chlor, Methyl, Cyclopropyl und/oder Trifluormethyl;

Weiterhin insbesondere bevorzugt sind Verbindungen der Formel **(I)**, in welchen R¹, R² und R⁴ bis R¹⁰ die oben genannten Bedeutungen haben, Z für S(O)ₙR⁴ steht, n gleich 0, 1 oder 2 sein kann und
- R³: steht für einen Phenylring, welcher in 2-Position monosubstituiert, in 2,4-, 2,5- oder 2,6-Position bissubstituiert oder in 2,4,6-, 2,3,4- oder 2,4,5-Position trisubstituiert ist unabhängig voneinander durch folgende gleiche oder verschiedene Reste substituiert sein kann:
Chlor, Brom, Fluor, Trifluormethyl, Trifluormethoxy, Trifluormethylthio, Hydroxy, Methoxy, Methoxyethoxy, Nitro, Cyano, Methyl, Amino, Methylamino, Dimethylamino, 2-(Dimethylamino)ethoxy, 2-Morpholin-4-ylethoxy, 3-(Dimethylamino)propoxy oder 3-Morpholin-4-ylpropoxy;
- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach, unabhängig voneinander gleich oder verschieden substituiert sein können durch Fluor, Chlor, Methyl, Cyclopropyl und/oder Trifluormethyl;

Weiterhin insbesondere bevorzugt sind Verbindungen der Formel **(I)**, in welchen R¹, R² und R⁴ bis R¹⁰ die oben genannten Bedeutungen haben, Z für OR⁸ oder OR¹⁰ steht und
- R³: steht für einen Phenylring, welcher in 2-Position monosubstituiert, in 2,4-, 2,5- oder 2,6-Position bissubstituiert oder in 2,4,6-, 2,3,4- oder 2,4,5-Position trisubstituiert ist unabhängig voneinander durch folgende gleiche oder verschiedene Reste substituiert sein kann: Chlor, Brom, Fluor, Trifluormethyl, Trifluormethoxy, Trifluormethylthio, Hydroxy, Methoxy, Methoxyethoxy, Nitro, Cyano, Methyl, Amino, Methylamino, Dimethylamino, 2-(Dimethylamino)ethoxy, 2-Morpholin-4-ylethoxy, 3-(Dimethylamino)propoxy oder 3-Morpholin-4-ylpropoxy;
- oder: zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach, unabhängig voneinander gleich oder verschieden substituiert sein können durch Fluor, Chlor, Methyl, Cyclopropyl und/oder Trifluormethyl;

Weiterhin insbesondere bevorzugt sind Verbindungen der Formel **(I)**, in welchen R¹, R² und R⁴ bis R¹⁰ die oben genannten Bedeutungen haben, Z für NR⁴R⁵ oder NR⁴R⁹ steht und
- R³: steht für 2-Thienyl, 3-Thienyl, 2-Thiazolyl, 4-Thiazolyl, 2-Pyridinyl, 4-Pyridinyl, 3-Pyridazinyl, 2-Pyrazinyl, 2-Pyrimidinyl, 4-Pyrimidinyl, welches jeweils einfach bis dreifach unabhängig voneinander durch gleiche oder verschiedenene Reste substituiert sein kann ausgewählt aus Fluor, Chlor, Brom, Methyl, Methoxy, Trifluormethyl, Trifluormethoxy, Trifluormethylthio, 2-Methoxyethoxy, Acetyl, Methoxycarbonyl, N,N-Dimethylamino, Methoxycarbonylamino,
- oder: zwei benachbarte Reste am Heterocyclus bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl,
- oder: zwei Reste am Heterocyclus bilden ganz besonders bevorzugt mit dem Kohlenstoffatom, an das sie gebundenen sind, eine Carbonyl- oder Thiocarbonylgruppe.

Weiterhin insbesondere bevorzugt sind Verbindungen der Formel **(I)**, in welchen R¹, R² und R⁴ bis R¹⁰ die oben genannten Bedeutungen haben, Z für S(O)ₙR⁴ steht, n gleich 0, 1 oder 2 sein kann und
- R³: steht für 2-Thienyl, 3-Thienyl, 2-Thiazolyl, 4-Thiazolyl, 2-Pyridinyl, 4-Pyridinyl, 3-Pyridazinyl, 2-Pyrazinyl, 2-Pyrimidinyl, 4-Pyrimidinyl, welches jeweils einfach bis dreifach unabhängig voneinander durch gleiche oder verschiedenene Reste substituiert sein kann ausgewählt aus Fluor, Chlor, Brom, Methyl, Methoxy, Trifluormethyl, Trifluormethoxy, Trifluormethylthio, 2-Methoxyethoxy, Acetyl, Methoxycarbonyl, N,N-Dimethylamino, Methoxycarbonylamino,
- oder: zwei benachbarte Reste am Heterocyclus bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl,
- oder: zwei Reste am Heterocyclus bilden ganz besonders bevorzugt mit dem Kohlenstoffatom, an das sie gebundenen sind, eine Carbonyl- oder Thiocarbonylgruppe.

Weiterhin insbesondere bevorzugt sind Verbindungen der Formel **(I)**, in welchen R¹, R² und R⁴ bis R¹⁰ die oben genannten Bedeutungen haben, Z für OR⁸ oder OR¹⁰ steht und
- R³: steht für 2-Thienyl, 3-Thienyl, 2-Thiazolyl, 4-Thiazolyl, 2-Pyridinyl, 4-Pyridinyl, 3-Pyridazinyl, 2-Pyrazinyl, 2-Pyrimidinyl, 4-Pyrimidinyl, welches jeweils einfach bis dreifach unabhängig voneinander durch gleiche oder verschiedenene Reste substituiert sein kann ausgewählt aus Fluor, Chlor, Brom, Methyl, Methoxy, Trifluormethyl, Trifluormethoxy, Trifluormethylthio, 2-Methoxyethoxy, Acetyl, Methoxycarbonyl, N,N-Dimethylamino, Methoxycarbonylamino,
- oder: zwei benachbarte Reste am Heterocyclus bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl,
- oder: zwei Reste am Heterocyclus bilden ganz besonders bevorzugt mit dem Kohlenstoffatom, an das sie gebundenen sind, eine Carbonyl- oder Thiocarbonylgruppe.

Weiterhin insbesondere bevorzugt sind Verbindungen der Formel **(I)**, in welchen R¹, R² und R⁴ bis R¹⁰ die oben genannten Bedeutungen haben, Z für NR⁴R⁵ oder NR⁴R⁹ steht und
- R³: steht für 2-Thienyl, 3-Thienyl, 4-Thiazolyl, welches unsubstituiert oder bis zu zweifach unabhängig voneinander durch Chlor oder Methyl substituiert ist,
oder
- R³: steht für 2-Pyridinyl, welches unsubstituiert oder bis zu zweifach unabhängig voneinander durch Fluor, Chlor, Methoxy, Trifluormethyl oder Methyl substituiert ist, und zwar in den Positionen 3, 4 und 6 des Pyridylsubstituenten,
oder
- R³: steht für 3-Pyridazinyl, welches unsubstituiert oder bis zu zweifach unabhängig voneinander durch Fluor, Chlor oder Methyl substituiert ist, und zwar in den Positionen 4 und 6 des Pyridazinylsubstituenten,
oder
- R³: steht für 2-Pyrazinyl, welches unsubstituiert oder substituiert ist durch Chlor oder Methyl substituiert ist, und zwar in der Position 3 des Pyrazinylsubstituenten,
oder
- R³: steht für 2-Pyrimidinyl oder 4-Pyrimidinyl, welches jeweils unsubstituiert oder einfach substituiert ist durch Fluor, Chlor, Methyl, Methoxy.

Weiterhin insbesondere bevorzugt sind Verbindungen der Formel **(I)**, in welchen R¹, R² und R⁴ bis R¹⁰ die oben genannten Bedeutungen haben, Z für S(O)ₙR⁴ steht, n gleich 0, 1 oder 2 sein kann und
- R³: steht für 2-Thienyl, 3-Thienyl, 4-Thiazolyl, welches unsubstituiert oder bis zu zweifach unabhängig voneinander durch Chlor oder Methyl substituiert ist,
oder
- R³: steht für 2-Pyridinyl, welches unsubstituiert oder bis zu zweifach unabhängig voneinander durch Fluor, Chlor, Methoxy, Trifluormethyl oder Methyl substituiert ist, und zwar in den Positionen 3, 4 und 6 des Pyridylsubstituenten,
oder
- R³: steht für 3-Pyridazinyl, welches unsubstituiert oder bis zu zweifach unabhängig voneinander durch Fluor, Chlor oder Methyl substituiert ist, und zwar in den Positionen 4 und 6 des Pyridazinylsubstituenten,
oder
- R³: steht für 2-Pyrazinyl, welches unsubstituiert oder substituiert ist durch Chlor oder Methyl substituiert ist, und zwar in der Position 3 des Pyrazinylsubstituenten,
oder
- R³: steht für 2-Pyrimidinyl oder 4-Pyrimidinyl, welches jeweils unsubstituiert oder einfach substituiert ist durch Fluor, Chlor, Methyl, Methoxy.

Weiterhin insbesondere bevorzugt sind Verbindungen der Formel **(I)**, in welchen R¹, R² und R⁴ bis R¹⁰ die oben genannten Bedeutungen haben, Z für OR⁸ oder OR¹⁰ steht und
- R³: steht für 2-Thienyl, 3-Thienyl, 4-Thiazolyl, welches unsubstituiert oder bis zu zweifach unabhängig voneinander durch Chlor oder Methyl substituiert ist,
oder
- R³: steht für 2-Pyridinyl, welches unsubstituiert oder bis zu zweifach unabhängig voneinander durch Fluor, Chlor, Methoxy, Trifluormethyl oder Methyl substituiert ist, und zwar in den Positionen 3, 4 und 6 des Pyridylsubstituenten,
oder
- R³: steht für 3-Pyridazinyl, welches unsubstituiert oder bis zu zweifach unabhängig voneinander durch Fluor, Chlor oder Methyl substituiert ist, und zwar in den Positionen 4 und 6 des Pyridazinylsubstituenten,
oder
- R³: steht für 2-Pyrazinyl, welches unsubstituiert oder substituiert ist durch Chlor oder Methyl substituiert ist, und zwar in der Position 3 des Pyrazinylsubstituenten,
oder
- R³: steht für 2-Pyrimidinyl oder 4-Pyrimidinyl, welches jeweils unsubstituiert oder einfach substituiert ist durch Fluor, Chlor, Methyl, Methoxy.

Die zuvor genannten Reste-Definitionen können untereinander in beliebiger Weise kombiniert werden. Außerdem können einzelne Definitionen entfallen.

Je nach Art der oben definierten Substituenten weisen die Verbindungen der Formel (I) saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel (I) Amino, Alkylamino oder andere, basische Eigenschaften induzierende Gruppen, so können diese Verbindungen mit Säuren zu Salzen umgesetzt werden oder fallen durch die Synthese direkt als Salze an.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄.

Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkylresten mit 1 bis 20 Kohlenstoffatomen), Arylphosphonsäuren oder - diphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc..

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Tragen die Verbindungen der Formel (I) Hydroxy, Carboxy oder andere, saure Eigenschaften induzierende Gruppen, so können diese Verbindungen mit Basen zu Salzen umgesetzt werden.

Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Di- und Trialkanolamine von (C₁-C₄)-Alkanolen, Cholin sowie Chlorcholin.

Die so erhältlichen Salze weisen ebenfalls fungizide Eigenschaften auf.

Bei den in den vorstehenden Formeln angegebenen Definitionen der Symbole wurden Sammelbegriffe verwendet, die allgemein repräsentativ für die folgenden Substituenten stehen:

**Halogen**: Fluor, Chlor, Brom und Jod;

**Alkyl**: gesättigte, geradkettige oder verzweigte Kohlenwasserstoffreste mit 1 bis 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;

**Halogenalkyl:** geradkettige oder verzweigte Alkylgruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl;

**Alkenyl**: ungesättigte, geradkettige oder verzweigte Kohlenwasserstoffreste mit 2 bis 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;

**Alkinyl**: geradkettige oder verzweigte Kohlenwasserstoffgruppen mit 2 bis 8 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;

**Alkoxy**: gesättigte, geradkettige oder verzweigte Alkoxyreste mit 1 bis 8 Kohlenstoffatomen, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;

**Halogenalkoxy**: geradkettige oder verzweigte Alkoxygruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluorethoxy und 1,1,1-Trifluorprop-2-oxy;

**Alkylamino**: gesättigte, geradkettige oder verzweigte Alkylaminoreste mit 1 bis 8 Kohlenstoffatomen, z.B. C₁-C₆-Alkylamino wie Methylamino, Ethylamino, Propylamino, 1-Methylethylamino, Butylamino, 1-Methyl-propylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino,1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino und 1-Ethyl-2-methylpropylamino;

**Alkylthio**: gesättigte, geradkettige oder verzweigte Alkylthioreste mit 1 bis 8 Kohlenstoffatomen, z.B. C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;

**Halogenalkylthio**: geradkettige oder verzweigte Alkylthiogruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio;

**Alkylsulfinyl**: gesättigte, geradkettige oder verzweigte Alkylsulfinylreste mit 1 bis 8 Kohlenstoffatomen, z.B. C₁-C₆-Alkylsulfinyl wie Methylsulfinyl, Ethylsulfinyl, Propylsulfinyl, 1-Methylethylsulfinyl, Butylsulfinyl, 1-Methyl-propylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Di-methylpropylsulfinyl, 1-Ethylpropylsulfinyl, Hexylsulfinyl, 1,1-Dimethylpropylsulfinyl, 1,2-Dimethylpropylsulfinyl,1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;

**Alkylsulfonyl**: gesättigte, geradkettige oder verzweigte Alkylsulfonylreste mit 1 bis 8 Kohlenstoffatomen, z.B. C₁-C₆-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 2,2-Di-methylpropylsulfonyl, 1-Ethylpropylsulfonyl, Hexylsulfonyl, 1,1-Dimethylpropylsulfonyl, 1,2-Dimethylpropylsulfonyl,1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;

**Cycloalkyl**: monocyclische, gesättigte Kohlenwasserstoffgruppen mit 3 bis 8 Kohlenstoffringgliedern, wie Cyclopropyl, Cyclobutyl, Cyclopentyl und Cyclohexyl;

**Cylcoalkenyl**: monocyclische, nicht aromatische Kohlenwasserstoffgruppen mit 3 bis 8 Kohlenstoffringgliedern mit mindestens einer Doppelbindung, wie Cyclopenten-1-yl, Cyclohexen-1-yl, Cyclohepta-1,3-dien-1-yl;

**Alkoxycarbonyl**: eine Alkoxygruppe mit 1 bis 6 Kohlenstoffatomen (wie vorstehend genannt), welche über eine Carbonylgruppe (-CO-) an das Gerüst gebunden ist;

**Oxyalkylenoxy**: divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O;

**drei- bis zehngliedriger gesättigter oder partiell ungesättigter Heterocyclus, enthaltend ein bis vier Heteroatome aus der Gruppe Sauerstoff, Stickstoff oder Schwefel**: mono- oder bicyclische Heterocyclen (Heterocyclyl) enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; z.B. Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;

**fünf- bis zehngliedriger aromatischer Heterocyclus, enthaltend ein bis vier Heteroatome aus der Gruppe Sauerstoff, Stickstoff oder Schwefel: ein- oder zweikerniges Heteroaryl, z.B.**
- 5-gliedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
- **benzokondensiertes 5-gliedriges Heteroaryl, enthaltend ein bis drei Stickstoffatome oder ein Stickstoffatom und ein Sauerstoff- oder Schwefelatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können;
- **über Stickstoff gebundenes 5-gliedriges Heteroaryl, enthaltend ein bis vier Stickstoffatome, oder über Stickstoff gebundenes benzokondensiertes 5-gliederiges Heteroaryl, enthaltend ein bis drei Stickstoffatome**: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können, in der ein oder zwei C-Atome durch N-Atome ersetzt sein können, wobei diese Ringe über eines der Stickstoffringglieder an das Gerüst gebunden sind, z.B. 1-Pyrrolyl, 1-Pyrazolyl, 1,2,4-Triazol-1-yl, 1-Imidazolyl, 1,2,3-Triazol-1-yl, 1,3,4-Triazol-1-yl; -

**6-gliedriges Heteroaryl, enthaltend ein bis drei bzw. ein bis vier Stickstoffatome**: 6-Ring Heteroarylgruppen, welche neben Kohlenstoffatomen ein bis drei bzw. ein bis vier Stickstoffatome als Ringglieder enthalten können, beispielsweise 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;

**Abgangsgruppe**: S_{N}1 oder S_{N}2-Abgangsgruppe, beispielsweise Halogen (Chlor, Brom, Jod), Alkylsulfonate (-OSO₂-Alkyl, z.B. -OSO₂CH₃, -OSO₂CF₃) oder Arylsulfonate (-OSO₂-Aryl, z.B. -OSO₂Ph, -OSO₂PhMe);

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

Ein weiterer Gegenstand der vorliegenden Erfindung betrifft Verfahren zum Herstellen der erfindungsgemäßen Pyridazine der Formel **(I)**, umfassend wenigstens einen der folgenden Schritte (a) bis (k):
(a) Halogenierung von Hydroxypyridazinonen **(VII)** mit einem Halogenierungsmittel und gegebenenfalls einer Base zu Dihalogenpyridazinen **(VIII)** und gegebenenfalls weitere Umsetzung mit Fluorid und gegebenenfalls einem Katalysator gemäß nachfolgendem Reaktionsschema: 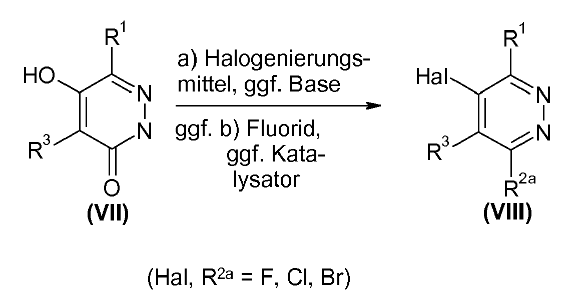
(b) Umsetzung von Dihalogenpyridazinen **(VIII)** mit Thioalkoholen R⁴-SH gegebenenfalls in Gegenwart einer Base gemäß nachfolgendem Reaktionsschema: 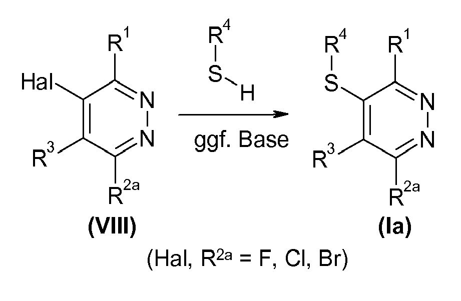
(c) Umsetzung von Dihalogenpyridazinen **(VIII)** mit Alkoholen R⁸-OH gegebenenfalls in Gegenwart einer Base gemäß nachfolgendem Reaktionsschema: 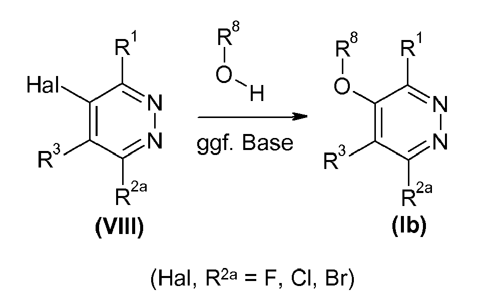
(d) Umsetzung von Dihalogenpyridazinen **(VIII)** mit Aminen R⁴R⁵NH und gegebenenfalls einer zusätzlichen Base gemäß nachfolgendem Reaktionsschema: 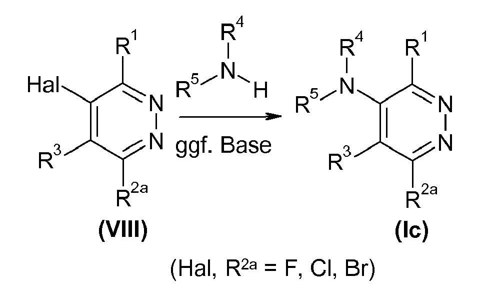
(e) Umsetzung von Pyridazinen der Formeln **(Ia), (Ib)** oder **(Ic)** mit einer Cyanidquelle gegebenenfalls in Gegenwart eines Katalysators gemäß nachfolgendem Reaktionsschema: 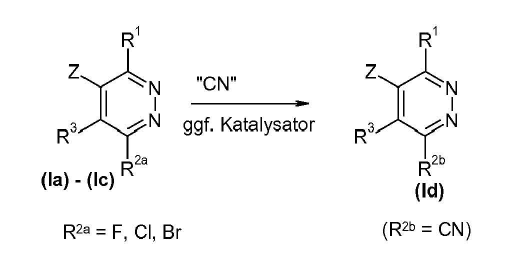
(f) Umsetzung von Pyridazinen der Formeln **(Ia), (Ib)** oder **(Ic)** mit einer Verbindung R^{2c}-SH gegebenenfalls in Gegenwart einer Base gemäß nachfolgendem Reaktionsschema: 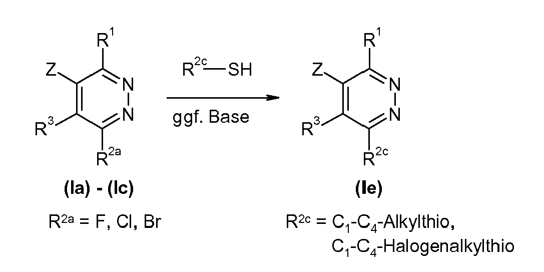
(g) Umsetzung von Pyridazinen der Formeln **(Ia), (Ib)** oder **(Ic)** mit einer Verbindung R^{2d}-OH gegebenenfalls in Gegenwart einer Base gemäß nachfolgendem Reaktionsschema: 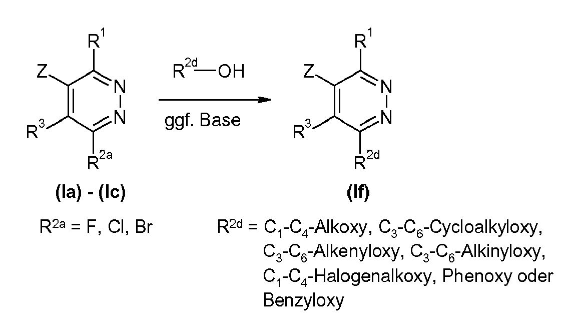
(h) Umsetzung von Pyridazinen der Formel **(Ie)** in Gegenwart eines Oxidationsmittels, gegebenenfalls in Gegenwart eines Katalysators und / oder gegebenenfalls in Gegenwart einer Base gemäß nachfolgendem Reaktionsschema: 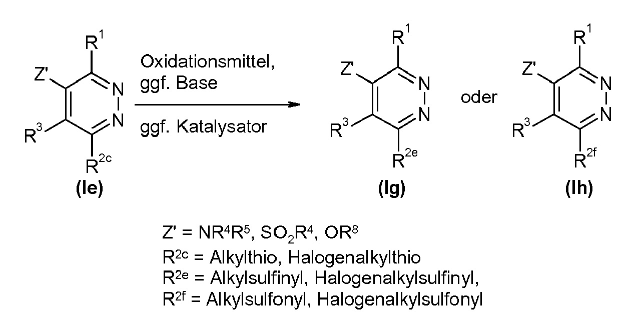
(i) Umsetzung von Pyridazinen der Formel **(Ia')** mit einem Oxidationsmittel gegebenenfalls in Gegenwart einer Base und / oder gegebenenfalls in Gegenwart eines Katalysators gemäß nachfolgendem Reaktionsschema: 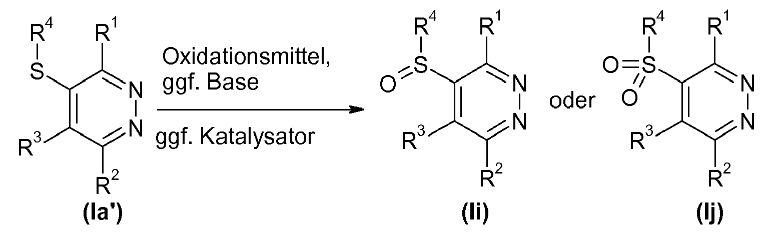
(j) Umsetzung von Pyridazinen der Formel **(Ic')** mit einer Verbindung R⁹-Y, gegebenenfalls in Gegenwart eines Katalysators und/ oder gegebenenfalls in Gegenwart einer Base gemäß nachfolgendem Reaktionsschema: 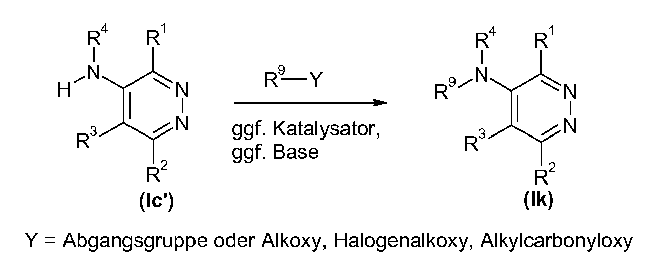
(k) Umsetzung von Pyridazinen der Formel **(Im)** mit einer Verbindung R¹⁰-Y, gegebenenfalls in Gegenwart eines Katalysators und / oder gegebenenfalls in Gegenwart einer Base gemäß nachfolgendem Reaktionsschema:

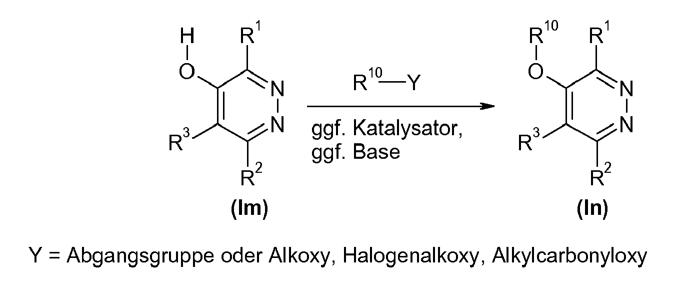

Wobei die Definitionen der Reste R¹ bis R¹⁰ in den obigen Schemata den oben angegebenen Definitionen entsprechen, Hal für Chlor, Brom oder Fluor stehen kann und R^{2a}, R^{2b}, R^{2c}, R^{2d}, R^{2e} und R^{2f}, Z' und Y den jeweils angegebenen Bedeutungen entsprechen.

Eine Möglichkeit zur Darstellung der Zwischenstufe **(VII),** deren Umsetzung zu erfindungsgemäßen Verbindungen der Formel **(VIII)** sowie **(Ia) - (In)** führt, ist in Schema 1 gezeigt.

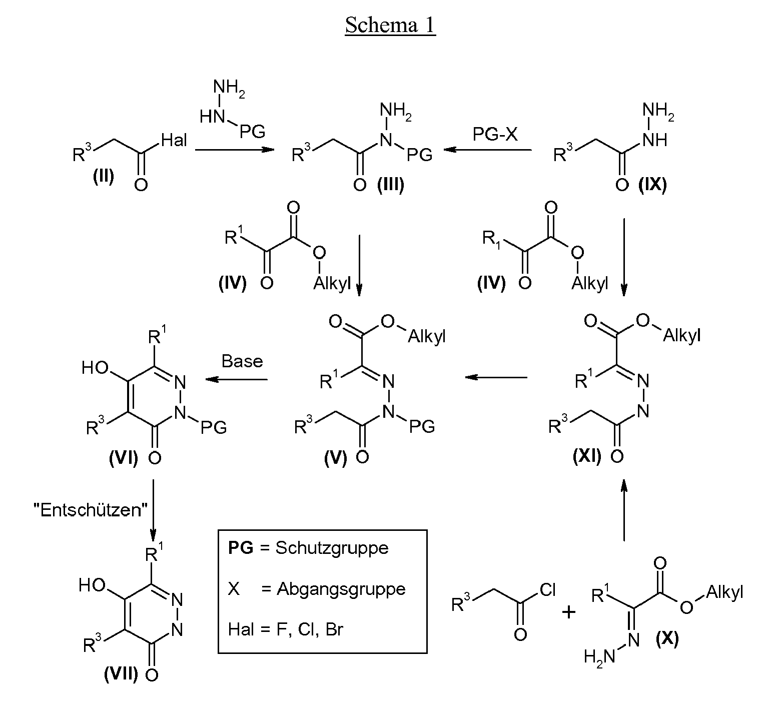

Geeignete substituierte Essigsäurederivate, beispielsweise substituierte Essigsäurehalogenide, die zur Herstellung von Verbindungen der Formel **(II)** dienen können, sind zum Teil kommerziell verfügbar oder lassen sich nach literaturbekannten Methoden herstellen. So sind verschiedene Methoden zur Synthese substituierter Arylessigsäurederivate beispielsweise aus WO 2005/075401, WO 2001/96277, WO 1996/35664 und WO 1996/25395 bekannt. Bestimmte Essigsäurederivate werden beispielsweise unter Verwendung von Essigesterenolaten in Gegenwart von Palladiumkatalysatoren, z.B. gebildet aus einer Palladiumquelle (z.B. Pd₂(dba)₃ oder Pd(OAc)₂) und einem Liganden (z.B. (t-Bu)₃P, iMes*HCl oder 2'-(N,N-Dimethylamino)-2-(dicyclohexylphosphanyl)biphenyl) hergestellt (WO 2005/048710, J. Am. Chem. Soc 2002. 124,. 12557, J. Am. Chem. Soc 2003. 125, 11176 oder J. Am. Chem. Soc. 2001, 123, 799). Weiterhin ist die Synthese bestimmter Heteroarylessigsäurederivate bekannt (beispielsweise beschrieben in WO 2001/96333, WO 2007/066601, WO 2007/080720 und Tetrahedron 2006, 62, 9919). Darüber hinaus lassen sich bestimmte substituierte Aryl- oder Heteroarylhalogenide unter Kupferkatalyse in die korrespondierenden substituierten Malonester überführen (z.B. beschrieben in Org. Lett. 2002, 2, 269, WO 2004/108727), welche nach bekannten Methoden in Aryl- bzw. Heteroarylessigsäuren überführt werden können.

Die Umsetzung von substituierten Essigsäurederivaten **(II)** mit einem einfach geschützten Hydrazinderivat liefert substituierte Hydrazide der Formel **(III).** Als geschütztes Hydrazinderivat kann beispielsweise ein Benzyl-substituiertes Hydrazin (analog z.B. beschrieben in *J. Org. Chem.* **1980**, 45, 3673) verwendet werden.

Alternativ ist die Herstellung von Hydraziden der Formel **(III)** durch die Umsetzung geeigneter Essigsäurehydrazide **(IX)** mit Benzylhalogeniden beschrieben (siehe z.B. *Aus. J. Chem.* **1984**, 37, 893). Die anschliessende Umsetzung mit 2-Ketoestern der Formel **(IV)** liefert Hydrazone der Formel **(V)**.

Alternativ lässt sich eine Zwischenstufe **(XI)** ausgehend von geeigneten Essigsäurehydraziden **(IX)** durch Umsetzung mit 2-Ketoestern der Formel **(IV)** darstellen (beispielsweise analog zu *Zh. Obs. Khim.* 1992, 62, 2262). Darüber hinaus können Hydrazone der Formel **(XI)** durch Acylierung von Hydrazonen der Formel **(X)** mit Acylchloriden erhalten werden. Durch Einführen einer Schutzgruppe PG lassen sich Verbindungen der Formel **(XI)** leicht in die entsprechenden N-geschützten Hydrazone der Formel **(V)** überführen (siehe beispielsweise T.W. Greene, P.G.M. Wuts, "Protective Groups in Organic Synthesis ", 3. Auflage, Wiley New York 1999; P.J. Kocienski, "Protecting Groups", 3. Auflage, G. Thieme Verlag, Stuttgart 2005).

Die so erhaltenen Hydrazone **(V)** lassen sich durch Verwendung geeigneter Basen, beispielsweise Natriumhydrid, Lithiumdiisopropylamid, LiHMDS oder Kalium-tert.-butylat, in einem geeigneten Lösungsmittel in die korrespondierenden N-geschützten 5-Hydroxypyridazinone **(VI)** überführen Als Verdünnungsmittel kommen bei der Durchführung der Reaktion alle für derartige Kondensationen üblichen Solventien in Frage; vorzugsweise verwendbar sind beispielsweise DMF oder NMP. Die Reaktion wird üblicherweise in einem Temperaturbereich von -20 °C bis 180 °C durchgeführt; vorzugsweise arbeitet man bei Temperaturen zwischen 20 °C und 150 °C.

Nach dem Entfernen der Schutzgruppe werden 5-Hydroxypyridazin-3-one der Formel **(VII)** erhalten; so gelingt beispielsweise die Entschützung von N-Benzyl-geschützten 5-Hydroxypyridazinonen **(VI**; PG = Benzyl) durch Einwirkung von Lewis-Säuren wie beispielsweise Aluminiumtrichlorid oder Bortrifluorid-Etherat. Als Verdünnungsmittel kommen bei der Durchführung der Reaktion alle für derartige Reaktionen üblichen inerten Solventien in Frage; vorzugsweise verwendbar sind beispielsweise . Toluol oder Chlorbenzol (siehe z.B. WO 2000/24719). Die Reaktion wird üblicherweise in einem Temperaturbereich von 0 °C bis 180 °C durchgeführt; vorzugsweise arbeitet man bei Temperaturen zwischen 20 °C und 150 °C.

Die Umsetzung der 5-Hydroxypyridazin-3-one **(VII)** mit einem Halogenierungsmittel gemäß Verfahren **a)** liefert die korrespondierenden 3,5-Dialogenpyridazine **(VIII)** (Hal und R^{2a} = Chlor, Brom) (siehe Schema 2). Als Halogenierungsmittel kommen bei der Durchführung des erfindungsgemäßen Verfahrens **a)** alle für den Ersatz von Hydroxygruppen durch Chlor oder Brom üblichen Substanzen in Betracht. Vorzugsweise verwendbar sind Phosphortrichlorid, Phosphortribromid, Phosphoroxybromid, Phosphorpentachlorid, Phosphoroxychlorid, Thionylchlorid, Oxalylchlorid, Thionylbromid oder deren Gemische oder Phosgen, Di- oder Triphosgen. Gegebenenfalls wird Chlor zu den genannten Halogenierungsmitteln oder ihren Mischungen gegeben. 3-Fluorpyridazine der Formel **(VIII)** (Hal und R^{2a} = Fluor) lassen sich aus den Chlor- oder Bromverbindungen durch Umsetzung mit einer Fluoridquelle, beispielsweise Kaliumfluorid, ggf. in Gegenwart eines Katalysators, beispielsweise Tetraphenylphosphoniumbromid, herstellen. Als Lösungsmittel kann beispielsweise Sulfolan verwendet werden.

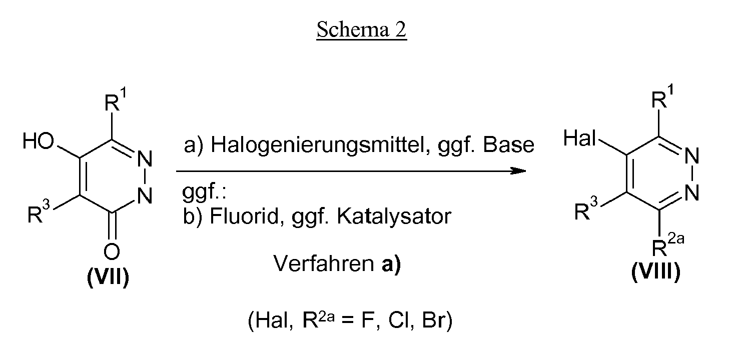

Die Reaktion wird üblicherweise in einem Temperaturbereich von 0 °C bis 180 °C durchgeführt; vorzugsweise arbeitet man bei Temperaturen zwischen 20 °C und 150 °C.

Als Verdünnungsmittel kommen bei der Durchführung des erfindungsgemäßen Verfahrens **a)** alle für derartige Halogenierungen üblichen Solventien in Frage. Vorzugsweise verwendbar sind halogenierte aliphatische oder aromatische Kohlenwasserstoffe wie beispielsweise Chlorbenzol. Als Verdünnungsmittel kann aber auch das Halogenierungsmittel selbst, z.B. Phosphoroxychlorid oder ein Gemisch von Halogenierungsmitteln fungieren.

Als Säureakzeptoren kommen bei der Durchführung des erfindungsgemäßen Verfahrens **a)** gegebenenfalls alle für derartige Umsetzungen üblichen anorganischen und organischen Basen in Frage. Vorzugsweise verwendbar sind Erdalkalimetall- oder Alkalimetallhydroxide, -acetate, -carbonate, -hydrogencarbonate oder -phosphate, wie beispielsweise Natriumhydroxid, Kaliumhydroxid, Natriumacetat, Natriumcarbonat, Kaliumcarbonat, Kaliumhydrogencarbonat, Natriumhydrogencarbonat, Cäsiumcarbonat oder Silberphosphat, sowie tertiäre Amine wie beispielsweise N,N-Diethylanilin oder Ethyldiisopropylamin.

Bei der Durchführung des Verfahrens **a)** setzt man Verbindungen der Formel **(VII)** im Allgemeinen mit einem Überschuss an Halogenierungsmittel um. Die Aufarbeitung erfolgt nach üblichen Methoden. (2*H*)-Pyridazinone der Formel **(VII),** bei denen R¹ und R³ bis R¹⁰ die oben angegebenen Bedeutungen haben, sowie deren Umsetzung in die korrespondierenden Verbindungen der Formel **(VIII)** (mit Hal, R^{2a} = Fluor, Chlor, Brom) gemäß Verfahren **a)** sind neu und daher ebenfalls Gegenstand der Erfindung.

Durch Umsetzung von Dichlorpyridazinen der Formel **(VIII)** mit verschiedenen Nukleophilen können die korrespondierenden Verbindungen der Formel **(Ia)** bis **(Ic)** erhalten werden. So ergibt beispielsweise die Umsetzung von Verbindungen der Formel **(VIII)** mit Thioalkoholen, Alkoholen oder Aminen die korrespondierenden 5-thiosubstituierten Pyridazine **(Ia),** die 5-oxysubstituierten Pyridazine **(Ib)** beziehungsweise die 5-aminosubstituierten Pyridazine **(Ic)** (siehe Schema 3).

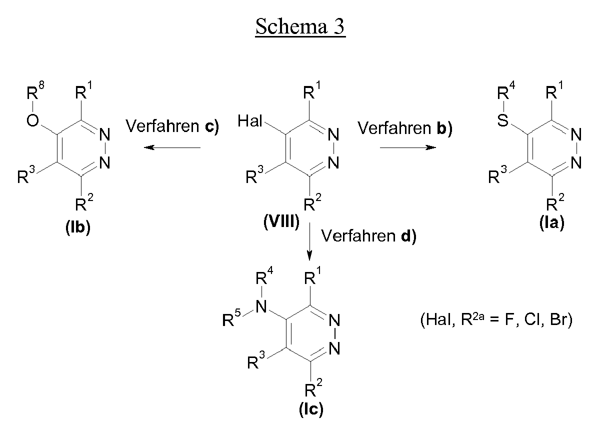

So ist beispielsweise die Kupfer-vermittelte C-O-, C-S- oder C-N-Kupplung zwischen Alkoholen, Aminen oder Thioalkoholen und Aryl- oder Hetarylhalogeniden in der Literatur beschrieben (siehe z.B. J. Am. Chem. Soc. 2006, 128, 8742; J. Am. Chem. Soc. 2007, 129, 3490; Synlett 2003, 2428; Angew. Chem. 2003, 115, 5558). Als Kupferquellen werden neben elementarem Kupfer üblicherweise Kupfer-(I)-Salze wie beispielsweise CuI, CuBr, CuCl, CuOAc oder Cu(OTf) verwendet. Als Liganden für Kupfer können beispielsweise Phenanthrolin, 2,2,6,6-Tetramethylheptan-3,5-dion, 2-(2-Methylpropanoyl-)cyclohexanon, Prolin oder N-Methylglycin verwendet werden.

Die Reaktion findet typischerweise in Gegenwart einer Base, z.B. Natriumcarbonat, Kaliumcarbonat, Cäsiumcarbonat oder Kaliumphosphat, und in einem Lösungsmittel wie beispielsweise THF, Dioxan, 1,2-Dimethoxyethan oder Toluol statt. Die Reaktion wird üblicherweise in einem Temperaturbereich von 0 °C bis 180 °C durchgeführt; vorzugsweise arbeitet man bei Temperaturen zwischen 20 °C und 150 °C.

Darüber hinaus sind Beispiele von Palladium-vermittelten C-O-, C-S- oder C-N-Kupplungen zwischen Alkoholen, Aminen oder Thioalkoholen und Aryl- oder Hetarylhalogeniden bekannt (siehe z.B. Top. Curr. Chem. 2002, 219, 131; Handbook of Organopalladium Chemistry for Organic Synthesis 2002, 1, 1051; J. J. Li, G. W. Gribble, "Palladium in Heterocyclic Chemistry", Pergamon, Amsterdam, 1. Auflage 2000).

Bestimmte 5-thiosubstituierte Pyridazine der Formel **(Ia),** bei denen R¹ und R³ bis R¹⁰ die oben angegebenen Bedeutungen haben und R^{2a} für Fluor, Chlor oder Brom steht, lassen sich durch Substitution von 5-halogensubstituierten Pyridazinen mit Thioalkoholen darstellen; zu diesem Zweck werden Verbindungen der Formel **(VIII),** bei denen Hal und R^{2a} für Fluor, Chlor oder Brom stehen, mit stöchiometrischen Mengen geeigneter Thioalkohole der Formel R⁴-SH in Gegenwart von typischen organischen oder anorganischen Basen, beispielsweise Natriumcarbonat, Kaliumcarbonat, Cäsiumcarbonat, Triethylamin, Ethyldiisopropylamin, DBU, DBN, DABCO, Natrium-tert-butylat, Kalium-tert-butylat, LiHMDS, NaHMDS, KHMDS, Lithiumdiisopropylamid oder Natriumhydrid, gemäß dem erfindungsgemäßen Verfahren **b)** umgesetzt.

Die Reaktion wird üblicherweise in einem Temperaturbereich von 0 °C bis 180 °C durchgeführt; vorzugsweise arbeitet man bei Temperaturen zwischen 20 °C und 150 °C.

Als Verdünnungsmittel kommen bei der Durchführung des erfindungsgemäßen Verfahrens **b)** alle für derartige Substitutionsreaktionen üblichen Solventien in Frage. Vorzugsweise verwendbar sind beispielsweise 1,4-Dioxan, N,N-Dimethylformamid, N-Methylpyrrolidon oder Dimethylsulfoxid. Darüber hinaus kann der Thioalkohol R⁴-SH selbst im Überschuß eingesetzt und als Lösungsmittel verwendet werden.

Dihalogenpyridazine **(VIII),** bei denen R¹ und R³ die oben angegebenen Bedeutungen haben und Hal und R^{2a} für Fluor, Chlor oder Brom stehen, sowie deren Umsetzung mit Thioalkoholen R⁴-SH in Gegenwart einer Base in die korrespondierenden Verbindungen der Formel **(Ia),** bei denen R^{2a} für Fluor, Chlor oder Brom steht, gemäß Verfahren **b)** sind neu und daher ebenfalls Gegenstand der Erfindung.

Bestimmte 5-oxysubstituierte Pyridazine der Formel **(Ib),** bei denen R¹ und R³ bis R¹⁰ die oben angegebenen Bedeutungen haben und R^{2a} für Fluor, Chlor oder Brom steht, lassen sich durch Substitution von 5-halogensubstituierten Pyridazinen mit Alkoholen darstellen; zu diesem Zweck werden Verbindungen der Formel **(VIII),** bei denen Hal und R^{2a} für Fluor, Chlor, oder Brom stehen, mit stöchiometrischen Mengen geeigneter Alkohole der Formel R⁸-OH in Gegenwart von typischen organischen oder anorganischen Basen, beispielsweise Natriumcarbonat, Kaliumcarbonat, Cäsiumcarbonat, Triethylamin, Ethyldiisopropylamin, DBU, DBN, DABCO, Natrium-tert-butylat, Kalium-tert-butylat, LiHMDS, NaHMDS, KHMDS, Lithiumdiisopropylamid oder Natriumhydrid, gemäß Verfahren c) umgesetzt.

Die Reaktion wird üblicherweise in einem Temperaturbereich von 0 °C bis 180 °C durchgeführt; vorzugsweise arbeitet man bei Temperaturen zwischen 20 °C und 150 °C.

Als Verdünnungsmittel kommen bei der Durchführung des erfindungsgemäßen Verfahrens **c)** alle für derartige Substitutionsreaktionen üblichen Solventien in Frage. Vorzugsweise verwendbar sind beispielsweise 1,4-Dioxan, N,N-Dimethylformamid, N-Methylpyrrolidon oder Dimethylsulfoxid. Darüber hinaus kann der Alkohol R⁸-OH selbst im Überschuß eingesetzt und als Lösungsmittel verwendet werden.

Dihalogenpyridazine **(VIII)**, bei denen R¹ und R³ die oben angegebenen Bedeutungen haben und Hal und R^{2a} für Fluor, Chlor oder Brom stehen, sowie deren Umsetzung, gegebenenfalls in Gegenwart eines Katalysators, mit Alkoholen R⁸-OH in die korrespondierenden Verbindungen der Formel **(Ib),** bei denen R^{2a} für Fluor, Chlor oder Brom steht, gemäß Verfahren **c)** sind neu und daher ebenfalls Gegenstand der Erfindung.

Bestimmte 5-aminosubstituierte Pyridazine der Formel **(Ic),** bei denen R¹ und R³ bis R¹⁰ die oben angegebenen Bedeutungen haben und R^{2a} für Fluor, Chlor oder Brom steht, lassen sich außerdem durch Substitution von 5-halogensubstituierten Pyridazinen mit Aminen darstellen; zu diesem Zweck werden Verbindungen der Formel **(VIII)** bei denen Hal und R^{2a} für Fluor, Chlor oder Brom stehen, mit stöchiometrischen Mengen geeigneter Amine der Formel (R⁴R⁵)NH gegebenenfalls in Gegenwart von typischen organischen oder anorganischen Basen, beispielsweise Natriumcarbonat, Kaliumcarbonat, Cäsiumcarbonat, Triethylamin, Ethyldiisopropylamin, DBU, DBN, DABCO, Natrium-tert-butylat, Kalium-tert-butylat, LiHMDS, NaHMDS, KHMDS, Lithiumdiisopropylamid, Natriumhydrid oder Amin (R⁴R⁵)NH, gemäß Verfahren **d)** umgesetzt.

Als Verdünnungsmittel kommen bei der Durchführung des erfindungsgemäßen Verfahrens **d)** alle für derartige Substitutionsreaktionen üblichen Solventien in Frage. Vorzugsweise verwendbar sind beispielsweise 1,4-Dioxan, N,N-Dimethylformamid, N-Methylpyrrolidon oder Dimethylsulfoxid. Darüber hinaus kann das Amin (R⁴R⁵)NH selbst im Überschuss eingesetzt und sowohl als Base als auch als Lösungsmittel Verwendung finden.

Die Reaktion wird üblicherweise in einem Temperaturbereich von 0 °C bis 180 °C durchgeführt; vorzugsweise arbeitet man bei Temperaturen zwischen 20 °C und 150 °C.

Dihalogenpyridazine **(VIII)**, bei denen R¹ und R³ die oben angegebenen Bedeutungen haben und Hal und R^{2a} für Fluor, Chlor oder Brom stehen, sowie deren Umsetzung in Gegenwart einer Base mit Aminen R⁴R⁵NH in die korrespondierenden Verbindungen der Formel **(Ic),** bei denen R^{2a} für Fluor, Chlor oder Brom steht, gemäß Verfahren **d)** sind neu und daher ebenfalls Gegenstand der Erfindung.

Bestimmte Pyridazine der Formel **(Id),** bei denen R^{2b} für Cyano steht, werden erhalten, indem man 3-halogensubstituierte Pyridazine der Formel **(Ia), (Ib)** oder **(Ic),** bei denen R¹ und R³ bis R¹⁰ die oben angegebenen Bedeutungen haben, Z für NR⁴R⁵, OR⁸ oder S(O)ₙR⁴ steht, n gleich 0, 1 oder 2 sein kann und R^{2a} für Fluor, Chlor oder Brom steht, gemäß Verfahren **e)** mit einer Cyanidquelle, gegebenenfalls in Gegenwart eines Katalysators und gegebenenfalls in Gegenwart eines Verdünnungsmittels umsetzt (siehe Schema 4).

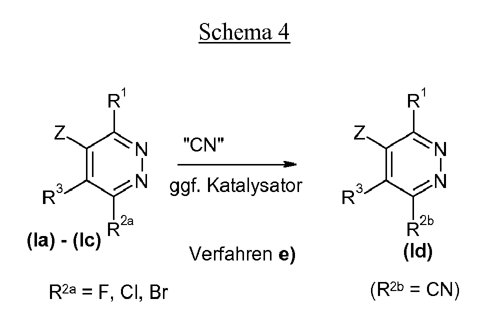

Die Einführung einer Cyanogruppe gelingt beispielsweise durch die Reaktion von z.B. einem Alkalicyanid, z.B. Kalium- oder Natriumcyanid, oder einer alternativen Cyanidquelle wie beispielsweise Kupfercyanid, Zinkcyanid, Trimethylsilylcyanid oder K₄[Fe(CN₆)] in einem geeigneten Lösungsmittel, beispielsweise N,N-Dimethylformamid oder Dimethylsulfoxid, in einem Temperaturbereich von 20 bis 150°C. Alternativ ist es möglich, die Reaktion in Gegenwart eines Katalysators, beispielsweise gebildet aus einer Palladiumquelle wie z.B. Pd₂(dba)₃ oder Pd(OAc)₂ und einem Phosphanliganden wie z.B. 1,10-Bis(diphenylphosphino)ferrocen (dppf) oder Butyldiadamantylphosphan, durchzuführen (analog beispielsweise beschrieben in Tetrahedron Lett. 2007, 48, 1087; DE-A 05/102005009517, Tetrahedron Lett. 2000, 41, 3271). Alternativ kann die Cyanierung kupferkatalysiert erfolgen, z.B. unter Verwendung einer Kupferquelle wie z.B. CuI und einem Liganden wie beispielsweise N,N'-Dimethylethylendiamin (analog beispielsweise beschrieben in J. Am. Chem. Soc. 2003, 125, 2890).

Die zur Durchführung des erfindungsgemässen Verfahrens **e)** als Ausgangsstoffe benötigten Pyridazine der Formel **(Ia), (Ib)** und **(Ic)** werden bereits oben beschrieben.

Substituierte Pyridazine **(Ia), (Ib)** und **(Ic),** bei denen R¹ und R³ bis R¹⁰ die oben angegebenen Bedeutungen haben, Z für NR⁴R⁵, OR⁸ oder S(O)ₙR⁴ steht, n gleich 0, 1 oder 2 sein kann und R^{2a} für Fluor, Chlor oder Brom steht, sowie deren weitere Umsetzung in die korrespondierenden Verbindungen der Formel **(Id),** bei denen R^{2b} für Cyano steht, gemäß Verfahren e) (siehe Schema 4) sind neu und daher ebenfalls Gegenstand der Erfindung.

Bestimmte 3-Thio-substituierte Pyridazine der Formel **(Ie)** (mit R^{2c} = C₁-C₄-Alkylthio oder C₁-C₄-Halogenalkylthio) beziehungsweise 3-Oxy-substituierte Pyridazine der Formel **(1f)** (mit R^{2d} = C₁-C₄-Alkoxy, C₃-C₆-Cycloalkyloxy, C₃-C₆-Alkenyloxy, C₃-C₆-Alkinyloxy, C₁-C₄-Halogenalkoxy, Phenoxy oder Benzyloxy) werden erhalten, indem man 3-halogensubstituierte Pyridazine der Formel **(Ia), (Ib)** oder **(Ic),** bei denen R¹ und R³ bis R¹⁰ die oben angegebenen Bedeutungen haben, Z für NR⁴R⁵, S(O)ₙR⁴ oder OR⁸ steht, n gleich 0, 1 oder 2 sein kann und R^{2a} für Fluor, Chlor oder Brom steht, gemäß Verfahren **f)** mit Thioalkoholen der Formel R^{2c}-SH oder gemäß Verfahren **g)** mit Alkoholen R^{2d}-OH gegebenenfalls in Gegenwart von Basen in einem Lösungsmittel umsetzt (siehe Schemata 5a und 5b).

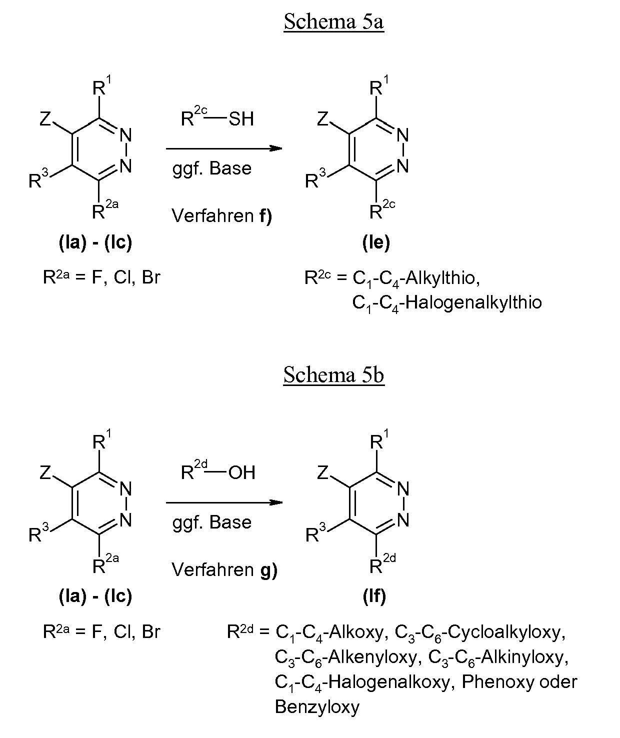

Zu diesem Zweck setzt man Verbindungen der Formel **(Ia), (Ib)** oder **(Ic)** mit entsprechenden Thioalkoholen R^{2c}-SH, beispielsweise Methanthiol oder Ethanthiol, oder Alkoholen R^{2d}-OH, beispielsweise Methanol, Isopropanol oder 2-Methoxyethanol, in Gegenwart von typischen organischen oder anorganischen Basen, beispielsweise Kaliumcarbonat, Kalium-tert-butylat oder Natriumhydrid um. Als Verdünnungsmittel kommen bei der Durchführung der erfindungsgemäßen Verfahren **f)** und **g)** alle für derartige Substitutionsreaktionen üblichen Solventien in Frage. Vorzugsweise verwendbar sind beispielsweise Tetrahydrofuran, 1,4-Dioxan, N,N-Dimethylformamid, N-Methylpyrrolidon oder Dimethylsulfoxid. Gegebenenfalls kann auch der Alkohol R^{2d}-OH oder Thioalkohol R^{2c}-SH selbst im Überschuß eingesetzt und als Lösungsmittel verwendet werden.

Die Temperaturen können bei der Durchführung der Verfahren **f)** und **g)** in einem größeren Bereich variiert werden. Üblicherweise wird die Reaktion in einem Temperaturbereich von 0 °C bis 180 °C durchgeführt; vorzugsweise arbeitet man bei Temperaturen zwischen 20 °C und 150 °C.

Die zur Durchführung der erfindungsgemäßen Verfahren **f)** und **g)** als Ausgangsstoffe benötigten Pyridazine der Formel **(Ia), (Ib)** und **(Ic)** werden bereits oben beschrieben.

Substituierte Pyridazine **(Ia), (Ib)** oder **(Ic),** bei denen R¹ und R³ bis R¹⁰ die oben angegebenen Bedeutungen haben, Z für NR⁴R⁵, S(O)ₙR⁴ oder OR⁸ steht, n gleich 0, 1 oder 2 sein kann und R^{2a} für Fluor, Chlor oder Brom steht, sowie deren weitere Umsetzung in die korrespondierenden Verbindungen der Formel **(Ie),** bei denen R^{2c} für C₁-C₄-Alkylthio oder C₁-C₄-Halogenalkylthio steht, oder **(If),** bei denen R^{2d} für C₁-C₄-Alkoxy, C₃-C₆-Cycloalkyloxy, C₃-C₆-Alkenyloxy, C₃-C₆-Alkinyloxy, C₁-C₄-Halogenalkoxy, Phenoxy oder Benzyloxy steht, gemäß Verfahren **f)** oder **g)** (siehe Schemata 5a und 5b) sind neu und daher ebenfalls Gegenstand der Erfindung.

Bestimmte Pyridazine der Formel (Ig) (mit R^{2e} gleich C₁-C₄-Alkylsulfinyl oder C₁-C₄-Halogenalkylsulfinyl) oder **(Ih)** (mit R^{2f} gleich C₁-C₄-Alkylsulfonyl oder C₁-C₄-Halogenalkylsulfonyl) werden erhalten, indem man Pyridazine der Formel **(Ie),** bei denen R¹ und R³ bis R¹⁰ die oben angegebenen Bedeutungen haben, Z für NR⁴R⁵, SO₂R⁴ oder OR⁸ steht und R^{2c} für C₁-C₄-Alkylthio oder C₁-C₄-Halogenalkylthio steht, gemäß Verfahren h) mit Oxidationsmitteln gegebenenfalls in Gegenwart eines Katalysators, gegebenenfalls in Gegenwart eines Säureakzeptors und gegebenenfalls in Gegenwart eines Lösungsmittels umsetzt (siehe Schema 6). Ausgenommen davon sind Verbindungen der Formel **(Ig)** für die die oben angegebenen Definitionen gültig sind und die zusätzlich zu R^{2c} mindestens einen weiteren schwefelhaltigen oxidierbaren Rest, beispielsweise Thiole, Thioether, Thiophene, Thiophen-S-oxide, Sulfinyl-oder Thionogruppen, enthalten. Das Produktverhälnis **(Ig)** zu **(Ih)** wird durch die Menge des eingesetzten Oxidationsmittels sowie durch geeignete Wahl von Reaktionsdauer und -temperatur gesteuert.

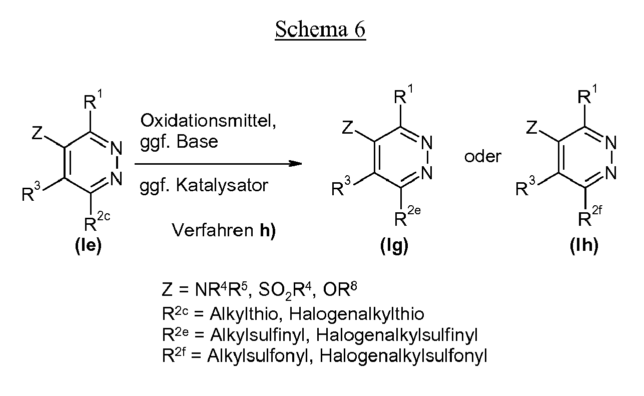

Bestimmte 3-Alkylthio- oder 3-Halogenalkylthio-substituierte Pyridazine der Formel **(Ie),** bei denen R^{2c} für Alkylthio oder Halogenalkylthio steht, lassen sich durch Oxidation, z.B. mit organischen Persäurederivaten, ggf. in Gegenwart eines Katalysators wie z.B. Ammoniummolybdat(VI) (NH₄)₂MoO₄, in geeigneten Lösungsmitteln wie beispielsweise Dichlormethan oder Trichlormethan oder Gemischen davon mit Wasser gemäß Verfahren **h)** in die korrespondierenden 3-Alkylsulfinyl- oder 3-Halogenalkylsulfinylpyridazine der Formel **(Ig)** und / oder 3-Alkylsulfonyl- oder Halogenalkylsulfonylpyridazine der Formel **(Ih)** überführen (siehe Schema 6). In Frage kommende Persäurederivate wie beispielsweise *m*-Chlorperbenzoesäure, Magnesiummonoperoxyphthalat, Peressigsäure oder Trifluorperessigsäure sind entweder kommerziell verfügbar oder werden *in situ* aus den korrespondierenden Carbonsäuren (z.B. Essigsäure oder Trifluoressigsäure) in Gegenwart von Wasserstoffperoxid erzeugt. Darüber hinaus kann die Reaktion gegebenenfalls in Gegenwart eines geeigneten Säureakzeptors wie beispielsweise Natriumcarbonat durchgeführt werden. Die Temperaturen können bei der Durchführung des Verfahrens **h)** in einem größeren Bereich variiert werden. Im Allgemeinen arbeitet man bei Temperaturen zwischen 0 °C und 100 °C, vorzugsweise zwischen 10 °C und 70 °C.

Die zur Durchführung des erfindungsgemässen Verfahrens **h)** als Ausgangsstoffe benötigten Pyridazine der Formel **(Ie)** werden bereits oben beschrieben.

Substituierte Pyridazine **(Ie),** bei denen R¹ und R³ bis R¹⁰ die oben angegebenen Bedeutungen haben und R^{2c} für Alkylthio oder Halogenalkylthio steht, sowie deren Oxidation in die korrespondierenden Verbindungen der Formel **(Ig)**, bei denen R^{2e} für Alkylsulfinyl oder Halogenalkylsulfinyl steht, oder **(Ih),** bei denen R^{2f} für Alkylsulfonyl oder Halogenalkylsulfonyl steht, gemäß Verfahren **h)** (siehe Schema 6) sind neu und daher ebenfalls Gegenstand der Erfindung. Ausgenommen davon sind Verbindungen der Formel **(Ie)** für die die oben angegebenen Definitionen gültig sind und bei denen zusätzlich zu R^{2c} mindestens ein schwefelhaltiger oxidierbarer Rest, beispielsweise Thiole, Thioether, Thiophene, Thiophen-S-oxide, Sulfinyl-oder Thionogruppen, gegenwärtig ist.

Bestimmte 5-thiosubstituierte Pyridazine der Formel **(Ia'),** bei denen R¹ bis R¹⁰ die oben angegebenen Bedeutungen haben und Z für SR⁴ steht, lassen sich analog dem oben beschriebenen Verfahren **i)** durch Oxidation, z.B. mit organischen Persäurederivaten, gegebenenfalls in Gegenwart eines Katalysators, in geeigneten Lösungsmitteln wie beispielsweise Dichlormethan oder Trichlormethan oder Gemischen davon mit Wasser in die korrespondierenden Sulfoxide der Formel **(Ii)** und / oder Sulfone der Formel **(Ij)** überführen (siehe Schema 7). Ausgenommen davon sind Verbindungen der Formel **(Ia')** für die die oben angegebenen Definitionen gültig sind und die zusätzlich zu SR⁴ mindestens einen weiteren schwefelhaltigen oxidierbaren Rest, beispielsweise Thiole, Thioether, Thiophene, Thiophen-S-oxide, Sulfinyl-oder Thionogruppen, enthalten. Das Produktverhälnis **(Ii)** zu **(Ij)** wird durch die Menge des eingesetzten Oxidationsmittels sowie durch geeignete Wahl von Reaktionsdauer und -temperatur gesteuert.

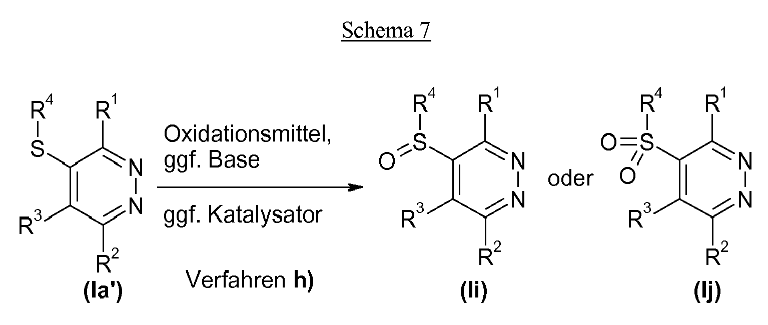

Thio-substituierte Pyridazine **(Ii)** und **(Ij),** bei denen R¹ bis R¹⁰ die oben angegebenen Bedeutungen haben und Z für SR⁴ steht, sind neu und daher ebenfalls Gegenstand der Erfindung.

Die als Ausgangsstoffe benötigten Pyridazine der Formel **(Ia')** sind für den Fall, dass R² einem Halogenatom entspricht, als Verbindungen der Formel **(Ia)** nach Verfahrensschritt b) erhältlich. Alle übrigen Verbindungen lassen sich analog nach den in Verfahrensschritt **e)** (Verbindungen der Formel **(Id),** R^{2b} = CN) oder Verfahrensschritt **f)** (Verbindungen der Formel **(Ie),** R^{2c} = Alkylthio, Halogenalkylthio) oder Verfahrensschritt **g)** (Verbindungen der Formel **(If),** R^{2d} = Alkoxy, Cycloalkyloxy, Alkenyloxy, Alkinyloxy, Halogenalkoxy, Phenoxy oder Benzyloxy) beschriebenen Reaktionsbedingungen aus Verbindungen der Formel **(Ia)** erhalten.

Bestimmte aminosubstituierte Pyridazine **(Ic'),** bei denen R¹ bis R⁴ und R⁶ bis R¹⁰ die oben angegebenen Bedeutungen haben und R⁵ gleich Wasserstoff ist, lassen sich gemäß Verfahren **j)** durch Umsetzung mit Acylierungsmitteln (beispielsweise Acylhalogenide, Carbonsäureanhydride, aktivierte Carbonsäureester oder Chlorkohlensäureester), Sulfonylierungsmitteln (beispielsweise Sulfonylchloriden) oder Silylierungsmitteln (beispielsweise Trialkylsilylchloride oder -triflate) der allgemeinen Formel R⁹-Y in die korrespondierenden N-acylierten, N-sulfonylierten oder N-silylierten Verbindungen der Formel **(Ik)** überführen (siehe Schema 8).

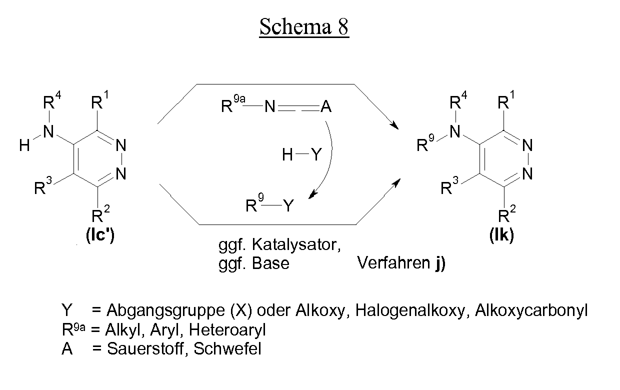

Bei der Durchführung des Verfahrens **j)** können gegebenenfalls Katalysatoren wie beispielsweise 4-(N,N-Dimethylamino)pyridin eingesetzt werden. Darüber hinaus kann die Reaktion gegebenenfalls in Gegenwart eines geeigneten Säureakzeptors wie beispielsweise Natriumcarbonat oder Triethylamin durchgeführt werden.

Als Verdünnungsmittel kommen bei der Durchführung des erfindungsgemäßen Verfahren **j)** alle für derartige Substitutionsreaktionen üblichen Solventien in Frage. Vorzugsweise verwendbar sind beispielsweise 2-Methoxyethanol, 1,2-Dimethoxyethan, 1,4-Dioxan, N,N-Dimethylformamid oder N-Methylpyrrolidon. Gegebenenfalls kann auch das entsprechende Acylierungs-, Sulfonylierungs- oder Silylierungsmittel selbst im Überschuß eingesetzt und als Lösungsmittel verwendet werden.

Die Temperaturen können bei der Durchführung des Verfahrens **j)** in einem größeren Bereich variiert werden. Im Allgemeinen arbeitet man bei Temperaturen zwischen 0°C und 160°C, vorzugsweise zwischen 20°C und 120°C.

Alternativ lassen sich Verbindungen der Formel **(Ik)**, bei denen R⁹ gleich Alkylcarbamoyl, Phenylcarbamoyl oder Heteroarylcarbamoyl ist, durch Addition von geeigneten Isocyanaten oder Isothiocyanaten der allgemeinen Formel R^{9a}-N=C=O bzw. R^{9a}-N=C=S (mit R^{9a} = Alkyl, Phenyl, Heteroaryl) an Verbindungen der Formel **(Ic')** herstellen. Darüber hinaus können Isocyanate und Isothiocyanate der Formel R^{9a}-N=C=O bzw. R^{9a}-N=C=S durch Addition von Nukleophilen H-Y nach bekannten Methoden in die korrespondierenden Verbindungen der Formel R⁹-Y überführt werden, welche ihrerseits gemäß Verfahren **j)** in Verbindungen der Formel **(Ik)** überführt werden können.

Aminosubstituierte Pyridazine **(Ic'),** bei denen R¹ bis R⁴ und R⁶ bis R¹⁰ die oben angegebenen Bedeutungen haben und R⁵ gleich Wasserstoff ist, sowie deren Umsetzung in die korrespondierenden Verbindungen der Formel **(Ik)** gemäß Verfahren **j)** sind neu und daher ebenfalls Gegenstand der Erfindung.

Die zur Durchführung des erfindungsgemässen Verfahrens **j)** als Ausgangsstoffe benötigten Pyridazine der Formel **(Ic'),** sind für den Fall, dass R² einem Halogenatom entspricht, als Verbindungen der Formel **(Ic)** nach Verfahrensschritt **d)** erhältlich. Alle übrigen Verbindungen lassen sich analog nach den in Verfahrensschritt e) (Verbindungen der Formel **(Id),** R^{2b} = CN) oder Verfahrensschritt **f)** (Verbindungen der Formel **(Ie),** R^{2c} = Alkylthio, Halogenalkylthio) oder Verfahrensschritt **g)** (Verbindungen der Formel **(If),** R^{2d} = Alkoxy, Cycloalkyloxy, Alkenyloxy, Alkinyloxy, Halogenalkoxy, Phenoxy oder Benzyloxy) oder Verfahrensschritt **h)** (Verbindungen der Formel **(Ig)**, R^{2e} = Alkylsulfinyl, Halogenalkylsulfinyl beziehungsweise Verbindungen der Formel **(Ih)** R^{2f} = Alkylsulfinyl, Halogenalkylsulfinyl) beschriebenen Reaktionsbedingungen aus Verbindungen der Formel **(Ic)** erhalten.

Bestimmte oxy-substituierte Pyridazine **(Ib'),** bei denen R¹ bis R¹⁰ die oben angegebenen Bedeutungen haben, lassen sich in 5-Hydroxypyridazine der Formel (Im) überführen, welche sich wiederum gemäß Verfahren k) in Verbindungen der Formel (In) überführen lassen (siehe Schema 9).

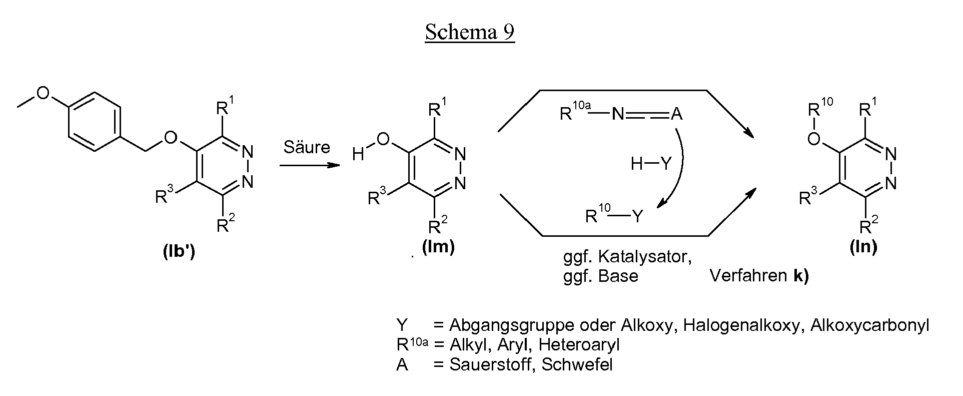

Bestimmte Reste R⁸ (beispielsweise Methyl, tert-Butyl, Allyl oder unsubstituiertes oder substituiertes Benzyl) können als Schutzgruppen für Alkoholfunktionen betrachtet werden und lassen sich unter Freisetzung der Alkoholfunktion selektiv abspalten (siehe dazu z.B. T.W. Greene, P.G.M. Wuts, "Protective Groups in Organic Synthesis ", 3. Auflage, Wiley New York 1999; P.J. Kocienski, "Protecting Groups", 3. Auflage, G. Thieme Verlag, Stuttgart 2005). So lässt sich beispielsweise ein p-Methoxybenzylether (z.B. R⁸ = p-Methoxybenzyl) unter sauren (z.B. Trifluoressigsäure, Salzsäure, Schwefelsäure, Methansulfonsäure, p-Toluolsulfonsäure), hydrogenolytischen (z.B. unter Verwendung von Pd auf einem interten Trägermaterial in einer Wasserstoffatmosphäre) oder oxidativen (z.B. unter Verwendung von DDQ oder Cer(IV)ammoniumnitrat) Bedingungen spalten um Hydroxyverbindungen der Formel **(Im)** zu erhalten.

Anschließend lassen sich die Hydroxyverbindungen der Formel **(Im)** gemäß Verfahren **k)** durch Umsetzung mit Acylierungsmitteln (beispielsweise Acylhalogenide, Carbonsäureanhydride, aktivierte Carbonsäureester oder Chlorkohlensäureester), Sulfonylierungsmitteln (beispielsweise Sulfonylchloride) oder Silylierungsmitteln (beispielsweise Trialkylsilylchloride oder -triflate) in die korrespondierenden O-acylierten, O-sulfonylierten oder O-silylierten Verbindungen der Formel (In), bei denen R¹ bis R¹⁰ die oben angegebenen Bedeutungen haben, überführen (siehe Schema 9).

Bei der Durchführung des Verfahrens **k)** können gegebenenfalls Katalysatoren wie beispielsweise 4-(N,N-Dimethylamino)pyridin eingesetzt werden. Darüber hinaus kann die Reaktion gegebenenfalls in Gegenwart eines geeigneten Säureakzeptors wie beispielsweise Natriumcarbonat oder Triethylamin durchgeführt werden.

Als Verdünnungsmittel kommen bei der Durchführung des erfindungsgemäßen Verfahrens **k)** alle für derartige Substitutionsreaktionen üblichen Solventien in Frage. Vorzugsweise verwendbar sind beispielsweise 2-Methoxyethanol, 1,2-Dimethoxyethan, 1,4-Dioxan, N,N-Dimethylformamid oder N-Methylpyrrolidon. Gegebenenfalls kann auch das Acylierungs-, Sulfonylierungs- oder Silylierungsmittel selbst im Überschuß eingesetzt und als Lösungsmittel verwendet werden.

Die Temperaturen können bei der Durchführung des Verfahrens **k)** in einem größeren Bereich variiert werden. Im Allgemeinen arbeitet man bei Temperaturen zwischen 0°C und 150°C, vorzugsweise zwischen 10°C und 100°C.

Alternativ lassen sich Verbindungen der Formel **(In)**, bei denen R¹⁰ gleich Alkylcarbamoyl, Phenylcarbamoyl oder Heteroarylcarbamoyl ist, durch Addition von geeigneten Isocyanaten oder Isothiocyanaten der allgemeinen Formel R^{10a}-N=C=O bzw. R^{10a}-N=C=S (mit R^{10a} = Alkyl, Phenyl, Heteroaryl) an Verbindungen der Formel **(Im)** herstellen. Darüber hinaus können Isocyanate und Isothiocyanate der Formel R^{10a}-N=C=O bzw. R^{10a}-N=C=S durch Addition von Nukleophilen H-Y nach bekannten Methoden in die korrespondierenden Verbindungen der Formel R¹⁰-Y überführt werden, welche ihrerseits gemäß Verfahren k) in Verbindungen der Formel **(In)** überführt werden können.

Oxy-substituierte Pyridazine **(Ib'),** bei denen R¹ bis R¹⁰ die oben angegebenen Bedeutungen haben, sowie deren Umsetzung in die Hydroxyverbindung **(Im)** und deren weitere Umsetzung in die korrespondierenden Verbindungen der Formel **(In)** gemäß Verfahren **k)** (siehe Schema 9) sind neu und daher ebenfalls Gegenstand der Erfindung.

Die zur Durchführung des erfindungsgemässen Verfahrens **k)** als Ausgangsstoffe benötigten Pyridazine der Formel **(Ib')** sind für den Fall, dass R² einem Halogenatom entspricht, als Verbindungen der Formel **(Ib)** nach Verfahrensschritt **c)** erhältlich. Alle übrigen Verbindungen lassen sich analog nach den in Verfahrensschritt e) (Verbindungen der Formel **(Id),** R^{2b} = CN) oder Verfahrensschritt **f)** (Verbindungen der Formel **(Ie),** R^{2c} = Alkylthio, Halogenalkylthio) oder Verfahrensschritt **g)** (Verbindungen der Formel **(If),** R^{2d} = Alkoxy, Cycloalkyloxy, Alkenyloxy, Alkinyloxy, Halogenalkoxy, Phenoxy oder Benzyloxy) oder Verfahrensschritt **h)** (Verbindungen der Formel **(Ig)**, R^{2e} = Alkylsulfinyl, Halogenalkylsulfinyl beziehungsweise Verbindungen der Formel **(Ih)** R^{2f} = Alkylsulfinyl, Halogenalkylsulfinyl) beschriebenen Reaktionsbedingungen aus Verbindungen der Formel **(Ib)** erhalten.

Die erfindungsgemäßen Verfahren zur Herstellung der Verbindungen der Formeln **(Ia)** bis (In) werden vorzugsweise unter Verwendung eines oder mehrerer Reaktionshilfsmittel durchgeführt.

Als Reaktionshilfsmittel kommen gegebenenfalls die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium- - Kalium- oder Calcium-hydroxid, Natrium- oder Kalium- -methanolat, -ethanolat, -n- oder -i-propanolat, -n-, -i-, -s- oder -tButanolat; weiterhin auch basische organische Stickstoffverbindungen, wie beispielsweise Trimethylamin, Triethylamin, Tripropylamin, Tributylamin, Ethyldiisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl-und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Als weitere Reaktionshilfsmittel kommen gegebenenfalls die üblichen anorganischen oder organischen Brönstedt- bzw. Lewis-Säuren in Betracht. Hierzu gehören vorzugsweise Halogenwasserstoffsäuren oder Mineralsäuren wie beispielsweise Chlor- oder Bromwasserstoffsäure, Schwefelsäure, schweflige Säure, Salpetersäure, salpetrige Säure, Phosphorsäure, Lewis-Säuren wie beispielsweise Aluminiumtrichlorid, Bortrifluorid, Bortrichlorid, Bortribromid, Titantetrachlorid, Zinntetrachlorid, Certrichlorid, (Übergangs-) Metalltriflate wie beispielsweise Trialkylsilyltriflate, Kupfertriflat, Ytterbiumtriflat, Imide wie beispielsweise Trifluormethansulfonimid, Sulfonsäuren wie beispielsweise Methansulfonsäure, Trifluormethansulfonsäure, p-Toluolsulfonsäure, Camphersulfonsäure, Carbonsäuren wie beispielsweise Ameisensäure, Essigsäure, Trifluoressigsäure, Propionsäure, Oxalsäure, Benzoesäure sowie an feste Phasen gebundene Säuren wie beispielsweise saure Ionentauscher.

Die erfindungsgemäßen Verfahren werden vorzugsweise unter Verwendung eines oder mehrerer Verdünnungsmittel durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methylethyl-, Methyl-isopropyl-oder Methyl-isobutyl-keton, Ester wie Essigsäuremethylester oder - ethylester, Nitrile wie z.B. Acetonitril oder Propionitril, Amide wie z.B. Dimethylformamid, Dimethylacetamid und N-Methyl-pyrrolidon, sowie Dimethylsulfoxid, Tetramethylensulfon und Hexamethylphosphorsäuretriamid und DMPU.

Die Reaktionstemperaturen können bei den erfindungsgemäßen Verfahren in einem größeren Bereich variiert werden. Im allgemeinen arbeitet man bei Temperaturen zwischen 0°C und 250°C, vorzugsweise bei Temperaturen zwischen 10°C und 185°C.

Die erfindungsgemäßen Verfahren werden im allgemeinen unter Normaldruck durchgeführt. Es ist jedoch auch möglich, unter erhöhtem oder vermindertem Druck zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Die erfindungsgemäßen Pyridazine der Formel **(I)** besitzen sehr gute fungizide Eigenschaften und lassen sich im Pflanzenschutz beispielsweise zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz beispielsweise zur Bekämpfung von Pseudomonadaceae, Rhizobiaceae, Enterobacteriaceae, Corynebacteriaceae und Streptomycetaceae einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen. Bevorzugt werden Getreidepflanzen erfindungsgemäß behandelt.

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus;
Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Altemaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum;
   Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Taphrina-Arten, wie beispielsweise Taphrina deformans;
Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;
Bevorzugt können die folgenden Krankheiten von Soja-Bohnen bekämpft werden:
   Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).
   Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine sehr gute stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze und Bakterien zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die erfindungsgemäßen Wirkstoffe mit besonders gutem Erfolg zur Bekämpfung von Getreidekrankheiten, wie beispielsweise gegen Erysiphe-Arten, gegen Puccinia und gegen Fusarien-Arten, von Reiskrankheiten, wie beispielsweise gegen Pyricularia und Rhizoctonia und von Krankheiten im Wein-, Obst-und Gemüseanbau, wie beispielsweise gegen Botrytis-, Venturia-, Sphaerotheca-und Podosphaera-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Die erfindungsgemäßen Wirkstoffe bzw. Mittel können außerdem im Materialschutz zum Schutz von technischen Materialien gegen Befall und Zerstörung durch unerwünschten Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern.

Das erfindungsgemäße Verfahren zum Bekämpfen von unerwünschten Pilzen kann auch zum Schutz von so genannte Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Altemaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Die vorliegende Erfindung betrifft weiterhin ein Mittel zum Bekämpfen unerwünschter Mikroorganismen, umfassend wenigstens eines der erfindungsgemäßen Pyridazine. Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-FettsäureEster, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP-POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder

Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Die Wirkstoffe können als solche, in Form ihrer Formulierungen oder den daraus bereiteten Anwendungsformen, wie gebrauchsfertige Lösungen, Emulsionen, wasser- oder ölbasierte Suspensionen, Pulver, Spritzpulver, Pasten, lösliche Pulver, Stäubemittel, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die zuvor beschriebenen Formulierungen können in einem erfindungsgemäßen Verfahren zum Bekämpfen unerwünschter Mikroorganismen verwendet werden, bei dem die erfindungsgemäßen Pyridazine auf die Mikroorganismen und/oder in deren Lebensraum ausgebracht werden.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Als Mischpartner kommen zum Beispiel bekannte Fungizide, Insektizide, Akarizide, Nematizide oder auch Bakterizide (siehe auch Pesticide Manual, 13th ed.) infrage.

Auch eine Mischung mit anderen bekannten Wirkstoffen, wie Herbiziden, oder mit Düngemitteln und Wachstumsregulatoren, Safenern bzw. Semiochemicals ist möglich.

Die Erfindung umfasst weiterhin ein Verfahren zur Behandlung von Saatgut.

Ein weiterer Aspekt der vorliegenden Erfindung betrifft insbesondere ein Saatgut, welches mit wenigstens einem der erfindungsgemäßen Pyridazine behandelt ist. Die erfindungsgemäßen Saatgüter finden Anwendung in Verfahren zum Schutz von Saatgut vor unerwünschten Mikroorganismen. Bei diesen wird ein mit wenigstens einem erfindungsgemäßen Wirkstoff behandeltes Saatgut. verwendet.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von phytopathogenen Pilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von phytopathogenen Pilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Wirkstoffe können daher sowohl in medizinischen als auch in nicht-medizinischen Anwendungen eingesetzt werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz beson- ders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Das erfindungsgemäße Verfahren zum Bekämpfen von phytopathogenen Pilzen kann auch zur Behandlung von genetisch veränderten Organismen, z.B. Pflanzen oder Samen, verwendet werden. Genetisch veränderte Pflanzen sind solche, in deren Genom ein bestimmtes heterologes Gen, welches für ein bestimmtes Protein codiert, stabil integriert wurde. "Heterologes Gen" meint dabei ein Gen, welches der transformierten Pflanze neue agronomische Eigenschaften verleiht, oder ein Gen, welches die agronomische Qualität der modifizierten Pflanze verbessert.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio-und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser-bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser-bzw. Bodensalzgehalt, erhöhte Blühleistung, erleich*terte* Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready@ (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Weiterhin eignen sich die erfindungsgemäßen Verbindungen der Formel (I) zur Unterdrückung des Wachstums von Tumorzellen in Menschen und Säugetieren. Dies basiert auf einer Wechselwirkung der erfindungsgemäßen Verbindungen mit Tubulin und Mikrotubuli und durch Förderung der Mikrotubuli-Polymerisation.

Zu diesem Zweck kann man eine wirksame Menge an einer oder mehreren Verbindungen der Formel (I) oder pharmazeutisch verträglicher Salze davon verabreichen.

Die Herstellung (siehe Schema 10) und die Verwendung der erfindungsgemäßen Wirkstoffe geht aus den folgenden Beispielen hervor. Die Erfindung ist jedoch nicht auf diese Beispiele beschränkt.

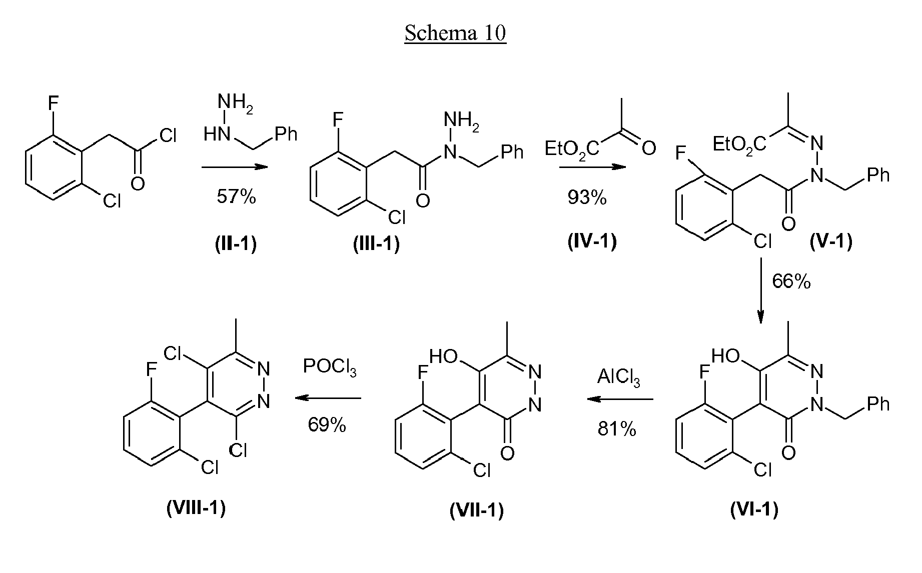

### Herstellung von Ausgangsstoffen der Formel (III):

### N-Benzyl-2-(2-chlor-6-fluorphenyl)acetohydrazid

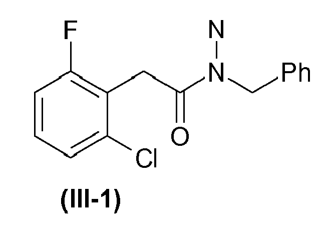

Unter Argon werden 32.45 g (266 mmol) Benzylhydrazin, 44.4 mL (319 mmol) Triethylamin und 1.62 g (13.3 mmol) 4-(N,N-Dimethylamino)pyridin in 500 mL Methylenchlorid vorgelegt und auf 0°C gekühlt. Anschließend wird eine Lösung von 55 g (266 mmol) 2-Chlor-6-fluorphenylacetylchlorid langsam zugetropft und die Mischung 16 Stunden gerührt; dabei wird die Reaktion auf Raumtemperatur gebracht. Zur Aufarbeitung wird die Reaktionsmischung mit gesättigter Ammoniumchloridlösung versetzt. Die organische Phase wird abgetrennt, über Natriumsulfat getrocknet und am Rotationsverdampfer konzentriert. Nach Chromatographie an Kieselgel wurden 49.3 g (51% Ausbeute, 80%ig; logP = 2.69) N-Benzyl-2-(2-chlor-6-fluorphenyl)acetohydrazid (III-1) als farbloses Öl erhalten. Das Material wurde ohne weitere Reinigung in der Folgestufe eingesetzt.

### Herstellung von Ausgangsstoffen der Formel (V):

### Ethyl (2Z)-2-{benzyl[(2-chlor-6-fluorphenyl)acetyl] hydrazono}propanoat

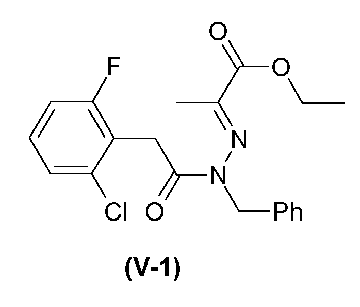

49.3 g (135 mmol) 80%iges N-Benzyl-2-(2-chlor-6-fluorphenyl)acetohydrazid und 15.6 g (135 mmol) Ethylpyruvat werden in 500 mL Ethanol gelöst und 4 Stunden unter Rückfluss gerührt. Das Reaktionsgemisch wird am Rotationsverdampfer konzentriert und an Kieselgel chromatographiert. Es wurden 50 g (93% Ausbeute; logP = 3.87) Ethyl (2Z)-2-{benzyl[(2-chlor-6-fluorphenyl)acetyl] hydrazono}propanoat (V-1) als gelbliches Öl erhalten.

### Herstellung von Ausgangsstoffen der Formel (VI):

### 2-Benzyl-4-(2-chlor-6-fluorphenyl)-5-hydroxy-6-methylpyridazin-3(2H)-on

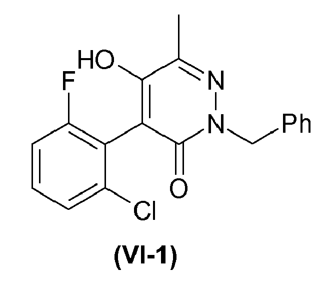

28.7 g (256 mmol) Kalium-tert.-butylat werden in 250 mL DMF vorgelegt, auf 0°C gekühlt und tropfenweise mit einer Lösung aus 50 g (128 mmol) Ethyl (2Z)-2-{benzyl[(2-chlor-6-fluorphenyl)acetyl] hydrazono}propanoat in 500 mL DMF versetzt. Die Reaktionsmischung wird 30 min. bei 0°C gerührt und anschliessend in eiskalte 1N HCl eingerührt. Das Gemisch wird mehrfach mit Ethylessigester extrahiert, getrocknet und am Rotationsverdampfer konzentriert. Nach Chromatographie an Kieselgel wurden 34 g (68% Ausbeute, 88%ig, logP = 2.58) 2-Benzyl-4-(2-chlor-6-fluorphenyl)-5-hydroxy-6-methylpyridazin-3(2H)-on (VI-1) als farbloser Feststoff erhalten.

### Herstellung von Ausgangsstoffen der Formel (VII):

### 4-(2-Chlor-6-fluorphenyl)-5-hydroxy-6-methylpyridazin-3(2H)-on

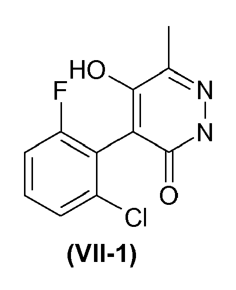

Unter Argon werden 11.3 g (29 mmol) 88%iges 2-Benzyl-4-(2-chlor-6-fluorphenyl)-5-hydroxy-6-methylpyridazin-3(2H)-on in 100 mL Toluol vorgelegt, mit 11.56 g (86.7 mmol) wasserfreiem Aluminiumtrichlorid versetzt und 30 min. bei 50°C gerührt. Die Reaktionsmischung wird abgekühlt, das Lösungsmittel abdekantiert, der feste Rückstand vorsichtig mit Eiswasser versetzt und das Gemisch unter Einsatz des Ultraschallbades hydrolysiert. Der dabei anfallende Feststoff wird abgesaugt, mehrfach mit Wasser und Diethylether gewaschen und getrocknet. Es wurden 6.3 g (81% Ausbeute, 95%ig; logP = 0.88) 4-(2-Chlor-6-fluorphenyl)-5-hydroxy-6-methylpyridazin-3(2*H*)-on (VII-1) als farbloser Feststoff erhalten.

### Herstellung von Ausgangsstoffen der Formel (VIII):

### 3,5-Dichlor-4-(2-chlor-6-fluorphenyl)-6-methylpyridazin

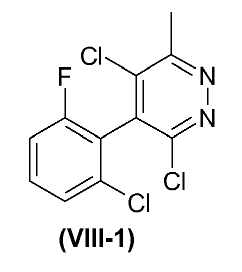

19 g (74.6 mmol) 4-(2-Chlor-6-fluorphenyl)-5-hydroxy-6-methylpyridazin-3(2*H*)-on werden in 104 mL (1.12 mol) Phosphorylchlorid suspendiert und eine Stunde bei 110°C gerührt. Das Reaktionsgemisch wird am Rotationsverdampfer konzentriert, und nach Chromatographie an Kieselgel werden 15 g (69% Ausbeute, logP = 3.24) 3,5-Dichlor-4-(2-chlor-6-fluorphenyl)-6-methylpyridazin (VIII-1) als farbloser Feststoff erhalten.

Analog können beispielsweise folgende Verbindungen der Formel **(VIII)** hergestellt werden:
3,5-Dichlor-4-(2,6-difluorphenyl)-6-methylpyridazin (VIII-2); logP = 2.93

### Herstellungsbeispiele:

### 3-Chlor-4-(2-chlor-6-fluorphenyl)-6-methyl-5-(4-methylpiperidin-1-yl)pyridazin (Verbindung 1-93)

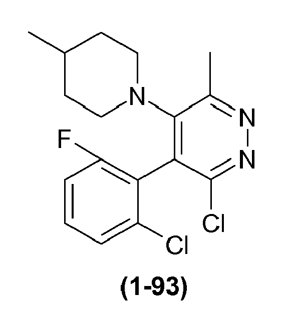

0.5 g (1.71 mmol) 3,5-Dichlor-4-(2-chlor-6-fluorphenyl)-6-methylpyridazin und 0.85 g (8.6 mmol,) 4-Methylpiperidin werden in 6 mL DMF gelöst und 16 h bei 100°C gerührt. Das Reaktionsgemisch wird abgekühlt, mit Wasser versetzt und mehrfach mit Ethylacetat extrahiert. Nach Trocknen der vereinigten organischen Phase über Natriumsulfat wird die Lösung konzentriert, und nach Chromatographie an Kieselgel wurden 0.3 g (47% Ausbeute, 95%ig; logP = 4.11) 3-Chlor-4-(2-chlor-6-fluorphenyl)-6-methyl-5-(4-methylpiperidin-1-yl)pyridazin (1-93) als weißer Feststoff erhalten.

Analog können beispielsweise folgende Verbindungen der Formel **(Ic)** hergestellt werden (siehe Tabelle 1):

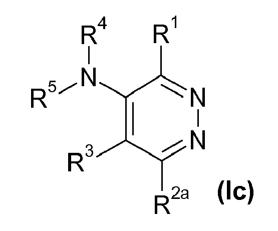

**Tabelle 1**

| **Nr.** | **R¹** | **R^{2a}** | **R³** | **NR⁴R⁵** | **logP**^{a)} |
|---|---|---|---|---|---|
| 1-1 | CH₃ | Cl | 2,3,6-Trifluorphenyl | (1,2-Dimethylpropyl)amino | |
| 1-2 | CH₃ | Cl | 2,3,6-Trifluorphenyl | Sec-Butylamino | |
| 1-3 | CH₃ | Cl | 2,3,6-Trifluorphenyl | Isobutylamino | |
| 1-4 | CH₃ | Cl | 2,3,6-Trifluorphenyl | (2,2-Dimethylpropyl)amino | |
| 1-5 | CH₃ | Cl | 2,3,6-Trifluorphenyl | Cyclopropylamino | |
| 1-6 | CH₃ | Cl | 2,3,6-Trifluorphenyl | (Cyclopropylmethyl)amino | |
| 1-7 | CH₃ | Cl | 2,3,6-Trifluorphenyl | (2-Methoxy-1-methylethyl)amino | |
| 1-8 | CH₃ | Cl | 2,3,6-Trifluorphenyl | (1-Cyclopropylethyl)amino | |
| 1-9 | CH₃ | Cl | 2,3,6-Trifluorphenyl | [(Trimethylsilyl)methyl]amino | |
| 1-10 | CH₃ | Cl | 2,3,6-Trifluorphenyl | Cyclopentylamino | |
| 1-11 | CH₃ | Cl | 2,3,6-Trifluorphenyl | Piperidin-1-yl | |
| 1-12 | CH₃ | Cl | 2,3,6-Trifluorphenyl | 4-Methylpiperidin-1-yl | |
| 1-13 | CH₃ | Cl | 2,3,6-Trifluorphenyl | Morpholin-4-yl | |
| 1-14 | CH₃ | Cl | 2,3,6-Trifluorphenyl | 4,4-dimethyl-1,4-azasilinan-1-yl | |
| 1-15 | CH₃ | Cl | 2,3,6-Trifluorphenyl | 6-Methyl-3-azabicyclo[4.1.0]hept-3-yl | |
| 1-16 | CH₃ | Cl | 2,4,6-Trichlorphenyl | (1,2-Dimethylpropyl)amino | |
| 1-17 | CH₃ | Cl | 2,4,6-Trichlorphenyl | sec-Butylamino | |
| 1-18 | CH₃ | Cl | 2,4,6-Trichlorphenyl | Isobutylamino | |
| 1-19 | CH₃ | Cl | 2,4,6-Trichlorphenyl | (2,2-Dimethylpropyl)amino | |
| 1-20 | CH₃ | Cl | 2,4,6-Trichlorphenyl | Cyclopropylamino | |
| 1-21 | CH₃ | Cl | 2,4,6-Trichlorphenyl | (Cyclopropylmethyl)amino | |
| 1-22 | CH₃ | Cl | 2,4,6-Trichlorphenyl | (2-Methoxy-1-methylethyl)amino | |
| 1-23 | CH₃ | Cl | 2,4,6-Trichlorphenyl | (1-Cyclopropylethyl)amino | |
| 1-24 | CH₃ | Cl | 2,4,6-Trichlorphenyl | [(Trimethylsilyl)methyl]amino | |
| 1-25 | CH₃ | Cl | 2,4,6-Trichlorphenyl | Cyclopentylamino | |
| 1-26 | CH₃ | Cl | 2,4,6-Trichlorphenyl | Piperidin-1-yl | |
| 1-27 | CH₃ | Cl | 2,4,6-Trichlorphenyl | 4-Methylpiperidin-1-yl | |
| 1-28 | CH₃ | Cl | 2,4,6-Trichlorphenyl | Morpholin-4-yl | |
| 1-29 | CH₃ | Cl | 2,4,6-Trichlorphenyl | 4,4-dimethyl-1,4-azasilinan-1-yl | |
| 1-30 | CH₃ | Cl | 2,4,6-Trichlorphenyl | 6-Methyl-3-azabicyclo[4.1.0]hept-3-yl | |
| 1-31 | CH₃ | Cl | 2,4,6-Trifluorphenyl | (1,2-Dimethylpropyl)amino | |
| 1-32 | CH₃ | Cl | 2,4,6-Trifluorphenyl | sec-Butylamino | |
| 1-33 | CH₃ | Cl | 2,4,6-Trifluorphenyl | Isobutylamino | |
| 1-34 | CH₃ | Cl | 2,4,6-Trifluorphenyl | (2,2-Dimethylpropyl)amino | |
| 1-35 | CH₃ | Cl | 2,4,6-Trifluorphenyl | Cyclopropylamino | |
| 1-36 | CH₃ | Cl | 2,4,6-Trifluorphenyl | (Cyclopropylmethyl)amino | |
| 1-37 | CH₃ | Cl | 2,4,6-Trifluorphenyl | (2-Methoxy-1-methylethyl)amino | |
| 1-38 | CH₃ | Cl | 2,4,6-Trifluorphenyl | (1-Cyclopropylethyl)amino | |
| 1-39 | CH₃ | Cl | 2,4,6-Trifluorphenyl | [(Trimethylsilyl)methyl]amino | |
| 1-40 | CH₃ | Cl | 2,4,6-Trifluorphenyl | Cyclopentylamino | |
| 1-41 | CH₃ | Cl | 2,4,6-Trifluorphenyl | Piperidin-1-yl | |
| 1-42 | CH₃ | Cl | 2,4,6-Trifluorphenyl | 4-Methylpiperidin-1-yl | |
| 1-43 | CH₃ | Cl | 2,4,6-Trifluorphenyl | Morpholin-4-yl | |
| 1-44 | CH₃ | Cl | 2,4,6-Trifluorphenyl | 4,4-dimethyl-1,4-azasilinan-1-yl | |
| 1-45 | CH₃ | Cl | 2,4,6-Trifluorphenyl | 6-Methyl-3-azabicyclo[4.1.0]hept-3-yl | |
| 1-46 | CH₃ | Cl | 2,6-Difluorphenyl | R-(1,2-Dimethylpropyl)amino | 3,04 |
| 1-47 | CH₃ | Cl | 2,6-Difluorphenyl | sec-Butylamino | |
| 1-48 | CH₃ | Cl | 2,6-Difluorphenyl | Isobutylamino | 2,64 |
| 1-49 | CH₃ | Cl | 2,6-Difluorphenyl | (2,2-Dimethylpropyl)amino | 3,06 |
| 1-50 | CH₃ | Cl | 2,6-Difluorphenyl | Cyclopropylamino | |
| 1-51 | CH₃ | Cl | 2,6-Difluorphenyl | Isopropylamino | 2,36 |
| 1-52 | CH₃ | Cl | 2,6-Difluorphenyl | (Cyclopropylmethyl)amino | 2,46 |
| 1-53 | CH₃ | Cl | 2,6-Difluorphenyl | (2-Methoxy-1-methylethyl)amino | 2,25 |
| 1-54 | CH₃ | Cl | 2,6-Difluorphenyl | (1-Cyclopropylethyl)amino | |
| 1-55 | CH₃ | Cl | 2,6-Difluorphenyl | [(Trimethylsilyl)methyl]amino | |
| 1-56 | CH₃ | Cl | 2,6-Difluorphenyl | Cyclopentylamino | |
| 1-57 | CH₃ | Cl | 2,6-Difluorphenyl | Piperidin-1-yl | |
| 1-58 | CH₃ | Cl | 2,6-Difluorphenyl | 4-Methylpiperidin-1-yl | 3,87 |
| 1-59 | CH₃ | Cl | 2,6-Difluorphenyl | Morpholin-4-yl | 2,19 |
| 1-60 | CH₃ | Cl | 2,6-Difluorphenyl | 4,4-dimethyl-1,4-azasilinan-1-yl | |
| 1-61 | CH₃ | Cl | 2,6-Difluorphenyl | 6-Methyl-3-azabicyclo[4.1.0]hept-3-yl | |
| 1-62 | CH₃ | Cl | 2,6-Difluorphenyl | rac-(1,2-Dimethylpropyl)amino | 2,99 |
| 1-63 | CH₃ | Cl | 2,6-Difluorphenyl | Benzylamino | 2,67 |
| 1-64 | CH₃ | Cl | 2,6-Difluorphenyl | 2-Methylpyrrolidin-1-yl | 3,08 |
| 1-65 | CH₃ | Cl | 2,6-Difluorphenyl | Pyrrolidin-1-yl | 2,57 |
| 1-66 | CH₃ | Cl | 2,6-Difluorphenyl | N-Methyl-N-(2-methylpropyl)amino | 3,65 |
| 1-67 | CH₃ | Cl | 2-Chlor-4,6-difluorphenyl | (1,2-Dimethylpropyl)amino | |
| 1-68 | CH₃ | Cl | 2-Chlor-4,6-difluorphenyl | sec-Butylamino | |
| 1-69 | CH₃ | Cl | 2-Chlor-4,6-difluorphenyl | Isobutylamino | |
| 1-70 | CH₃ | Cl | 2-Chlor-4,6-difluorphenyl | (2,2-Dimethylpropyl)amino | |
| 1-71 | CH₃ | Cl | 2-Chlor-4,6-difluorphenyl | Cyclopropylamino | |
| 1-72 | CH₃ | Cl | 2-Chlor-4,6-difluorphenyl | (Cyclopropylmethyl)amino | |
| 1-73 | CH₃ | Cl | 2-Chlor-4,6-difluorphenyl | (2-Methoxy-1-methylethyl)amino | |
| 1-74 | CH₃ | Cl | 2-Chlor-4,6-difluorphenyl | (1-Cyclopropylethyl)amino | |
| 1-75 | CH₃ | Cl | 2-Chlor-4,6-difluorphenyl | [(Trimethylsilyl)methyl]amino | |
| 1-76 | CH₃ | Cl | 2-Chlor-4,6-difluorphenyl | Cyclopentylamino | |
| 1-77 | CH₃ | Cl | 2-Chlor-4,6-difluorphenyl | Piperidin-1-yl | |
| 1-78 | CH₃ | Cl | 2-Chlor-4,6-difluorphenyl | 4-Methylpiperidin-1-yl | |
| 1-79 | CH₃ | Cl | 2-Chlor-4,6-difluorphenyl | Morpholin-4-yl | |
| 1-80 | CH₃ | Cl | 2-Chlor-4,6-difluorphenyl | 4,4-dimethyl-1,4-azasilinan-1-yl | |
| 1-81 | CH₃ | Cl | 2-Chlor-4,6-difluorphenyl | 6-Methyl-3-azabicyclo[4.1.0]hept-3-yl | |
| 1-82 | CH₃ | Cl | 2-Chlor-6-fluorphenyl | rac-(1,2-Dimethylpropyl)amino | 3,21 |
| 1-83 | CH₃ | Cl | 2-Chlor-6-fluorphenyl | sec-Butylamino | |
| 1-84 | CH₃ | Cl | 2-Chlor-6-fluorphenyl | Isobutylamino | |
| 1-85 | CH₃ | Cl | 2-Chlor-6-fluorphenyl | (2,2-Dimethylpropyl)amino | |
| 1-86 | CH₃ | Cl | 2-Chlor-6-fluorphenyl | Cyclopropylamino | 2,35 |
| 1-87 | CH₃ | Cl | 2-Chlor-6-fluorphenyl | (Cyclopropylmethyl)amino | |
| 1-88 | CH₃ | Cl | 2-Chlor-6-fluorphenyl | (2-Methoxy-1-methylethyl)amino | |
| 1-89 | CH₃ | Cl | 2-Chlor-6-fluorphenyl | (1-Cyclopropylethyl)amino | |
| 1-90 | CH₃ | Cl | 2-Chlor-6-fluorphenyl | [(Trimethylsilyl)methyl]amino | |
| 1-91 | CH₃ | Cl | 2-Chlor-6-fluorphenyl | Cyclopentylamino | |
| 1-92 | CH₃ | Cl | 2-Chlor-6-fluorphenyl | Piperidin-1-yl | |
| 1-93 | CH₃ | Cl | 2-Chlor-6-fluorphenyl | 4-Methylpiperidin-1-yl | 4,11 |
| 1-94 | CH₃ | Cl | 2-Chlor-6-fluorphenyl | Morpholin-4-yl | 2,39 |
| 1-95 | CH₃ | Cl | 2-Chlor-6-fluorphenyl | 4,4-dimethyl-1,4-azasilinan-1-yl | 4,75 |
| 1-96 | CH₃ | Cl | 2-Chlor-6-fluorphenyl | 6-Methyl-3-azabicyclo[4.1.0]hept-3-yl | 4,25 |
| 1-97 | CH₃ | Cl | 2-Chlor-6-fluorphenyl | 2-Methylpyrrolidin-1-yl | 3,32 |
| 1-98 | CH₃ | Cl | 2-Chlor-6-fluorphenyl | Pyrrolidin-1-yl | 2,82 |
| 1-99 | CH₃ | Cl | 2-Chlor-6-fluorphenyl | 4-Chlorphenylamino | 3,18 |
| 1-100 | CH₃ | Cl | 2-Chlor-6-fluorphenyl | R-(1,2-Dimethylpropyl)amino | 3,22 |
| 1-101 | CH₃ | Cl | 4-Chlor-2,6-difluorphenyl | (1,2-Dimethylpropyl)amino | |
| 1-102 | CH₃ | Cl | 4-Chlor-2,6-difluorphenyl | sec-Butylamino | |
| 1-103 | CH₃ | Cl | 4-Chlor-2,6-difluorphenyl | Isobutylamino | |
| 1-104 | CH₃ | Cl | 4-Chlor-2,6-difluorphenyl | (2,2-Dimethylpropyl)amino | |
| 1-105 | CH₃ | Cl | 4-Chlor-2,6-difluorphenyl | Cyclopropylamino | |
| 1-106 | CH₃ | Cl | 4-Chlor-2,6-difluorphenyl | (Cyclopropylmethyl)amino | |
| 1-107 | CH₃ | Cl | 4-Chlor-2,6-difluorphenyl | (2-Methoxy-1-methylethyl)amino | |
| 1-108 | CH₃ | Cl | 4-Chlor-2,6-difluorphenyl | (1-Cyclopropylethyl)amino | |
| 1-109 | CH₃ | Cl | 4-Chlor-2,6-difluorphenyl | [(Trimethylsilyl)methyl]amino | |
| 1-110 | CH₃ | Cl | 4-Chlor-2,6-difluorphenyl | Cyclopentylamino | |
| 1-111 | CH₃ | Cl | 4-Chlor-2,6-difluorphenyl | Piperidin-1-yl | |
| 1-112 | CH₃ | Cl | 4-Chlor-2,6-difluorphenyl | 4-Methylpiperidin-1-yl | |
| 1-113 | CH₃ | Cl | 4-Chlor-2,6-difluorphenyl | Morpholin-4-yl | |
| 1-114 | CH₃ | Cl | 4-Chlor-2,6-difluorphenyl | 4,4-dimethyl-1,4-azasilinan-1-yl | |
| 1-115 | CH₃ | Cl | 4-Chlor-2,6-difluorphenyl | 6-Methyl-3-azabicyclo[4.1.0]hept-3-yl | |

### 3-Chlor-4-(2-chlor-6-fluorphenyl)-5-(4-chlorphenoxy)-6-methylpyridazin (Verbindung 2-60)

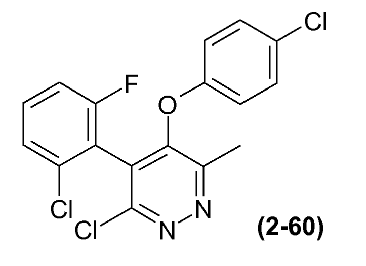

0.035 g (0.87 mmol) Natriumhydrid (als 60%ige Dispersion in Mineralöl) werden in 3 mL DMF vorgelegt und bei Raumtemperatur mit 0.1g (0.79 mmol) 4-Chlorphenol versetzt und 30 min. bei Raumtemperatur gerührt. Anschliessend werden 0.21 g (0.72 mmol) 3,5-Dichlor-4-(2-chlor-6-fluorphenyl)-6-methylpyridazin zugegeben und das Gemisch drei Stunden bei 60°C gerührt. Die Lösung wird abgekühlt, in gesättigte Ammoniumchloridlösung eingerührt und mehrfach mit Ethylacetat extrahiert. Danach wird die organische Phase über Natriumsulfat getrocknet und am Rotationsverdampfer konzentriert. Nach Chromatographie an Kieselgel wurden 0.22 g (73% Ausbeute, 95% Reinheit; logP = 4.13) 3-Chlor-4-(2-chlor-6-fluorphenyl)-5-(4-chlorphenoxy)-6-methylpyridazin als weißer Feststoff erhalten.

Analog können beispielsweise folgende Verbindungen der Formel **(Ib)** hergestellt werden (siehe Tabelle 2):

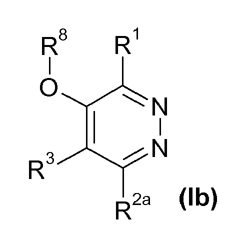

**Tabelle 2**

| **Nr.** | **R¹** | **R^{2a}** | **R³** | **OR⁸** | **logP^{a)}** |
|---|---|---|---|---|---|
| 2-1 | CH₃ | Cl | 2,3,6-Trifluorphenyl | 1,2-Dimethylpropoxy | |
| 2-2 | CH₃ | Cl | 2,3,6-Trifluorphenyl | Isobutoxy | |
| 2-3 | CH₃ | Cl | 2,3,6-Trifluorphenyl | 2,2-Dimethylpropoxy | |
| 2-4 | CH₃ | Cl | 2,3,6-Trifluorphenyl | sec-Butoxy | |
| 2-5 | CH₃ | Cl | 2,3,6-Trifluorphenyl | Allyloxy | |
| 2-6 | CH₃ | Cl | 2,3,6-Trifluorphenyl | Cyclohexyloxy | |
| 2-7 | CH₃ | Cl | 2,3,6-Trifluorphenyl | Phenoxy | |
| 2-8 | CH₃ | Cl | 2,3,6-Trifluorphenyl | 4-Chlorphenoxy | |
| 2-9 | CH₃ | Cl | 2,3,6-Trifluorphenyl | (4-Methoxybenzyl)oxy | |
| 2-10 | CH₃ | Cl | 2,3,6-Trifluorphenyl | Cyclopropylmethoxy | |
| 2-11 | CH₃ | Cl | 2,4,6-Trichlorphenyl | 1,2-Dimethylpropoxy | |
| 2-12 | CH₃ | Cl | 2,4,6-Trichlorphenyl | Isobutoxy | |
| 2-13 | CH₃ | Cl | 2,4,6-Trichlorphenyl | 2,2-Dimethylpropoxy | |
| 2-14 | CH₃ | Cl | 2,4,6-Trichlorphenyl | sec-Butoxy | |
| 2-15 | CH₃ | Cl | 2,4,6-Trichlorphenyl | Allyloxy | |
| 2-16 | CH₃ | Cl | 2,4,6-Trichlorphenyl | Cyclohexyloxy | |
| 2-17 | CH₃ | Cl | 2,4,6-Trichlorphenyl | Phenoxy | |
| 2-18 | CH₃ | Cl | 2,4,6-Trichlorphenyl | 4-Chlorphenoxy | |
| 2-19 | CH₃ | Cl | 2,4,6-Trichlorphenyl | (4-Methoxybenzyl)oxy | |
| 2-20 | CH₃ | Cl | 2,4,6-Trichlorphenyl | Cyclopropylmethoxy | |
| 2-21 | CH₃ | Cl | 2,4,6-Trifluorphenyl | 1,2-Dimethylpropoxy | |
| 2-22 | CH₃ | Cl | 2,4,6-Trifluorphenyl | Isobutoxy | |
| 2-23 | CH₃ | Cl | 2,4,6-Trifluorphenyl | 2,2-Dimethylpropoxy | |
| 2-24 | CH₃ | Cl | 2,4,6-Trifluorphenyl | sec-Butoxy | |
| 2-25 | CH₃ | Cl | 2,4,6-Trifluorphenyl | Allyloxy | |
| 2-26 | CH₃ | Cl | 2,4,6-Trifluorphenyl | Cyclohexyloxy | |
| 2-27 | CH₃ | Cl | 2,4,6-Trifluorphenyl | Phenoxy | |
| 2-28 | CH₃ | Cl | 2,4,6-Trifluorphenyl | 4-Chlorphenoxy | |
| 2-29 | CH₃ | Cl | 2,4,6-Trifluorphenyl | (4-Methoxybenzyl)oxy | |
| 2-30 | CH₃ | Cl | 2,4,6-Trifluorphenyl | Cyclopropylmethoxy | |
| 2-31 | CH₃ | Cl | 2,6-Difluorphenyl | 1,2-Dimethylpropoxy | |
| 2-32 | CH₃ | Cl | 2,6-Difluorphenyl | Isobutoxy | 3,57 |
| 2-33 | CH₃ | Cl | 2,6-Difluorphenyl | 2,2-Dimethylpropoxy | |
| 2-34 | CH₃ | Cl | 2,6-Difluorphenyl | sec-Butoxy | |
| 2-35 | CH₃ | Cl | 2,6-Difluorphenyl | Allyloxy | |
| 2-36 | CH₃ | Cl | 2,6-Difluorphenyl | Propargyloxy | 2,50 |
| 2-37 | CH₃ | Cl | 2,6-Difluorphenyl | Cyclohexyloxy | |
| 2-38 | CH₃ | Cl | 2,6-Difluorphenyl | Phenoxy | |
| 2-39 | CH₃ | Cl | 2,6-Difluorphenyl | 4-Chlorphenoxy | 3,86 |
| 2-40 | CH₃ | Cl | 2,6-Difluorphenyl | (4-Methoxybenzyl)oxy | |
| 2-41 | CH₃ | Cl | 2,6-Difluorphenyl | Cyclopropylmethoxy | 3,10 |
| 2-42 | CH₃ | Cl | 2,6-Difluorphenyl | 4-Methoxybenzyloxy | 3,35 |
| 2-43 | CH₃ | Cl | 2,6-Difluorphenyl | (1-Methylcyclopropyl)methoxy | 3,53 |
| 2-44 | CH₃ | Cl | 2-Chlor-4,6-difluorphenyl | 1,2-Dimethylpropoxy | |
| 2-45 | CH₃ | Cl | 2-Chlor-4,6-difluorphenyl | Isobutoxy | |
| 2-46 | CH₃ | Cl | 2-Chlor-4,6-difluorphenyl | 2,2-Dimethylpropoxy | |
| 2-47 | CH₃ | Cl | 2-Chlor-4,6-difluorphenyl | sec-Butoxy | |
| 2-48 | CH₃ | Cl | 2-Chlor-4,6-difluorphenyl | Allyloxy | |
| 2-49 | CH₃ | Cl | 2-Chlor-4,6-difluorphenyl | Cyclohexyloxy | |
| 2-50 | CH₃ | Cl | 2-Chlor-4,6-difluorphenyl | Phenoxy | |
| 2-51 | CH₃ | Cl | 2-Chlor-4,6-difluorphenyl | 4-Chlorphenoxy | |
| 2-52 | CH₃ | Cl | 2-Chlor-4,6-difluorphenyl | (4-Methoxybenzyl)oxy | |
| 2-53 | CH₃ | Cl | 2-Chlor-4,6-difluorphenyl | Cyclopropylmethoxy | |
| 2-54 | CH₃ | Cl | 2-Chlor-6-fluorphenyl | 1,2-Dimethylpropoxy | |
| 2-55 | CH₃ | Cl | 2-Chlor-6-fluorphenyl | Isobutoxy | |
| 2-56 | CH₃ | Cl | 2-Chlor-6-fluorphenyl | 2,2-Dimethylpropoxy | |
| 2-57 | CH₃ | Cl | 2-Chlor-6-fluorphenyl | sec-Butoxy | |
| 2-58 | CH₃ | Cl | 2-Chlor-6-fluorphenyl | Allyloxy | |
| 2-59 | CH₃ | Cl | 2-Chlor-6-fluorphenyl | Cyclohexyloxy | 4,25 |
| 2-60 | CH₃ | Cl | 2-Chlor-6-fluorphenyl | Phenoxy | |
| 2-61 | CH₃ | Cl | 2-Chlor-6-fluorphenyl | 4-Chlorphenoxy | 4,13 |
| 2-62 | CH₃ | Br | 2-Chlor-6-fluorphenyl | 4-Chlorphenoxy | 4,12 |
| 2-63 | CH₃ | Cl | 2-Chlor-6-fluorphenyl | (4-Methoxybenzyl)oxy | |
| 2-64 | CH₃ | Cl | 2-Chlor-6-fluorphenyl | Cyclopropylmethoxy | |
| 2-65 | CH₃ | Cl | 2-Chlor-6-fluorphenyl | 2-Fluorphenoxy | 3,51 |
| 2-66 | CH₃ | Cl | 2-Chlor-6-fluorphenyl | 3-Fluorphenoxy | 3,65 |
| 2-67 | CH₃ | Cl | 2-Chlor-6-fluorphenyl | 4-Fluorphenoxy | 3,56 |
| 2-68 | CH₃ | Cl | 2-Chlor-6-fluorphenyl | 3-(Trifluormethyl)phenoxy | 4,13 |
| 2-69 | CH₃ | Cl | 2-Chlor-6-fluorphenyl | 3-Cyanophenoxy | 3,14 |
| 2-70 | CH₃ | Cl | 2-Chlor-6-fluorphenyl | 2,4-Dichlorphenoxy | 4,46 |
| 2-71 | CH₃ | Cl | 2-Chlor-6-fluorphenyl | 3-(tert-Butyl-)phenoxy | 4,92 |
| 2-72 | CH₃ | Cl | 2-Chlor-6-fluorphenyl | 3-Chlor-5-methoxyphenoxy | 4,13 |
| 2-73 | CH₃ | Cl | 2-Chlor-6-fluorphenyl | 3-Methoxyphenoxy | 3,56 |
| 2-74 | CH₃ | Cl | 2-Chlor-6-fluorphenyl | 3-Chlorphenoxy | 4,03 |
| 2-75 | CH₃ | Cl | 2-Chlor-6-fluorphenyl | 3,4-Dichlorphenoxy | 4,41 |
| 2-76 | CH₃ | Cl | 2-Chlor-6-fluorphenyl | 3-Methylphenoxy | 3,99 |
| 2-77 | CH₃ | Cl | 2-Chlor-6-fluorphenyl | 4-Cyanophenoxy | 3,18 |
| 2-78 | CH₃ | Cl | 2-Chlor-6-fluorphenyl | 3-(N,N-Dimethylamino)phenoxy | 3,85 |
| 2-79 | CH₃ | Cl | 2-Chlor-6-fluorphenyl | 3-(Trifluormethoxy)phenoxy | 4,32 |
| 2-80 | CH₃ | Cl | 2-Chlor-6-fluorphenyl | 2,5-Dichlorphenoxy | 4,27 |
| 2-81 | CH₃ | Cl | 2-Chlor-6-fluorphenyl | 3,5-Dichlorphenoxy | 4,61 |
| 2-82 | CH₃ | Cl | 2-Chlor-6-fluorphenyl | 4-(4-Chlorphenoxy)phenoxy | 5,03 |
| 2-83 | CH₃ | Cl | 2-Chlor-6-fluorphenyl | 2-(2-Methoxyethoxy-)ethoxy | 2,58 |
| 2-84 | CH₃ | Cl | 2-Chlor-6-fluorphenyl | 2-Methoxyethoxy | 2,60 |
| 2-85 | CH₃ | Cl | 2-Chlor-6-fluorphenyl | 4-Oxacyclohexyloxy | 2,68 |
| 2-86 | CH₃ | Cl | 4-Chlor-2,6-difluorphenyl | 1,2-Dimethylpropoxy | |
| 2-87 | CH₃ | Cl | 4-Chlor-2,6-difluorphenyl | Isobutoxy | |
| 2-88 | CH₃ | Cl | 4-Chlor-2,6-difluorphenyl | 2,2-Dimethylpropoxy | |
| 2-89 | CH₃ | Cl | 4-Chlor-2,6-difluorphenyl | sec-Butoxy | |
| 2-90 | CH₃ | Cl | 4-Chlor-2,6-difluorphenyl | Allyloxy | |
| 2-91 | CH₃ | Cl | 4-Chlor-2,6-difluorphenyl | Cyclohexyloxy | |
| 2-92 | CH₃ | Cl | 4-Chlor-2,6-difluorphenyl | Phenoxy | |
| 2-93 | CH₃ | Cl | 4-Chlor-2,6-difluorphenyl | 4-Chlorphenoxy | |
| 2-94 | CH₃ | Cl | 4-Chlor-2,6-difluorphenyl | (4-Methoxybenzyl)oxy | |
| 2-95 | CH₃ | Cl | 4-Chlor-2,6-difluorphenyl | Cyclopropylmethoxy | |

### 3-Chlor-4-(2-chlor-6-fluorphenyl)-5-(4-chlorphenylsulfanyl)-6-methylpyridazin (Verbindung 3-33)

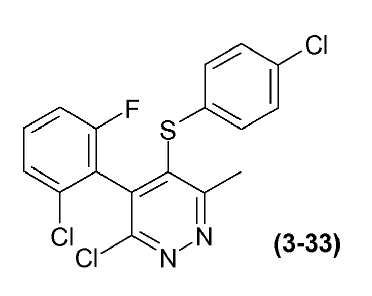

0.033 g (0.82 mmol) Natriumhydrid (als 60%ige Dispersion in Mineralöl) werden in 3 mL DMF vorgelegt und bei Raumtemperatur mit 0.11 g (0.76mmol) 4-Chlorthiophenol versetzt und 30 min. bei Raumtemperatur gerührt. Anschliessend werden 0.2 g (0.69 mmol) 3,5-Dichlor-4-(2-chlor-6-fluorphenyl)-6-methylpyridazin zugegeben und das Gemisch 90 Minuten bei 60°C gerührt. Anschliessend wird die Lösung abgekühlt, in Wasser eingerührt und mehrfach mit Ethylacetat extrahiert. Die organische Phase wird über Natriumsulfat getrocknet und am Rotationsverdampfer konzentriert. Nach Chromatographie an Kieselgel werden 0.16 g (56% Ausbeute, 98% Reinheit; logP = 4.65) 3-Chlor-4-(2-chlor-6-fluorphenyl)-5-[(4-chlorphenyl)thio]-6-methylpyridazin als weißer Feststoff erhalten.

Analog können beispielsweise folgende Verbindungen der Formel **(Ia)** hergestellt werden (siehe Tabelle 3):

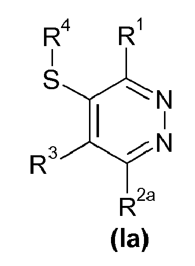

**Tabelle 3**

| **Nr.** | **R¹** | **R^{2a}** | **R³** | **SR⁴** | **logP^{a)}** |
|---|---|---|---|---|---|
| 3-1 | CH₃ | Cl | 2,3,6-Trifluorphenyl | Phenylsulfanyl | |
| 3-2 | CH₃ | Cl | 2,3,6-Trifluorphenyl | (4-Chlorphenyl)sulfanyl | |
| 3-3 | CH₃ | Cl | 2,3,6-Trifluorphenyl | (1,2-Dimethylpropyl)sulfanyl | |
| 3-4 | CH₃ | Cl | 2,3,6-Trifluorphenyl | Isobutylsulfanyl | |
| 3-5 | CH₃ | Cl | 2,3,6-Trifluorphenyl | Sec-Butylsulfanyl | |
| 3-6 | CH₃ | Cl | 2,3,6-Trifluorphenyl | (2,2-Dimethylpropyl)sulfanyl | |
| 3-7 | CH₃ | Cl | 2,4,6-trichlorphenyl | Phenylsulfanyl | |
| 3-8 | CH₃ | Cl | 2,4,6-trichlorphenyl | (4-Chlorphenyl)sulfanyl | |
| 3-9 | CH₃ | Cl | 2,4,6-trichlorphenyl | (1,2-Dimethylpropyl)sulfanyl | |
| 3-10 | CH₃ | Cl | 2,4,6-trichlorphenyl | Isobutylsulfanyl | |
| 3-11 | CH₃ | Cl | 2,4,6-trichlorphenyl | Sec-Butylsulfanyl | |
| 3-12 | CH₃ | Cl | 2,4,6-trichlorphenyl | (2,2-Dimethylpropyl)sulfanyl | |
| 3-13 | CH₃ | Cl | 2,4,6-Trifluorphenyl | Phenylsulfanyl | |
| 3-14 | CH₃ | Cl | 2,4,6-Trifluorphenyl | (4-Chlorphenyl)sulfanyl | |
| 3-15 | CH₃ | Cl | 2,4,6-Trifluorphenyl | (1,2-Dimethylpropyl)sulfanyl | |
| 3-16 | CH₃ | Cl | 2,4,6-Trifluorphenyl | Isobutylsulfanyl | |
| 3-17 | CH₃ | Cl | 2,4,6-Trifluorphenyl | Sec-Butylsulfanyl | |
| 3-18 | CH₃ | Cl | 2,4,6-Trifluorphenyl | (2,2-Dimethylpropyl)sulfanyl | |
| 3-19 | CH₃ | Cl | 2,6-Difluorphenyl | Phenylsulfanyl | |
| 3-20 | CH₃ | Cl | 2,6-Difluorphenyl | (4-Chlorphenyl)sulfanyl | 4,35 |
| 3-21 | CH₃ | Cl | 2,6-Difluorphenyl | (1,2-Dimethylpropyl)sulfanyl | 4,37 |
| 3-22 | CH₃ | Cl | 2,6-Difluorphenyl | Isobutylsulfanyl | |
| 3-23 | CH₃ | Cl | 2,6-Difluorphenyl | Sec-Butylsulfanyl | 3,99 |
| 3-24 | CH₃ | Cl | 2,6-Difluorphenyl | (2,2-Dimethylpropyl)sulfanyl | |
| 3-25 | CH₃ | Cl | 2,6-Difluorphenyl | Isopropylsulfanyl | 3,57 |
| 3-26 | CH₃ | Cl | 2-Chlor-4,6-difluorphenyl | Phenylsulfanyl | |
| 3-27 | CH₃ | Cl | 2-Chlor-4,6-difluorphenyl | (4-Chlorphenyl)sulfanyl | |
| 3-28 | CH₃ | Cl | 2-Chlor-4,6-difluorphenyl | (1,2-Dimethylpropyl)sulfanyl | |
| 3-29 | CH₃ | Cl | 2-Chlor-4,6-difluorphenyl | Isobutylsulfanyl | |
| 3-30 | CH₃ | Cl | 2-Chlor-4,6-difluorphenyl | Sec-Butylsulfanyl | |
| 3-31 | CH₃ | Cl | 2-Chlor-4,6-difluorphenyl | (2,2-Dimethylpropyl)sulfanyl | |
| 3-32 | CH₃ | Cl | 2-Chlor-6-fluorphenyl | Phenylsulfanyl | |
| 3-33 | CH₃ | Cl | 2-Chlor-6-fluorphenyl | (4-Chlorphenyl)sulfanyl | 4,65 |
| 3-34 | CH₃ | Cl | 2-Chlor-6-fluorphenyl | (1,2-Dimethylpropyl)sulfanyl | |
| 3-35 | CH₃ | Cl | 2-Chlor-6-fluorphenyl | Isobutylsulfanyl | |
| 3-36 | CH₃ | Cl | 2-Chlor-6-fluorphenyl | Sec-Butylsulfanyl | |
| 3-37 | CH₃ | Cl | 2-Chlor-6-fluorphenyl | (2,2-Dimethylpropyl)sulfanyl | |
| 3-38 | CH₃ | Cl | 2-Chlor-6-fluorphenyl | 3-Methylphenylsulfanyl | 4,41 |
| 3-39 | CH₃ | Cl | 2-Chlor-6-fluorphenyl | 2,4-Dichlorphenylsulfanyl | 4,87 |
| 3-40 | CH₃ | Cl | 2-Chlor-6-fluorphenyl | 2-Fluorphenylsulfanyl | 3,99 |
| 3-41 | CH₃ | Cl | 2-Chlor-6-fluorphenyl | 3-(Trifluormethyl)phenylsulfanyl | 4,51 |
| 3-42 | CH₃ | Cl | 2-Chlor-6-fluorphenyl | 3-Fluorphenylsulfanyl | 4,08 |
| 3-43 | CH₃ | Cl | 2-Chlor-6-fluorphenyl | 3-Methoxyphenylsulfanyl | 4,03 |
| 3-44 | CH₃ | Cl | 2-Chlor-6-fluorphenyl | 4-Fluorphenylsulfanyl | 4,03 |
| 3-45 | CH₃ | Cl | 2-Chlor-6-fluorphenyl | 3,5-Dichlorphenylsulfanyl | |
| 3-46 | CH₃ | Cl | 2-Chlor-6-fluorphenyl | 3-(Trifluormethoxy)phenylsulfanyl | 4,72 |
| 3-47 | CH₃ | Cl | 4-Chlor-2,6-difluorphenyl | Phenylsulfanyl | |
| 3-48 | CH₃ | Cl | 4-Chlor-2,6-difluorphenyl | (4-Chlorphenyl)sulfanyl | |
| 3-49 | CH₃ | Cl | 4-Chlor-2,6-difluorphenyl | (1,2-Dimethylpropyl)sulfanyl | |
| 3-50 | CH₃ | Cl | 4-Chlor-2,6-difluorphenyl | Isobutylsulfanyl | |
| 3-51 | CH₃ | Cl | 4-Chlor-2,6-difluorphenyl | Sec-Butylsulfanyl | |
| 3-52 | CH₃ | Cl | 4-Chlor-2,6-difluorphenyl | (2,2-Dimethylpropyl)sulfanyl | |

| | | | | | |
|---|---|---|---|---|---|
| ^{a)} Die Bestimmung der logP Werte erfolgte gemäß EEC Directive 79/831 Annex V.A8 durch HPLC (High Performance Liquid Chromatography) an reversed-phase Säulen (C 18), mit nachfolgender Methode: Temperatur: 40°C ; Mobile Phase : 0.1% wässrige Ameisensäure und Acetonitril ; Linearer Gradient von 10% Acetonitril bis 95% Acetonitril. Die Kalibrierung wurde jeweils mit unverzweigten Alkan-2-onen (3 bis 16 Kohlenstoff Atome) mit bekannten logP Werten durchgeführt (Bestimmung von den logP Werten über die Retentionszeiten durch lineare Interpolation zwischen zwei bestimmten Alkanonen). Die Lambda-max Werte wurden jeweils in den Maxima der Chromatographie-Signale durch UV Spektren zwischen 190 nm und 400 nm bestimmt. | | | | | |

### 3-Chlor-4-(2,6-difluorphenyl)-5-[(4-chlorphenyl)sulfinyl]-6-methylpyridazin (Verbindung 4-1)

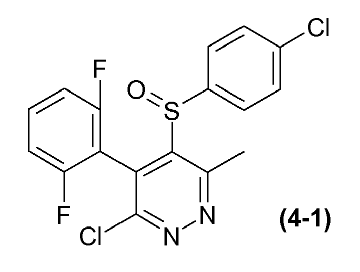

0.46 g (1.2 mmol) 3-Chlor-4-(2,6-difluorphenyl)-5-(4-chlorphenylsulfanyl)-6-methylpyridazin (Verbindung 3-20) werden bei 0 °C in 10 mL Methylenchlorid vorgelegt und nacheinander mit 1.36 mL (3.6 mmol) Ameisensäure und 0.05 g (0.24 mmol) Ammoniummolybdat(VI) versetzt. Danach werden bei 0 °C 0.36 mL (3.6 mmol) 30%ige wässrige Wasserstoffperoxidlösung langsam zugetropft und die Reaktion 18 Stunden gerührt; dabei kommt die Reaktionsmischung auf Raumtemperatur. Die organische Phase wird abgetrennt, mit Wasser gewaschen, getrocknet und einrotiert. Nach Chromatographie an Kieselgel werden 0.18 g (36% Ausbeute, 95% Reinheit; logP = 3.47) 3-Chlor-4-(2,6-difluorphenyl)-5-(4-chlorphenylsulfinyl)-6-methylpyridazin als weißer Feststoff erhalten.

### 3-Chlor-4-(2,6-difluorphenyl)-5-hydroxy-6-methylpyridazin (Verbindung 5-1)

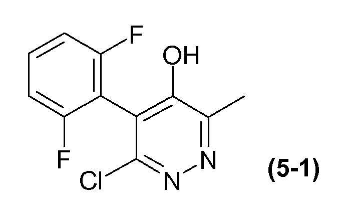

0.3 g (0.8 mmol) 3-Chlor-4-(2,6-difluorphenyl)-5-(4-Methoxybenzyloxy)-6-methylpyridazin (Beispiel 2-42) werden in 1 mL Trifluoressigsäure und 5 mL Methylenchlorid gelöst und 16 Stunden bei Raumtemperatur gerührt. Das Reaktionsgemisch wird einrotiert, und nach Chromatographie des Rückstands an Kieselgel wurden 0.19 g (74% Ausbeute, 80%ig; logP = 1.41) 3-Chlor-4-(2-chlor-6-fluorphenyl)-5-hyroxy-6-methylpyridazin (5-1) als beigefarbener Feststoff erhalten.

### 4-(2,6-Difluorphenyl)-3-methoxy-6-methyl-5-(4-methylpiperidin-1-yl)pyridazin (Verbindung 6-1)

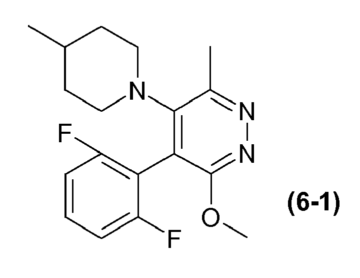

0.2 g (0.59 mmol) 3-Chlor-4-(2,6-difluorphenyl)-6-methyl-5-(4-methylpiperidin-1-yl)pyridazin (Verbindung 1-58) und 0.8 mL (3.55 mmol) einer 25%igen Natriummethylatlösung werden in 1.5 mL Methanol gelöst und 18 h bei 70°C gerührt. Das Reaktionsgemisch wird abgekühlt, mit Wasser versetzt und mehrfach mit Ethylacetat extrahiert. Nach Trocknen der vereinigten organischen Phase über Natriumsulfat wird die Lösung konzentriert, und nach Chromatographie an Kieselgel wurden 0.16 g (77% Ausbeute, 96%ig; logP = 3.39) 4-(2,6-Difluorphenyl)-3-methoxy-6-methyl-5-(4-methylpiperidin-1-yl)pyridazin (6-1) als weißer Feststoff erhalten.

### Verwendungsbeispiele

### Beispiel A

### Venturia inaequalis-Test (Apfel) / protektiv

| | |
|---|---|
| Lösungsmittel : | 24,5 Gewichtsteile Aceton |
| | 24,5 Gewichtsteile Dimethylacetamid |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers ***Venturia inaequalis*** inokuliert und verbleiben dann 1 Tag bei ca. 20 °C und 100% relativer Luftfeuchtigkeit in einer Inkubations-kabine.

Die Pflanzen werden dann im Gewächshaus bei ca. 21 °C und einer relativen Luftfeuchtigkeit von ca. 90% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen mit den Beispielnummern 1-93 und 2-61 bei einer Konzentration an Wirkstoff von 100 ppm einen Wirkungsgrad von 70% oder mehr:

### Beispiel B

### Botrytis cinerea-Test (Bohne) / protektiv

| | |
|---|---|
| Lösungsmittel : | 24,5 Gewichtsteile Aceton |
| | 24,5 Gewichtsteile Dimethylacetamid |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit ***Botrytis cinerea*** bewachsene Agarstückchen aufgelegt. Die inokulierten Pflanzen werden in einer abgedunkelten Kammer bei ca. 20 °C und 100% relativer Luftfeuchtigkeit aufgestellt.

2 Tage nach der Inokulation wird die Größe der Befallsflecken auf den Blättern ausgewertet. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigt die erfindungsgemäße Verbindungen mit der Beispielnummer 1-93 bei einer Konzentration an Wirkstoff von 100 ppm einen Wirkungsgrad von 70% oder mehr.

### Beispiel C

### Septoria tritici-Test (Weizen) / protektiv

| | |
|---|---|
| Lösungsmittel: | 50 Gewichtsteile *N,N-Dimethylacetamid* |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Sporensuspension von *Septoria tritici* besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von 80 % aufgestellt.

21 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen mit den Beispielnummern 1-93 und 2-61 bei einer Konzentration an Wirkstoff von 1000 ppm einen Wirkungsgrad von 70% oder mehr.

### Beispiel D

### Leptosphaeria nodorum-Test (Weizen) / protektiv

| | |
|---|---|
| Lösungsmittel: | 50 Gewichtsteile *N,N-Dimethylacetamid* |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Sporensuspension von *Leptosphaeria nodor um* besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von 80 % aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen mit den Beispielnummern 1-93 und 2-61 bei einer Konzentration an Wirkstoff von 1000 ppm einen Wirkungsgrad von 70% oder mehr.

### Beispiel E

### Pyricularia oryzae-Test (Reis) / protektiv

| | |
|---|---|
| Lösungsmittel: | 49 Gewichtsteile N, N - Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Reispflanzen mit der Wirkstoffzubereitung in der angegebenen Aufwandmenge. Nach dem Antrocknen des Spritzbelages werden die Pflanzen mit einer wäßrigen Sporensuspension von *Pyricularia oryzae* inokuliert und verbleiben dann 24h bei 100% rel. Luftfeuchte und 26°C. Anschließend werden die Pflanzen in einem Gewächshaus bei 80 % rel. Luftfeuchtigkeit und einer Temperatur von 26 °C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die erfindungsgemäßen Verbindungen mit den Beispielnummern 1-93, 1-100 und 2-59 bei einer Konzentration an Wirkstoff von 500 ppm einen Wirkungsgrad von 70% oder mehr.

### Beispiel F

### Alternaria solani-Test (Tomate) / protektiv

| | |
|---|---|
| Lösungsmittel: | 49 Gewichtsteile N, N - Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von *Alternaria solani* inokuliert und stehen dann 24h bei 100% rel. Feuchte und 20°C. Anschließend stehen die Pflanzen bei 96% rel. Luftfeuchtigkeit und einer Temperatur von 20 C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen Verbindungen mit den Beispielnummern 1-93, 1-100 und 2-58 bei einer Konzentration an Wirkstoff von 500 ppm einen Wirkungsgrad von 70% oder mehr.

### Beispiel G

### Sphaerotheca fuliginea-Test (Gurke) / protektiv

| | |
|---|---|
| Lösungsmittel: | 49 Gewichtsteile N, N - Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von *Sphaerotheca fuliginea* inokuliert. Anschließend werden die Pflanzen in einem Gewächshaus bei 70 % relativer Luftfeuchtigkeit und einer Temperatur von 23 °C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die erfindungsgemäßen Verbindungen Verbindungen mit den Beispielnummern 1-100 und 2-59 bei einer Konzentration an Wirkstoff von 500 ppm einen Wirkungsgrad von 70% oder mehr.

## Patentansprüche

1. Verbindungen der Formel (I), 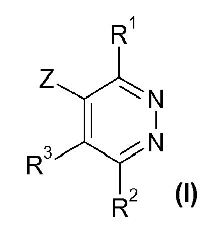 in welcher die Symbole folgende Bedeutung haben:
R¹ steht für Wasserstoff, jeweils geradkettiges oder verzweigtes C₁-C₆-Alkyl, C₂-C₆-Alkenyl, C₂-C₆-Alkinyl, C₃-C₇-Cycloalkyl oder C₃-C₇-Cycloalkenyl, wobei R¹ für den Fall, dass es ungleich Wasserstoff ist, durch einen bis drei gleiche oder verschiedene Reste substituiert sein kann, ausgewählt aus Halogen, Cyano, Oxo, Nitro, C₁-C₄-Alkylamino, Di-(C₁-C₄-) Alkylamino, C₁-C₄-Akl, C₁-C₄-Halogenalkyl, C₁-C₄-Alkoxy oder unsubstituiertes oder durch Halogen, C₁-C₄-Alkyl oder C₁-C₄-Halogenalkyl substituiertes C₃-C₆-Cycloalkyl,
R² steht für Fluor, Chlor, Brom, Cyano, jeweils geradkettiges oder verzweigtes C₁-C₄-Alkoxy, C₃-C₆-Cycloalkyloxy, C₃-C₆-Alkenyloxy, C₃-C₆-Alkinyloxy, C₁-C₄-Alkylthio, C₁-C₄-Alkylsulfinyl, C₁-C₄-Alkylsulfonyl, C₁-C₄-Halogenalkoxy, C₁-C₄-Halogenalkylthio, C₁-C₄-Halogenalkylsulfinyl oder C₁-C₄-Halogenalkylsulfonyl wobei die genannten Reste ausser für die Fälle, in denen R² Fluor, Chlor, Brom oder Cyano ist, durch einen bis drei gleiche oder verschiedene Reste ausgewählt aus Halogen, Cyano, Oxo, Nitro, C₁-C₄-Alkylamino, C₁-C₄-Dialkylamino, C₁-C₄-Alkylthio, C₁-C₄-Alkyl, C₁-C₄-Halogenalkyl, tri(C₁-C₄-Alkyl)silyl, C₁-C₄-Alkoxy oder unsubstituiertem oder durch Halogen, C₁-C₄-Alkyl oder C₁-C₄-Halogenalkyl substituiertem C₃-C₈-Cycloalkyl substituiert sein können,
oder
R² steht für jeweils unsubstituiertes oder im Phenylteil einfach bis dreifach substituiertes Phenoxy oder Benzyloxy, wobei die Substituenten im Phenylteil unabhängig voneinander gleichartig oder verschieden sein können ausgewählt aus Fluor, Chlor, Brom, Cyano, Nitro, Methyl, Methoxy, Dimethylamino, Trifluormethyl oder Trifluormethoxy,
R³ steht für einen Phenylring oder einen ungesättigten Heterocyclus mit fünf bis sieben Ringgliedern und ein bis vier Heteroatomen aus der Gruppe Stickstoff, Sauerstoff und Schwefel, mit der Maßgabe dass zwei Sauerstoffatome nicht benachbart sein dürfen, gegebenenfalls annelliert mit einem weiteren fünf- bis sechsgliedrigen gesättigten oder ganz oder teilweise ungesättigten carbocyclischen oder heterocyclischen Ring, wobei der zusätzliche heterocyclische Ring gegebenenfalls ein bis zwei zusätzliche Heteroatome aus der Gruppe Stickstoff, Sauerstoff und Schwefel enthalten kann, mit der Maßgabe dass zwei Sauerstoffatome nicht benachbart sein dürfen,
welche jeweils einfach oder mehrfach unabhängig voneinander gleich oder verschieden durch folgende Reste substituiert sein können:
Wasserstoff, Fluor, Chlor, Brom, Jod, Cyano, Nitro, Amino, Hydroxy, Mercapto, Formyl, Carboxy, Carbamoyl, Thiocarbamoyl,
jeweils geradkettiges oder verzweigtes C₁-C₆-Alkyl, C₁-C₆-Alkoxy, C₁-C₆-Alkylthio, C₁-C₆-Alkylsulfinyl oder C₁-C₆-Alkylsulfonyl;
jeweils geradkettiges oder verzweigtes C₂-C₆-Alkenyl, C₂-C₆-Alkenylamino oder C₂-C₆-Alkenyloxy;
jeweils geradkettiges oder verzweigtes C₂-C₆-Alkinyl, C₂-C₆-Alkinylamino oder C₂-C₆-Alkinyloxy;
jeweils geradkettiges oder verzweigtes C₁-C₆-Halogenalkyl, C₁-C₆-Halogenalkoxy, C₁-C₇-Halogenalkcylcarbonyl, C₁-C₆-Halogenalkylthio, C₁-C₆-Halogenalkylsulfinyl oder C₁-C₆-Halogenalkylsulfonyl mit jeweils 1 bis 13 gleichen oder verschiedenen Halogenatomen;
jeweils geradkettiges oder verzweigtes C₂-C₆-Halogenalkenyl oder C₂-C₆-Halogenalkenyloxy mit jeweils 1 bis 11 gleichen oder verschiedenen Halogenatomen oder geradkettiges oder verzweigtes C₂-C₆-Halogenalkinyloxy;
jeweils geradkettiges oder verzweigtes C₁-C₆-Alkylamino, Di-(C₁-C₆-alkyl)amino, C₁-C₆-Alkylcarbonyl, C₁-C₆-Alkylcarbonyloxy, C₁-C₆-Alkylcarbonylamino, C₁-C₆-Alkoxycarbonyl, C₁-C₆-Alkoxycarbonyloxy, C₁-C₆-Alkoxycarbonylamino, C₁-C₆-Alkylaminocarbonyl, C₁-C₆-Alkylaminocarbonyloxy, C₁-C₆-Alkylaminosulfonyl, Di-(C₁-C₆-alkyl)aminocarbonyl, Di-(C₁-C₆-alkyl)aminocarbonyloxy, Di-(C₁-C₆-alkyl)aminosulfonyl, C₁-C₆-Allcylsulfonylamino, C₁-C₆-Alkylsulfonyloxy, Hydroximino-C₁-C₆-alkyl oder C₁-C₆-Alkoximino-C₁-C₆-alkyl;
unsubstituiertes oder in den Alkylteilen einfach oder mehrfach substituiertes C₃-C₈-Cycloalkyl, C₃-C₈-Cycloalkylamino, C₃-C₈-Cycloalkylaminocarbonyl, C₃-C₈-Cycloalkylaminocarbonyloxy, wobei die Substituenten unabhängig voneinander gleich oder verschieden sein können ausgewählt aus Fluor, Chlor, Methyl, Cyclopropyl oder Trifluormethyl;
jeweils geradkettiges oder verzweigtes C₁-C₆-Alkoxy-C₁-C₆-alkyl, C₁-C₆-Halogenalkoxy-C₁-C₆-allcyl, C₁-C₆-Alkylamino-C₁-C₆-alkyl, Di-(C₁-C₆-alkyl)amino-C₁-C₆-alkyl;
jeweils geradkettiges oder verzweigtes C₁-C₆-Alkoxy-C₁-C₆-alkoxy, C₁-C₆-Alkoxy-C₁-C₆-alkoxy-C₁-C₆-alkoxy, C₁-C₆-Halogenalkoxy-C₁-C₆-alkoxy, C₁-C₆-Alkylamino-C₁C₆-alkoxy, Di-(C₁-C₆-alkyl)amino-C₁C₆-alkoxy;
unsubstituiertes oder einfach bis fünffach substituiertes Phenyl, wobei die Substituenten unabhängig voneinander gleich oder verschieden sein können ausgewählt aus Fluor, Chlor, Methyl, Trifluormethyl, N-Methoxyethanimidoyl, (Methoxyimino)methyl;
oder zwei benachbarte Reste am Phenylring oder am Heterocyclus bilden zusammen einen fünf- bis siebengliedrigen gesättigten, teilweise gesättigten oder ungesättigten substituierten oder unsubstituierten Ring ggf. enthaltend bis zu zwei Heteroatome ausgewählt aus Stickstoff, Sauerstoff oder Schwefel, mit der Maßgabe dass zwei Sauerstoffatome nicht benachbart sein dürfen und wobei die Substituenten, unabhängig voneinander gleich oder verschieden sein können ausgewählt aus Fluor, Chlor, Methyl, Cyclopropyl oder Trifluormethyl;
oder zwei Reste am Heterocyclus bilden mit dem Kohlenstoffatom, an das sie gebundenen sind, eine Carbonyl- oder Thiocarbonylgruppe;
Z steht für NR⁴R⁵, NR⁴R⁹, OR⁸, OR¹⁰ oder S(O)ₙR⁴, wobei n gleich 0, 1 oder 2 sein kann;
R⁴ ist gleich R³, Wasserstoff oder jeweils geradkettiges oder verzweigtes C₁-C₈-Alkyl, C₂-C₈-Alkenyl, C₂-C₆-Alkinyl, C₃-C₇-Cycloalkyl oder C₃-C₇-Cycloalkenyl, wobei R⁴, für den Fall, dass es ungleich R³ oder Wasserstoff ist, jeweils unsubstituiert oder einfach oder mehrfach gleich oder verschieden unabhängig voneinander substituiert sein kann durch Cyano, Oxo, Nitro, C₁-C₄-Alkoxycarbonyl, C₁-C₄-Alkylcarbonyl, Di-(C₁-C₄-Alkyl)aminocarbonyl oder einen gesättigten oder teilweise ungesättigten drei- bis siebengliedrigen Ring, gegebenenfalls enthaltend bis zu drei Heteroatome ausgewählt aus Stickstoff, Sauerstoff oder Schwefel mit der Maßgabe dass zwei Sauerstoffatome nicht benachbart sein dürfen, wobei der Ring selbst gegebenenfalls einfach oder mehrfach gleich oder verschieden unabhängig voneinander durch Fluor, C₁-C₄-Alkyl oder C₁-C₄-Halogenalkyl substituiert sein kann,
oder
R⁴ steht für jeweils geradkettiges oder verzweigtes C₁-C₆-Halogenalkyl oder C₂-C₆-Halogenalkenyl mit jeweils 1 bis 11 gleichen oder verschiedenen Halogenatomen,jeweils geradkettiges oder verzweigtes C₁-C₆-Allcylamino-C₁-C₆-alkyl, Di-(C₁-C₆-alkyl)amino-C₁-C₆-alkyl, C₁-C₆-Alkoxy-C₁-C₆-alkyl oder C₁-C₆-Halogenalkoxy-C₁-C₆-alkyl, geradkettiges oder verzweigtes Phenyl-(C₁-C₆)alkyl, welches im Phenylteil unsubstituiert oder einfach oder mehrfach unabhängig voneinander gleich oder verschieden substituiert sein kann mit Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy oder für einen fünf- bis siebengliedrigen gesättigten oder teilweise ungesättigten substituierten oder unsubstituierten Ring, gegebenenfalls enthaltend bis zu drei Heteroatome ausgewählt aus Stickstoff, Sauerstoff oder Schwefel, mit der Maßgabe dass zwei Sauerstoffatome nicht benachbart sein dürfen;
R⁵ steht für NR⁶R⁷, Wasserstoff, geradkettiges oder verzweigtes C₁-C₆-Halogenalkyl mit jeweils 1 bis 11 gleichen oder verschiedenen Halogenatomen, jeweils geradkettiges oder verzweigtes C₁-C₆-Allcyl, C₂-C₆-Alkenyl oder C₃-C₇-Cycloalkyl, jeweils geradkettiges oder verzweigtes C₁-C₆-Alkoxy, C₂-C₆-Alkenyloxy, C₂-C₆-Alkinyloxy oder C₃-C₇-Cycloalkoxy, wobei R⁵, falls es ungleich NR⁶R⁷ oder Wasserstoff ist, jeweils unsubstituiert oder einfach oder mehrfach unabhängig voneinander gleich oder verschieden substituiert sein kann durch Cyano, Oxo, Nitro, C₁-C₄-Alkoxycarbonyl, C₁-C₄-Alkylcarbonyl, Di-(C₁-C₄-Alkyl)aminocarbonyl oder durch einen gesättigten oder teilweise ungesättigten drei- bis siebengliedrigen Ring, gegebenenfalls enthaltend bis zu drei Heteroatome ausgewählt aus Stickstoff, Sauerstoff oder Schwefel mit der Maßgabe, dass zwei Sauerstoffatome nicht benachbart sein dürfen, wobei der Ring selbst gegebenenfalls einfach oder mehrfach gleich oder verschieden unabhängig voneinander durch Fluor, C₁-C₆-Alkyl oder C₁-C₆-Halogenalkyl substituiert sein kann;
oder
R⁵ steht für jeweils geradkettiges oder verzweigtes C₁-C₆-Halogenalkoxy oder C₂-C₆-Halogenalkenyloxy mit jeweils 1 bis 11 gleichen oder verschiedenen Halogenatomen,jeweils geradkettiges oder verzweigtes C₁-C₆-Alkylamino-C₁-C₆-alkoxy, Di-(C₁-C₆-alkyl)amino-C₁-C₆-alkoxy, C₁-C₆-Alkoxy-C₁-C₆-alkoxy oder C₁-C₆-Halogenalkoxy-C₁-C₆-alkoxy, geradkettiges oder verzweigtes Phenyl-(C₁-C₆)alkoxy, welches im Phenylteil unsubstituiert oder einfach oder mehrfach unabhängig voneinander gleich oder verschieden mit Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy substituiert sein kann,
oder
R⁵ ist gleich Phenoxy, welches einfach bis fünffach unabhängig voneinander gleich oder verschiedenen durch folgende Reste substituiert sein kann:
Wasserstoff, Fluor, Chlor, Brom, Jod, Cyano, Nitro, Amino, Hydroxy, Mercapto, Formyl, Carboxy, Carbamoyl, Thiocarbamoyl,
jeweils geradkettiges oder verzweigtes C₁-C₆-Alkyl, C₁-C₆-Alkoxy, C₁-C₆-Alkylthio, C₁-C₆-Alkylsulfinyl oder C₁-C₆-Alkylsulfonyl,
jeweils geradkettiges oder verzweigtes C₂-C₆-Alkenyl, C₂-C₆-Alkenylamino oder C₂-C₆-Alkenyloxy,
jeweils geradkettiges oder verzweigtes C₂-C₆-Alkinyl, C₂-C₆-Alkinylamino oder C₂-C₆-Alkinyloxy,
jeweils geradkettiges oder verzweigtes C₁-C₆-Halogenalkyl, C₁-C₆-Halogenalkoxy, C₁-C₇-Halogenalkylcarbonyl, C₁-C₆-Halogenalkylthio, C₁-C₆-Halogenalkylsulfinyl oder C₁-C₆-Halogenalkylsulfonyl mit jeweils 1 bis 13 gleichen oder verschiedenen Halogenatomen,
jeweils geradkettiges oder verzweigtes C₂-C₆-Halogenalkenyl oder C₂-C₆-Halogenalkenyloxy mit jeweils 1 bis 11 gleichen oder verschiedenen Halogenatomen oder geradkettiges oder verzweigtes C₂-C₆-Halogenalkinyloxy,
jeweils geradkettiges oder verzweigtes C₁-C₆-Alkylamino, Di-(C₁-C₆-alkyl)amino, C₁-C₆-Alkylcarbonyl, C₁-C₆-Alkylcarbonyloxy, C₁-C₆-Alkylcarbonylamino, C₁-C₆-Alkoxycarbonyl, C₁-C₆-Alkoxycarbonyloxy, C₁-C₆-Alkoxycarbonylamino, C₁-C₆-Alkylaminocarbonyl, C₁-C₆-Alkylaminocarbonyloxy, C₁-C₆-Alkylaminosulfonyl, Di-(C₁-C₆-alkyl)aminocarbonyl, Di-(C₁-C₆-alkyl)aminocarbonyloxy, Di-(C₁-C₆-alkyl)aminosulfonyl, C₁-C₆-Alkylsulfonylamino, C₁-C₆-Alkylsulfonyloxy, Hydroximino-C₁-C₆-allcyl oder C₁-C₆-Alkoximino-C₁-C₆-alkyl,
unsubstituiertes oder in den Alkylteilen einfach oder mehrfach substituiertes C₃-C₈-Cycloalkyl, C₃-C₈-Cycloalkylamino, C₃-C₈-Cycloalkylaminocarbonyl, C₃-C₈-Cycloalkylaminocarbonyloxy, wobei die Substituenten unabhängig voneinander gleich oder verschieden sein können ausgewählt aus Fluor, Chlor, Methyl, Cyclopropyl oder Trifluormethyl,
jeweils geradkettiges oder verzweigtes C₁-C₆-Alkoxy-C₁-C₆-alkyl, C₁-C₆-Halogenalkoxy-C₁-C₆-allcyl, C₁-C₆-Alkylamino-C₁-C₆-alkyl, Di-(C₁-C₆-alkyl)amino-C₁-C₆-alkyl,
jeweils geradkettiges oder verzweigtes C₁-C₆-Alkoxy-C₁-C₆-alkoxy, C₁-C₆-Alkxoy-C₁-C₆-alkoxy-C₁-C₆-alkoxy, C₁-C₆-Halogenalkoxy-C₁-C₆-alkoxy, C₁-C₆-Alkylamino-C₁C₆-alkoxy, Di-(C₁-C₆-alkyl)amino-C₁C₆-alkoxy,
unsubstituiertes oder einfach bis fünffach substituiertes Phenyl, wobei die Substituenten unabhängig voneinander gleich oder verschieden sein können ausgewählt aus Fluor, Chlor, Methyl, Trifluormethyl, N-Methoxyethanimidoyl, (Methoxyimino)methyl,
oder zwei benachbarte Reste am Phenoxyring bilden zusammen einen fünf- bis siebengliedrigen gesättigten, teilweise gesättigten oder ungesättigten substituierten oder unsubstituierten Ring ggf. enthaltend bis zu zwei Heteroatome ausgewählt aus Stickstoff, Sauerstoff oder Schwefel, mit der Maßgabe dass zwei Sauerstoffatome nicht benachbart sein dürfen und wobei die Substituenten, unabhängig voneinander gleich oder verschieden sein können ausgewählt aus Fluor, Chlor, Methyl, Cyclopropyl oder Trifluormethyl;
oder
R⁴ und R⁵ bilden zusammen einen drei bis zwölf Ringatome enthaltenden gesättigten oder teilweise ungesättigten substituierten oder unsubstituierten monocyclischen oder bicyclischen Ring gegebenenfalls enthaltend bis zu zwei zusätzliche Heteroatome ausgewählt aus Stickstoff, Sauerstoff, Schwefel oder Silizium, mit der Maßgabe dass zwei Sauerstoffatome nicht benachbart sein dürfen;
R⁶ und R⁷ stehen unabhängig voneinander gleichartig oder verschieden für R³, C₁-C₆-Alkyl, C₃-C₇-Cycloalkyl, C₁-C₆-Alkoxy, C₁-C₆-Alkylcarbonyl, C₁-C₆-Alkoxycarbonyl, C₁-C₆-Alkylaminocarbonyl, Di-(C₁-C₆-Alkyl)-aminocarbonyl oder geradkettiges oder verzweigtes Phenyl-(C₁-C₆)alkyl, welches im Phenylteil unsubstituiert oder einfach oder mehrfach unabhängig voneinander gleich oder verschieden mit Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy substituiert sein kann,
oder
R⁶ und R⁷ bilden zusammen einen drei bis zwölf Ringatome enthaltenden gesättigten oder teilweise ungesättigten substituierten oder unsubstituierten monocyclischen oder bicyclischen Ring gegebenenfalls enthaltend bis zu zwei zusätzliche Heteroatome ausgewählt aus Stickstoff, Sauerstoff, Schwefel oder Silizium, mit der Maßgabe dass zwei Sauerstoffatome nicht benachbart sein dürfen,
R⁸ steht für R³, NR⁶R⁷, jeweils geradkettiges oder verzweigtes C₁-C₈-Alkyl, C₂-C₈-Alkenyl, C₂-C₈-Alkinyl, C₃-C₇-Cycloalkyl oder C₃-C₇-Cycloalkenyl, wobei R⁸ für den Fall, dass es ungleich R³ und NR⁶R⁷ ist, jeweils unsubstituiert oder einfach oder mehrfach unabhängig voneinander gleich oder verschieden substituiert sein kann durch Cyano, Oxo, Nitro, C₁-C₄-Alkoxycarbonyl, C₁-C₄-Alkylcarbonyl, Di-(C₁-C₄-alkyl)aminocarbonyl oder durch einen gesättigten oder teilweise ungesättigten drei- bis siebengliedrigen Ring, gegebenenfalls enthaltend bis zu drei Heteroatome ausgewählt aus Stickstoff, Sauerstoff oder Schwefel mit der Maßgabe dass zwei Sauerstoffatome nicht benachbart sein dürfen, wobei der Ring selbst gegebenenfalls einfach oder mehrfach unabhängig voneinander gleich oder verschieden durch Fluor, C₁-C₆-Alkyl oder C₁-C₆-Halogenalkyl substituiert sein kann,
oder
R⁸ steht für jeweils geradkettiges oder verzweigtes C₁-C₆-Halogenalkyl oder C₂-C₆-Halogenalkenyl mit jeweils 1 bis 11 gleichen oder verschiedenen Halogenatomen, jeweils geradkettiges oder verzweigtes C₁-C₆Alkylamino-C₁-C₆-alkyl, Di-(C₁-C₆-alkyl)amino-C₁-C₆-alkyl, C₁-C₆-Alkoxy-C₁-C₆-alkyl, C₁-C₆-Halogenalkoxy-C₁-C₆-alkyl oder geradkettiges oder verzweigtes Phenyl-(C₁-C₆)alkyl, welches im Phenylteil unsubstituiert oder einfach oder mehrfach unabhängig voneinander gleich oder verschieden mit Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy substituiert sein kann oder für einen fünf- bis siebengliedrigen gesättigten oder teilweise ungesättigten substituierten oder unsubstituierten Ring, gegebenenfalls enthaltend bis zu drei Heteroatome ausgewählt aus Stickstoff, Sauerstoff oder Schwefel, mit der Maßgabe dass zwei Sauerstoffatome nicht benachbart sein dürfen,
R⁹ steht für Formyl, geradkettiges oder verzweigtes Tri-(C₁-C₆-alkyl)silyl, jeweils geradkettiges oder verzweigtes C₁-C₆-Alkylcarbonyl, C₃-C₇-Cycloalkylcarbonyl, C₁-C₆-Alkoxycarbonyl, C₁-C₆-Alkylaminocarbonyl, Di-(C₁-C₆-Alkyl)aminocarbonyl, C₁-C₆-Alkylaminothiocarbonyl, Di-(C₁-C₆-alkyl)aminothiocarbonyl, C₁-C₆-Alkylsulfmyl oder C₁-C₆-Alkylsulfonyl, wobei R⁹ für den Fall, dass es ungleich Formyl ist, jeweils unsubstituiert oder einfach oder mehrfach unabhängig voneinander gleich oder verschieden substituiert sein kann durch Fluor, Chlor, Cyano, C₁-C₄-Alkoxy oder C₁-C₄-Alkyl,
oder
R⁹ steht für jeweils geradkettiges oder verzweigtes C₁-C₆-Halogenalkylcarbonyl, C₁-C₆-Halogenalkylsulfinyl oder C₁-C₆-Halogenalkylsulfonyl mit jeweils 1 bis 13 gleichen oder verschiedenen Halogenatomen, jeweils unsubstituiertes oder einfach oder mehrfach substituiertes Phenylcarbonyl, Phenylacetyl, Phenoxycarbonyl, Phenylaminocarbonyl, Phenylaminothiocarbonyl, Phenylsulfinyl, Phenylsulfonyl, Heteroarylcarbonyl, oder Heteroarylaminocarbonyl, wobei die Substituenten im Phenylteil oder Heteroarylteil unabhängig voneinander gleichartig oder verschieden sein können ausgewählt aus Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, oder Trifluormethoxy,
R¹⁰ steht für jeweils geradkettiges oder verzweigtes C₁-C₆-Alkylcarbonyl, C₃-C₇-Cycloalkylcarbonyl, C₁-C₆-Alkoxycarbonyl, C₁-C₆-Alkylaminocarbonyl oder Di-(C₁-C₆-alkyl)aminocarbonyl, welches jeweils unsubstituiert oder einfach oder mehrfach unabhängig voneinander durch Fluor, Chlor, Cyano, C₁-C₄-Alkoxy oder C₁-C₄-Alkyl substituiert sein kann, geradkettiges oder verzweigtes Tri-(C₁-C₆-alkyl)silyl, jeweils unsubstituiertes oder einfach oder mehrfach substituiertes Phenylcarbonyl, Phenylacetyl, Phenylaminocarbonyl, Phenylaminothiocarbonyl oder Heteroarylcarbonyl, wobei die Substituenten im Phenylteil unabhängig voneinander gleichartig oder verschieden sein können ausgewählt aus Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy,
sowie agrochemisch wirksame Salze davon.

2. Verbindungen der Formel (I) gemäß Anspruch 1, **dadurch gekennzeichnet, dass** die Symbole folgende Bedeutung haben:
R¹ steht für Wasserstoff, Methyl, Ethyl, n-Propyl, Isopropyl, Cyclopropyl, Cyclopropylmethyl, n-Butyl, Allyl, Propargyl, Methoxymethyl, Fluormethyl, Difluormethyl, Trifluormethyl oder 2,2,2-Trifluorethyl,
R² steht für Fluor, Chlor, Brom, Cyano, Methoxy, Ethoxy, 2,2,2-Trifluorethoxy, Methylthio, Ethylthio, Methylsulfinyl, Ethylsulfinyl, Methylsulfonyl oder Ethylsulfonyl,
R³ steht für einen Phenylring, welcher einfach bis dreifach unabhängig voneinander gleich oder verschieden durch folgende Reste substituiert sein kann:
Chlor, Brom, Iod, Fluor, Fluormethyl, Difluormethyl, Trifluormethyl, Trifluormethoxy, Trifluormethylthio, Hydroxy, Methoxy, Methoxyethoxy, Nitro, Cyano, Methyl, Ethyl, Amino, Methylamino, Dimethylamino, 2-(Dimethylamino)ethoxy, 2-Morpholin-4-ylethoxy, 3-(Dimethylamino)propoxy oder 3-Morpholin-4-ylpropoxy,
oder zwei benachbarte Reste am Phenylring bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach, unabhängig voneinander gleich oder verschieden substituiert sein können durch Fluor, Chlor, Methyl, Cyclopropyl und/oder Trifluormethyl;
oder
R³ steht für 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4-Triazol-1-yl, 1,2,4-Triazol-3-yl, 1,2,4-Triazol-4-yl, 1,2,4-Triazol-5-yl, 1,2,3-Triazol-1-yl, 1,2,3-Triazol-2-yl, 1,2,3-Triazol-4-yl, Tetrazol-1-yl, Tetrazol-2-yl, Tetrazol-5-yl, Indol-1-yl, Indol-2-yl, Indol-3-yl, Isoindol-1-yl, Isoindol-2-yl, Benzofur-2-yl, Benzothiophen-2-yl, Benzofur-3-yl, Benzothiophen-3-yl, Benzoxazol-2-yl, Benzothiazol-2-yl, Benzimidazol-2-yl, Indazol-1-yl, Indazol-2-yl, Indazol-3-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl oder 1,2,4-Triazin-6-yl, welches jeweils einfach oder mehrfach unabhängig voneinander gleich oder verschieden durch folgende Reste substituiert sein kann ausgewählt aus Wasserstoff, Fluor, Chlor, Brom, Iod, Cyano, Hydroxy, Mercapto, Amino, Methyl, Ethyl, n-Propyl, Isopropyl, n-Butyl, Isobutyl, sek-Butyl, tert-Butyl, Cyclopropyl, 1-Chlorcyclopropyl, Vinyl, Ethinyl, Methoxy, Ethoxy, Isopropoxy, Methylthio, Ethylthio, Trifluormethylthio, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2,2-Trifluorethyl, Trifluormethoxy, Trifluormethylthio, 2-Methoxyethoxy, Acetyl, Propionyl, Methoxycarbonyl, N-Methylamino, N,N-Dimethylamino, Aminocarbonyl, Methylaminocarbonyl, Dimethylaminocarbonyl, Dimethylcarbamoylamino, Methoxycarbonylamino, Methoxycarbonyloxy, Methylsulfamoyl, Dimethylsulfamoyl, Phenyl oder Phenoxy,
oder zwei benachbarte Reste am Heterocyclus bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach unabhängig voneinander gleich oder verschieden substituiert sein können durch Fluor, Chlor, Methyl, Cyclopropyl und/oder Trifluormethyl,
oder zwei Reste am Heterocyclus bilden ganz besonders bevorzugt mit dem Kohlenstoffatom, an das sie gebundenen sind, eine Carbonyl- oder Thiocarbonylgruppe,
R⁴ ist Wasserstoff oder ein unsubstituierter oder einfach bis dreifach substituierter Phenylring, dessen Substituenten unabhängig voneinander gleichartig oder verschieden sein können ausgewählt aus Chlor, Brom, Iod, Fluor, Fluormethyl, Difluormethyl, Trifluormethyl, Trifluormethoxy, Trifluormethylthio, Hydroxy, Methoxy, Methoxyethoxy, Cyano, Methyl, Ethyl, Amino, Methylamino, Dimethylamino, Acetyl, Methoxycarbonyl, N,N-Dimethylcarbamoyl,
oder
R⁴ ist ein heteroaromatischer Rest ausgewählt aus 2-Furyl, 3-Furyl, 2-Thienyl, 3-Thienyl, 2-Oxazolyl, 4-Oxazolyl, 5-Oxazolyl, 2-Thiazolyl, 4-Thiazolyl, 5-Thiazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, Benzofur-2-yl, Benzothiophen-2-yl, Benzofur-3-yl, Benzothiophen-3-yl, 2-Pyridinyl, 3-Pyridinyl, 4-Pyridinyl, 2-Pyrimidinyl, 4-Pyrimidinyl oder 5-Pyrimidinyl, welcher jeweils unsubstituiert oder einfach oder mehrfach substituiert ist unabhängig voneinander durch gleiche oder verschiedenene Reste ausgewählt aus Fluor, Chlor, Brom, Iod, Cyano, Hydroxy, Mercapto, Methyl, Ethyl, tert-Butyl, Cyclopropyl, Vinyl, Ethinyl, Methoxy, Ethoxy, Trifluormethyl, Trifluormethoxy, 2-Methoxyethoxy, Acetyl, N-Methylamino, N,N-Dimethylamino, N-Methylcarbamoyl oder N,N-Dimethylcarbamoyl ,
oder
R⁴ ist eine Gruppe ausgewählt aus 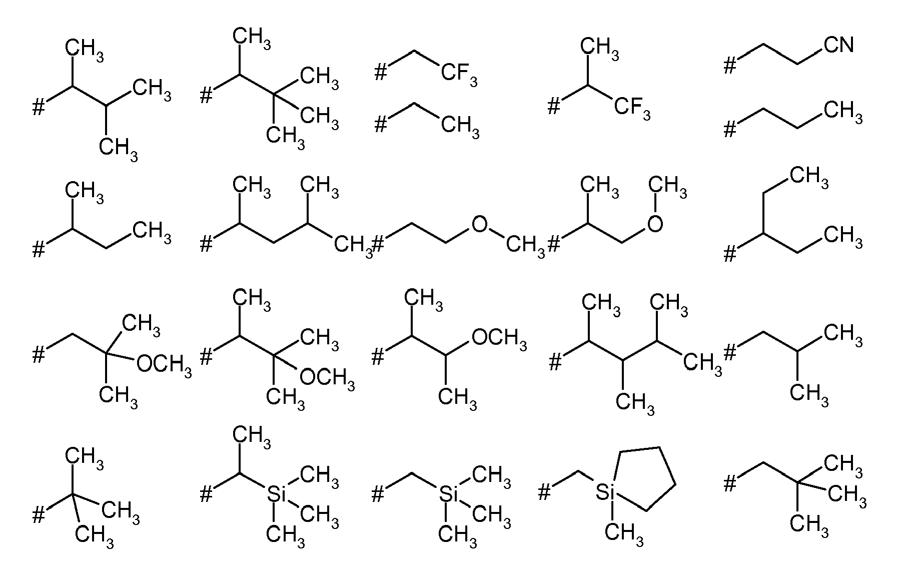 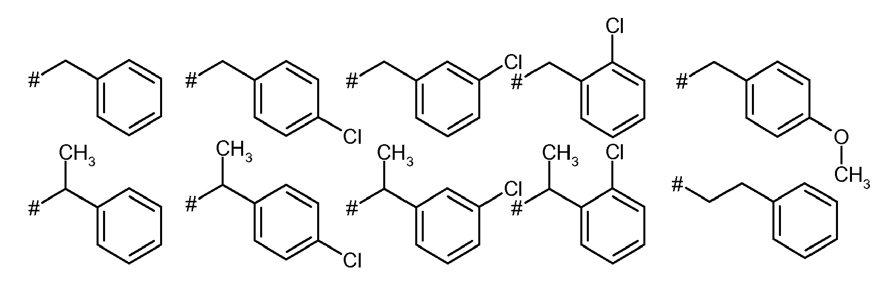 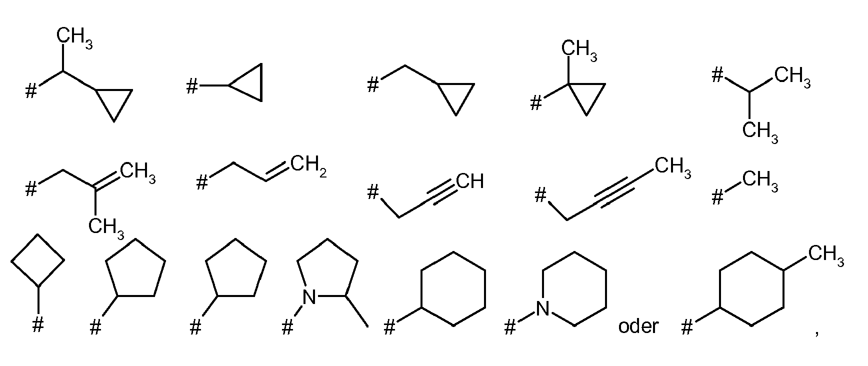 wobei "#" die Anknüpfungsstelle markiert;
R⁵ steht für Wasserstoff, Methyl, Ethyl, n-Propyl, Isopropyl, n-Butyl, Isobutyl, Pentyl, 2-Methylbutyl, 3-Methylbutyl, 2,2-Di-methylpropyl, Cyclopropyl, Cyclobutyl, 2,2,2-Trifluorethyl, 1-(Trifluormethyl-)propyl, 2-Cyanoethyl, 2-Methoxyethyl, 2-Methoxy-2-methylpropyl, Cyclopropylmethyl, 2-Methylprop-2-enyl, Allyl, Propargyl, 3-Methylpropargyl, Amino, Methylamino, Dimethylamino, Ethylamino, Diethylamino, Methylethylamino, Cyclopropylamino, Isopropylamino, N-Methyl-N-isopropylamino, Phenylamino, 4-Chlorphenylamino, 3-Chlorphenylamino, 2-Chlorphenylamino, N-Methyl-N-phenylamino, N-Methyl-N-(4-chlorphenyl)amino, N-Methyl-N-(3-chlorphenyl)amino, N-Methyl-N-(2-chlorphenyl)amino, Methoxy, Ethoxy, Isopropoxy, 1,1,1-Trifluorisopropoxy, Phenoxy, 2-Chlorphenoxy, 3-Chlorphenoxy oder 4-Chlorphenoxy,
oder
R⁴ und R⁵ bilden zusammen mit dem Stickstoffatom, an das sie gebunden sind, einen Ring ausgewählt aus 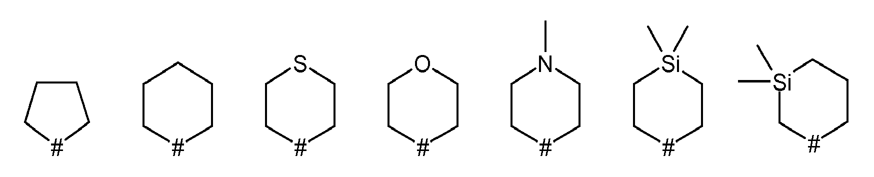 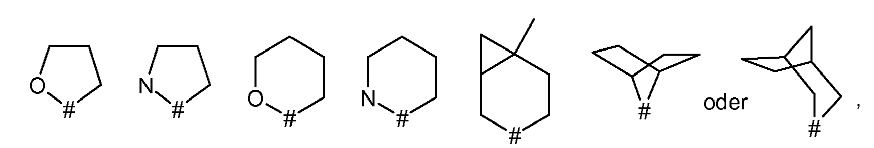 wobei der Ring unsubstituiert oder einfach bis dreifach unabhängig voneinander gleichartig oder verschieden durch Methyl, Fluor oder Trifluormethyl substituiert sein kann und "#" das Stickstoffatom symbolisiert über welches der Ring gebunden ist;
R⁸ ist ein einfach bis dreifach substituierter Phenylring, dessen Substituenten unabhängig voneinander gleichartig oder verschieden sein können ausgewählt aus Chlor, Brom, Iod, Fluor, Fluormethyl, Difluormethyl, Trifluormethyl, Trifluormethoxy, Trifluormethylthio, Hydroxy, Methoxy, Methoxyethoxy, Nitro, Cyano, Methyl, Ethyl, Amino, Methylamino, Dimethylamino, Acetyl, Methoxycarbonyl, N,N-Dimethylcarbamoyl, Phenyl oder Phenoxy;
oder
R⁸ ist ein heteroaromatischer Rest ausgewählt aus 2-Furyl, 3-Furyl, 2-Thienyl, 3-Thienyl, 2-Oxazolyl, 4-Oxazolyl, 5-Oxazolyl, 2-Thiazolyl, 4-Thiazolyl, 5-Thiazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, Benzofur-2-yl, Benzothiophen-2-yl, Benzofur-3-yl, Benzothiophen-3-yl, 2-Pyridinyl, 3-Pyridinyl, 4-Pyridinyl, 2-Pyrimidinyl, 4-Pyrimidinyl oder 5-Pyrimidinyl, welcher jeweils unsubstituiert oder einfach oder mehrfach durch gleiche oder verschiedenene Reste ausgewählt aus Fluor, Chlor, Brom, Iod, Cyano, Hydroxy, Mercapto, Methyl, Ethyl, tert-Butyl, Cyclopropyl, Vinyl, Ethinyl, Methoxy, Ethoxy, Trifluormethyl, Trifluormethoxy, 2-Methoxyethoxy, Acetyl, N-Methylamino, N,N-Dimethylamino, N-Methylcabamoyl oder N,N-Dimethylcarbamoyl substituiert sein kann;
oder
R⁸ ist jeweils unsubstituiertes oder im Phenylteil einfach bis dreifach substituiertes Benzyl, 1-Phenylethyl oder 2-Phenylethyl, wobei die Substituenten im Phenylteil unabhängig voneinander gleichartig oder verschieden sein können ausgewählt aus Fluor, Chlor, Brom, Iod, Cyano, Nitro, Methyl, 1,1-Dimethylethyl, Methoxy, Methylamino, Dimethylamino, Fluormethyl, Difluormethyl, Trifluormethyl, Trifluormethylthio oder Trifluormethoxy;
oder
R⁸ ist gleich Benzyl, 2-Chlorbenzyl, 3-Chlorbenzyl, 4-Chlorbenzyl, 4-Methoxybenzyl, 3,4-Dimethoxybenzyl, 1-(Phenyl)ethyl, 1-(2-Chlorphenyl)ethyl, 1-(3-Chlorphenyl)ethyl, 1-(4-Chlorphenyl)ethyl, Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, 1,1-Dimethylpropyl, 1,2-Dimethylpropyl, 1-Methyl-pentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; Cyclopropyl, 1-Methoxycyclopropyl, 1-Chlorcyclopropyl, 1-Methylcyclopropyl, Cyclobutyl, Cyclopentyl, Cyclohexyl, 4-Methylcyclohexyl, 2,2,2-Trifluorethyl, 1-(Trifluormethyl-)propyl, 2-Cyanoethyl, 1,3-Dimethylbutyl, 2-Methoxyethyl, 1-Methyl-2-methoxypropyl, 2-Methoxy-2-methylpropyl, 2-Methoxy-1,2-dimethylpropyl, 2-Methoxy-1-methylpropyl, 1-(Cyclopropyl)ethyl, Cyclopropylmethyl, 2-Methylprop-2-enyl, Allyl, Propargyl, 3-Methylpropargyl, Methylamino, Dimethylamino, Ethylamino, Diethylamino, Methylethylamino, Cyclopropylamino, 1-Piperidinyl, 1-Pyrrolidinyl, Phenylamino, 4-Chlorphenylamino, N-Phenyl-N-methylamino, 1,1-Dimethylethylamino, Cyclohexylamino oder Isopropylamino;
R⁹ steht für Formyl, Acetyl, 2-Chloracetyl, 2-Methoxyacetyl, n-Propionyl, i-Propionyl, 2-Methoxypropionyl, Pivaloyl, Phenylacetyl, 4-Chlorphenylacetyl, Methoxycarbonyl, Ethoxycarbonyl, n-Propoxycarbonyl, i-Propoxycarbonyl, tert.-Butoxycarbonyl, N,N-Dimethylcarbamoyl, Methylsulfonyl, Ethylsulfonyl, Benzoyl, 2-Chlorbenzoyl, 3-Chlorbenzoyl, 4-Chlorbenzoyl, Phenylcarbamoyl, 2-Chlorphenylcarbamoyl, 3-Chlorphenylcarbamoyl, 4-Chlorphenylcarbamoyl, 2-Methoxyphenylcarbamoyl, 3-Methoxyphenylcarbamoyl, 4-Methoxyphenylcarbamoyl, Phenylthiocarbamoyl, 2-Methoxyphenylthiocarbamoyl, 3-Methoxyphenylthiocarbamoyl, 4-Methoxyphenylthiocarbamoyl, Phenylsulfonyl, 4-Methylphenylsulfonyl, Trimethylsilyl, Triethylsilyl, tert-Butyldimethylsilyl oder Triisopropylsilyl,
R¹⁰ steht für Formyl, Acetyl, 2-Chloracetyl, 2-Methoxyacetyl, n-Propionyl, i-Propionyl, Pivaloyl, Phenylacetyl, 2-Chlorphenylacetyl, 3-Chlorphenylacetyl, 4-Chlorphenylacetyl, Methoxycarbonyl, Ethoxycarbonyl, n-Propoxycarbonyl, i-Propoxycarbonyl, Cyclopropylcarbonyl, tert.-Butoxycarbonyl, N,N-Dimethylcarbamoyl, Phenylcarbamoyl, Trimethylsilyl, Triethylsilyl, tert-Butyldimethylsilyl, Triisopropylsilyl, Phenylcarbamoyl, 2-Chlorphenylcarbamoyl, 4-Chlorphenylcarbamoyl, 4-Chlorphenylcarbamoyl, 2-Methoxyphenylcarbamoyl, 4-Methoxyphenylcarbamoyl, 4-Methoxyphenylcarbamoyl, Phenylcarbonyl, 2-Chlorphenylcarbonyl, 4-Chlorphenylcarbonyl, 4-Chlorphenylcarbonyl, 2-Methoxyphenylcarbonyl, 4-Methoxyphenylcarbonyl, 4-Methoxyphenylcarbonyl, 2-Cyanophenylcarbonyl, 4-Cyanophenylcarbonyl oder 4-Cyanophenylcarbonyl,
sowie agrochemisch wirksame Salze davon.

3. Mittel zur Bekämpfung von unerwünschten Mikroorganismen, **gekennzeichnet durch** einen Gehalt an mindestens einem Pyridazin der Formel (I) gemäß einem der Ansprüche 1 oder 2 neben Streckmitteln und/oder oberflächenaktiven Stoffen und/oder Trägerstoffen.

4. Verfahren zur Bekämpfung von unerwünschten Mikroorganismen, **dadurch gekennzeichnet, dass** man Verbindungen der Formel (I) gemäß einem der Ansprüche 1 oder 2 bzw. Mittel wie in Anspruch 3 definiert auf die unerwünschten Mikroorganismen und/oder deren Lebensraum ausbringt.

5. Verwendung von Verbindungen der Formel (I) gemäß einem der Ansprüche 1 oder 2 bzw. Mitteln wie in Anspruch 3 definiert zur Bekämpfung von unerwünschten Mikroorganismen.

6. Verwendung von Verbindungen der Formel (I) gemäß einem der Ansprüche 1 oder 2 zur Behandlung von Saatgut.

7. Verfahren zum Herstellen der erfindungsgemäßen Pyridazine der Formel (I) gemäß Anspruch 1, umfassend wenigstens einen der folgenden Schritte (a) bis (k):
a) Halogenierung von Hydroxypyridazinonen **(VII)** mit einem Halogenierungsmittel und gegebenenfalls einer Base zu Dihalogenpyridazinen **(VIII)** und gegebenenfalls weitere Umsetzung mit Fluorid und gegebenenfalls einem Katalysator gemäß nachfolgendem Reaktionsschema: 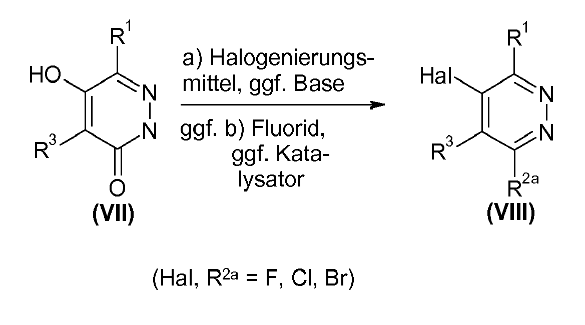
b) Umsetzung von Dihalogenpyridazinen **(VIII)** mit Thioalkoholen R⁴-SH und gegebenenfalls in Gegenwart einer Base gemäß nachfolgendem Reaktionsschema: 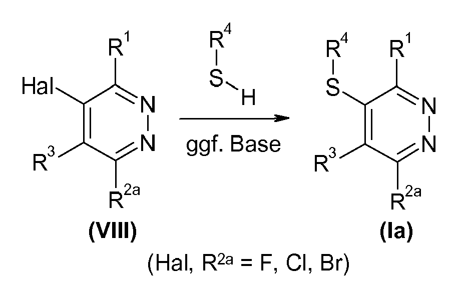
c) Umsetzung von Dihalogenpyridazinen **(VIII)** mit Alkoholen R⁸-OH und gegebenenfalls in Gegenwart einer Base gemäß nachfolgendem Reaktionsschema: 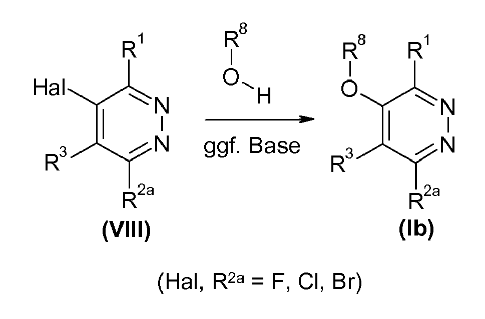
d) Umsetzung von Dihalogenpyridazinen **(VIII)** mit Aminen R⁴R⁵NH und gegenenfalls in Gegenwart einer zusätzlichen Base gemäß nachfolgendem Reaktionsschema: 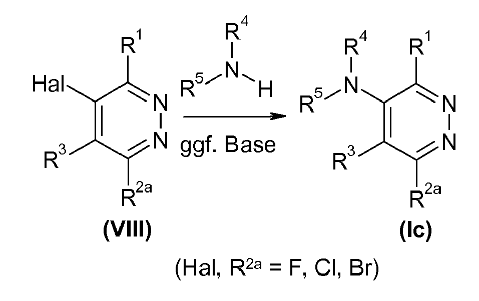
e) Umsetzung von Pyridazinen der Formeln **(Ia), (Ib)** oder **(Ic)** mit einer Cyanidquelle gegebenenfalls in Gegenwart eines Katalysators gemäß nachfolgendem Reaktionsschema: 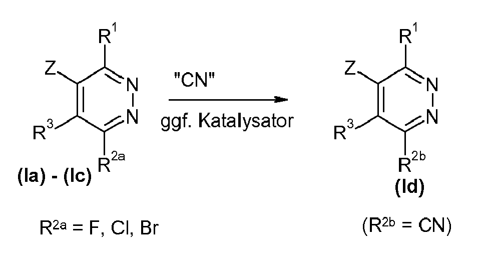
f) Umsetzung von Pyridazinen der Formeln **(Ia), (Ib)** oder **(Ic)** mit einer Verbindung R^{2c}-SH gegebenenfalls in Gegenwart einer Base gemäß nachfolgendem Reaktionsschema: 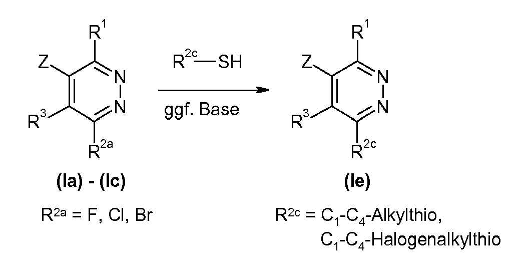
g) Umsetzung von Pyridazinen der Formeln **(Ia), (Ib)** oder **(Ic)** mit einer Verbindung R^{2d} -OH gegebenenfalls in Gegenwart einer Base gemäß nachfolgendem Reaktionsschema: 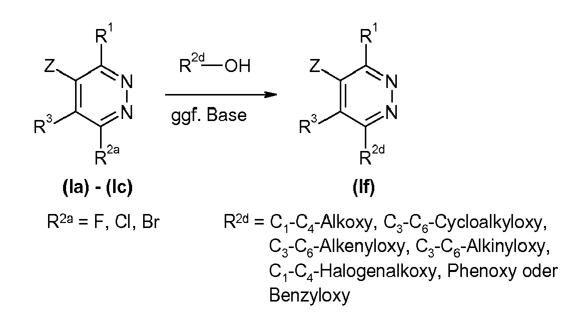
h) Umsetzung von Pyridazinen der Formel (**Ie**) in Gegenwart eines Oxidationsmittels, gegebenenfalls in Gegenwart eines Katalysators und / oder gegegebenenfalls in Gegenwart einer Base gemäß nachfolgendem Reaktionsschema: 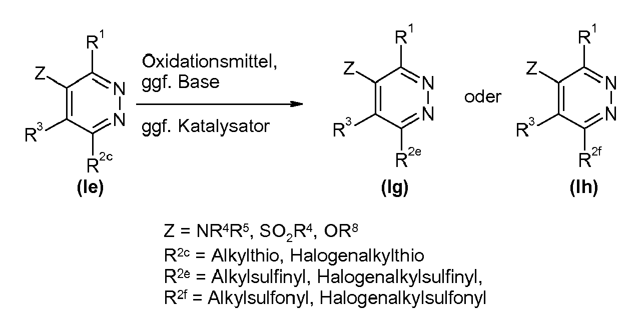
i) Umsetzung von Pyridazinen der Formel **(Ia')** mit einem Oxidationsmittel gegebenenfalls in Gegenwart einer Base und / oder gegebenenfalls in Gegenwart eines Katalysators gemäß nachfolgendem Reaktionsschema: 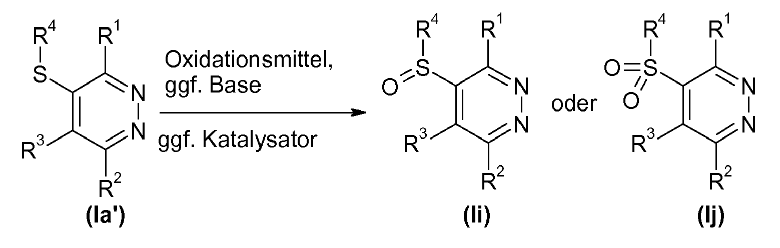
j) Umsetzung von Pyridazinen der Formel **(Ic')** mit einer Verbindung R⁹-Y, gegebenenfalls in Gegenwart eines Katalysators und / oder gegebenenfalls in Gegenwart einer Base gemäß nachfolgendem Reaktionsschema: 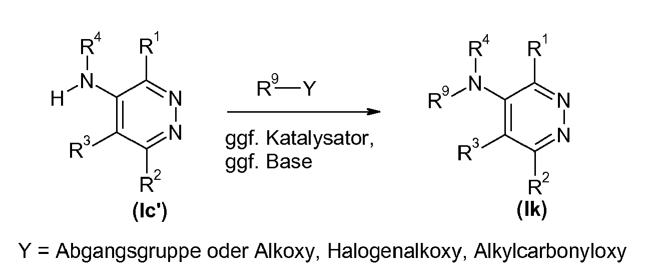
k) Umsetzung von Pyridazinen der Formel **(Im)** mit einer Verbindung R¹⁰-Y, gegebenenfalls in Gegenwart eines Katalysators und / oder gegebenenfalls in Gegenwart einer Base gemäß nachfolgendem Reaktionsschema: 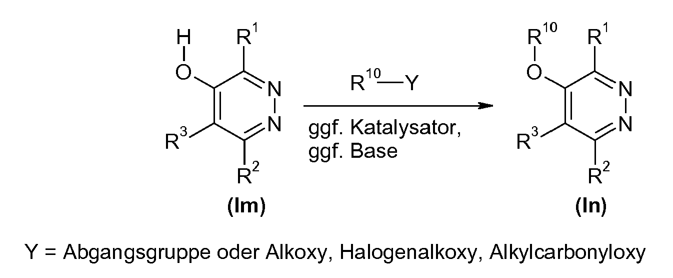
wobei die Definitionen der Reste R¹ bis R¹⁰ oben angegebenen Definitionen aus Anspruch 1 oder 2 entsprechen, Hal für Chlor, Brom oder Fluor stehen kann und
R^{2a} steht für Fluor, Chlor oder Brom,
R^{2b} steht für Cyano,
R^{2c} steht für C₁-C₄-Alkylthio oder C₁-C₄-Halogenalkylthio,
R^{2d} steht für C₁-C₄-Alkoxy, C₃-C₆-Cycloalkyloxy, C₃-C₆-Alkenyloxy, C₃-C₆-Alkinyloxy, C₁-C₄-Halogenalkoxy, Phenoxy oder Benzyloxy,
R^{2e} steht für C₁-C₄-Alkylsulfinyl oder C₁-C₄-Halogenalkylsulfinyl,
R^{2f} steht für C₁-C₄-Alkylsulfonyl oder C₁-C₄-Halogenalkylsulfonyl,
Z' steht für NR⁴R⁵, SO₂R⁴ oder OR⁸ und
Y steht für eine Abgangsgruppe, C₁-C₄-Alkoxy, C₁-C₄-Halogenalkoxy oder C₁-C₄-Alkylcarbonyloxy.

8. Verbindungen der Formel (VII), 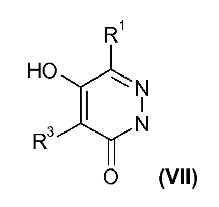 in denen die Reste R¹ und R³ die oben angegebenen Bedeutungen haben.

9. Verbindungen der Formel (VIII), 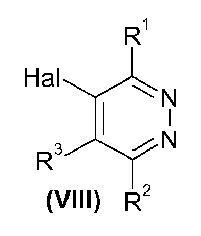 in denen die Reste R¹, R² und R³ die oben angegebenen Bedeutungen haben und Hal für F, Cl oder Br steht.
